# EUROPEAN PATENT SPECIFICATION

(11) **EP 4 530 667 B1**
(45) Date of publication and mention of the grant of the patent: **23.09.2026**
(21) Application number: 24824742.1
(22) Date of filing: 24.07.2024
(51) Int. Cl.: G01S 13/58, B60R 21/015, G01S 7/288, G01S 7/35, G01S 7/41, H04B 1/525, G01S 13/88

(54) **TARGET DETECTION METHOD, INTEGRATED CIRCUIT, ELECTROMAGNETIC WAVE SENSOR AND TERMINAL DEVICE**
ZIELERKENNUNGSVERFAHREN, INTEGRIERTE SCHALTUNG, SENSOR FÜR ELEKTROMAGNETISCHE WELLEN UND ENDGERÄT
PROCÉDÉ DE DÉTECTION DE CIBLE, CIRCUIT INTÉGRÉ, CAPTEUR D'ONDES ÉLECTROMAGNÉTIQUES ET DISPOSITIF TERMINAL

(30) Priority: 24.07.2023 CN 202310913653
(43) Date of publication of application: 02.04.2025
(73) Proprietor: Calterah Semiconductor Technology (Shanghai) Co., Ltd, Shanghai 201210 (CN)
(72) Inventor: WANG, Zhifei, Shanghai 201210 (CN); CHEN, Jiashu, Shanghai 201210 (CN)
(74) Representative: HGF
(86) International application number: PCT/CN2024/107406
(87) International publication number: WO 2025/021129

(56) References cited:
- WO-A1-2021/134449
- CN-A- 110 954 895
- CN-A- 112 180 340
- CN-A- 112 835 006
- CN-A- 113 885 003
- CN-A- 115 902 807
- US-A1- 2021 072 372
- US-A1- 2023 138 972

## Description

### Technical Field

Embodiments of the present disclosure relate to, but are not limited to, the technical field of electromagnetic wave sensors, and particularly relate to a method and system, an integrated circuit, a sensor, and a device of target detection.

### Background

When detecting targets in closed or relatively closed space regions, for example, detecting targets in automobile cabins, there are technical problems such as low detection rate, many false alarm targets, and inaccurate angle estimation.

Related technology is known from US2021072372A1.

### Summary

The invention is set out in the appended set of claims. It should be noted that embodiments of the invention are those whose scope is within that of the appended claims, and the implementations disclosed in this disclosure which do not fall under the scope of the appended claims are to be considered as examples for illustration. The following is a summary of the subject matter described in detail herein.

An embodiment of the invention defined in claim 1 provides a method of target detecting, performed by a processor, and the method comprising:
accumulating frame data from 1D-FFT data obtained through range FFT processing of an echo signal; wherein the frame data refers to data corresponding to the echo signal formed after a frame of detection signal is reflected by a target, and the frame of detection signal comprises a plurality of chirp signals; wherein the accumulating frame data from 1D-FFT data obtained through range FFT processing of an echo signal comprises: (1) performing 1D-FFT processing on chirp data of each frame to obtain the 1D-FFT data; and (2) accumulating the 1D-FFT data to obtain accumulated multi-frame 1D-FFT data;
conducting inter-frame FFT processing based on the accumulated multi-frame 1D-FFT to generate a RD spectrum; and
detecting a target within a region of interest based on the RD spectrum.

Optionally, the method may include: accumulating frame data from 1D-FFT data until a preset data amount is integrated, and then performing inter-frame FFT processing to obtain an RD spectrum.

Optionally, the noise floor estimation result for each individual transceiver channel is composed of the noise floor estimation results from each range bin on that transceiver channel;
the CFAR detection result for each individual transceiver channel is composed of the CFAR detection results from each Doppler bin within each range bin on that transceiver channel; and
the CFAR detection is individually applied to the corresponding transceiver channel based on the noise floor estimation result of that transceiver channel, as implemented by the following expression: $F \left(c, r, v\right) = \left\{\begin{matrix}0 , & if P \left(c, r, v\right) < β n ′ \left(c, r\right) \\ 1 , & if P \left(c, r, v\right) \geq β n ′ \left(c, r\right)\end{matrix},\right.$
wherein,F(c, r, v) is a CFAR detection result of a v-th Doppler bin of a r-th range bin of a c-th channel, P(c, r, v) is echo power on the v-th Doppler bin of the r-th range bin of the c-th channel, *n*'(*c, r*) is a noise floor estimation result of the r-th range bin of the c-th channel, B is a default parameter, and β≥1.

Optionally, conducting a noise floor estimation for each transceiver channel undergoing CFAR processing in accordance with the RD spectrum comprises: conducting a noise floor estimation on the transceiver channel undergoing individual CFAR processing within a predefined range based on the RD spectrum; wherein the upper boundary of the predefined range is determined based on the noise floor estimation result of the range bin with the maximum distance.

Optionally, the CFAR detection is performed according to the currently obtained candidate target data and the preset threshold value is realized by a following expression: ${F}_{sum} \left(r, v\right) = {\sum }_{c = 1}^{{N}_{c}} F \left(c, r, v\right) ,$ $F ′ \left(r, v\right) = \left\{\begin{matrix}0 , if {F}_{sum} \left(r, v\right) < {F}_{0} \\ 1 , if {F}_{sum} \left(r, v\right) \geq {F}_{0}\end{matrix},\right.$ wherein,F(c, r, v) is obtained candidate target data of a v-th Doppler bin of an r-th range bin of a c-th channel, N_{c} is a total number of channels, F₀ is the preset threshold value, F'(r, v) is a final target data of the v-th Doppler bin of the r-th range bin.

Optionally, targets are verified within each predefined zone based on either the relative proportion of targets within each zone to the overall detected targets or the signal-to-noise ratio (SNR) of the targets within each zone.

Optionally, for a multi-channel application scenario, the detection of the target in the region of interest based on the RD spectrum may include: after non-coherent integration of the RD spectrum of each channel, continuing a constant false alarm rate processing based on noise estimation, estimation of wave arrival direction, and determination, positioning, and classification operation of the target.

Optionally, the constant false alarm rate processing may be DAE CFAR processing for azimuth and elevation, respectively.

Optionally, the detection of the target in the region of interest may include: performing classification operation of the target based on extracting at least a part of the data integrated in multiple frames, the data after inter-frame FFT, the non-coherent integrated data, and/or the constant false alarm rate detection data.

Optionally, for a multi-channel application scenario, the detection of the target in the region of interest based on the RD spectrum may include: after performing constant false alarm rate processing on the RD spectrum of each channel, continuing binary integration processing in the channel domain and the estimation of wave arrival direction, and then performing determination, positioning, and classification operation of the target.

Optionally, the detection of the target in the region of interest based on the RD spectrum includes: performing two-dimensional digital beam forming on the RD spectrum, and continuing base target classification processing to realize determination, positioning, and classification operation of the target.

Optionally, performing deep learning target detection, deep learning target positioning, and/or deep learning target classification on the 1D-FFT data may include: obtaining target point cloud data based on the 1D-FFT data; and performing ML-based false alarm suppression, clustering processing and/or ML-based target classification processing on the target point cloud data to realize determination, positioning and/or classification operation of the target in the region of interest.

Optionally, the method may further include: performing two-dimensional digital beam forming on the 1D-FFT data, continuing to integrate frame data until a preset amount of data is integrated, and then performing inter-frame FFT processing to obtain an RD spectrum; and performing DL-based target classification processing based on the RD spectrum to realize determination, positioning, and classification operation of the target.

An embodiment of the present application further provides a target detection method, wherein the method may include: obtaining 1D-FFT data after performing range FFT processing based on an echo signal; after performing two-dimensional digital beam forming on the 1D-FFT data, continuing to accumulate frame data; and after integrating a preset amount of data, performing a DL-based target classification processing to realize determination, positioning and classification operation of the target.

An embodiment of the present application further provides an integrated circuit, which may include: a signal transmitting module configured for electromagnetic waves for target detection; a signal receiving module configured to receive an echo formed by reflection and/or scattering of the electromagnetic wave; and a processing module configured to perform signal and data processing on the echo according to the method of any of the preceding embodiments to achieve detection of a target.

Optionally, the processing module includes a baseband unit and an MCU unit, the baseband unit is configured to implement range FFT processing and inter-frame FFT processing in the method according to any embodiment of the present application, and the MCU unit is configured to implement accumulating frame data and target detection in a region of interest in the method according to any embodiment of the present application.

Optionally, when the method includes digital beam forming and accumulating frame data, the baseband unit may be configured to implement the digital beam forming, and the MCU unit may be configured to implement accumulating frame data.

Optionally, the integrated circuit is a millimeter wave chip or a sensor chip.

An embodiment of the present application further provides an electromagnetic wave sensor, which may include: a carrier; the integrated circuit as described in any one of the embodiments of the present application, which is provided on the carrier; an antenna, the antenna is provided on the carrier, or the antenna is integrated with the integrated circuit as an integral device provided on the carrier; wherein the integrated circuit is connected with the antenna for transmitting the electromagnetic wave signal and/or receiving the echo signal.

An embodiment of the present application further provides a terminal device, which may includes: a device body; and the electromagnetic wave sensor according to any of the embodiments disposed on the device body; wherein the electromagnetic wave sensor is used for target detection and/or communication to provide reference information for operation of the device body.

An example, which is not part of the invention as claimed, further provides a non-transitory computer-readable storage medium having computer-readable instructions stored thereon, and when the instructions are executed by a processor, the processor is enabled to execute the above method.

After the drawings and detailed description are read and understood, other aspects can be understood.

### Brief Description of Drawings

The above and other objects, features, and advantages of the present application will become more apparent from the following description of embodiments of the present application with reference to the drawings.
FIG. 1 is a schematic flow diagram of implementing target detection based on SISO-combine according to an embodiment of the present application.
FIG. 2 is a schematic flow diagram of another method of implementing target detection based on SISO-combine in an embodiment of the present application.
FIG. 3 is a flowchart of a target detection method provided in an embodiment of the present application.
FIG. 4 is a schematic diagram of a duty cycle of a radar provided in an embodiment of the present application.
FIG. 5 is a schematic diagram of a waveform of a detection signal of an FMCW radar provided in an embodiment of the present application.
FIG. 6 is another flowchart of a target detection method provided in an embodiment of the present application.
FIG. 7 is another flowchart of a target detection method provided in an embodiment of the present application.
FIG. 8 is another flowchart of a target detection method provided in an embodiment of the present application.
FIG. 9 is another flowchart of a target detection method provided in an embodiment of the present application.
FIG. 10 is a schematic diagram of data reading involved in a target detection method provided in an embodiment of the present application.
FIG. 11 is another flowchart of a target detection method provided in an embodiment of the present application.
FIG. 12 is another flowchart of a target detection method provided in an embodiment of the present application.
FIG. 13 is a schematic diagram of data transfer involved in the target detection method provided in an embodiment of the present application.
FIG. 14 is another flowchart of a target detection method provided in an embodiment of the present application.
FIG. 15 is a schematic diagram of dividing a predefined zone involved in the target detection method provided in an embodiment of the present application.
FIG. 16 is another flowchart of a target detection method provided in an embodiment of the present application.
FIG. 17 is another flowchart of a target detection method provided in an embodiment of the present application.
FIG. 18 is a schematic diagram of a distribution of target points in a predefined zone involved in the target detection method provided in the embodiment of the present application.
FIG. 19 is another flowchart of a target detection method provided in an embodiment of the present application.
FIG. 20 is a schematic flow diagram of implementing target detection based on per-channel in an embodiment of the present application.
FIG. 21 is a flowchart of a target detection method provided in an embodiment of the present application.
FIG. 22 is another flowchart of a target detection method provided in an embodiment of the present application.
FIG. 23 is another flowchart of a target detection method provided in an embodiment of the present application.
FIG. 24 is a schematic flow diagram of target detection after a target detection method is combined with a multi-frame joint processing scheme provided in an embodiment of the present application.
FIG. 25 is another flowchart of a target detection method provided in an embodiment of the present application.
FIG. 26 is another flowchart of a target detection method provided in an embodiment of the present application.
FIG. 27 is another flowchart of a target detection method provided in an embodiment of the present application.
FIG. 28 is schematic diagram of output involved in a target detection method provided in an embodiment of the present application.
FIG. 29 is a schematic flow diagram of implementing target detection based on frame-level FFT combined with DAE CFAR in an embodiment of the present application.
FIG. 30 is a schematic diagram of an azimuth or elevation CFAR in the target detection flow based on an illustration in FIG. 3
FIG. 31 is a flowchart of a target detection method according to an embodiment of the present disclosure
FIG. 32 is a schematic flow diagram of implementing target detection based on frame-level FFT combined with DAE CFAR in an embodiment of the present disclosure.
FIG. 33 is a schematic diagram of a result after non-coherent integration.
FIG. 34 is a partial diagram of a result of non-coherent integration.
FIG. 35 is a schematic diagram of target points in sixth regions according to an embodiment of the present disclosure.
FIG. 36A-FIG. 36D show the processing results in a single-person scenario (a baby in aisle C).
FIG. 37A-FIG. 37D show the processing results in a two-person scenario (a baby in seat B and an adult in seat A).
FIG. 38 is a schematic flow diagram of post-processing for a target according to an embodiment of the present application.
FIG. 39 is a schematic flow diagram of post-processing for a target in combination with a DL algorithm in an embodiment of the present application.
FIG. 40 is a schematic flow diagram of a target detection method combined with a DL algorithm according to an embodiment of the present application.
FIG. 41 is a schematic flow diagram of another target detection method combined with a DL algorithm according to an embodiment of the present application.
FIG. 42 is a schematic flow diagram of a further target detection method combined with a DL algorithm according to an embodiment of the present application.

### Detailed Description

In order to facilitate the understanding of the present application, the present application will be described more fully below with reference to the drawings. Preferred embodiments of the present application are shown in the drawings.

The embodiments are provided for the purpose of providing a more thorough and comprehensive understanding of the contents of the present application.

Unless otherwise defined, all technical and scientific terms used herein have the same meaning as commonly understood by those skilled in the art to which the present application belongs. The terminology used in the specification of the present application is for the purpose of describing specific embodiments only, and is not intended to limit the present application.

An example, which is not part of the invention as claimed, provides a method of target detection, including:
S1, accumulating frame data from 1D-FFT data, wherein the 1D-FFT data is obtained based on range FFT processing of an echo signal;
S2, performing inter-frame FFT processing based on the accumulated frame data to obtain an RD spectrum; and
S3, implementing detection of a target in a region of interest based on the RD spectrum.

For example, all of the 1DFFT data after the 1D-FFT processing may be subjected to inter-frame integration and subjected to inter-frame FFT processing, or part of the 1DFFT data may be subjected to inter-frame integration, for example, when the region of interest is smaller than a range of echo data, the 1DFFT data obtained by performing range FFT processing on only part of the echo signal may be subjected to inter-frame integration and inter-frame FFT processing, or part of the 1DFFT data may be subjected to inter-frame integration and inter-frame FFT processing.

For example, the range FFT processing and the inter-frame FFT processing may be performed simultaneously, for example, the range FFT processing continues to be performed simultaneously with the inter-frame FFT processing.

When detecting a target in a closed or relatively closed space region, in some optional embodiments, the target detection may be realized by performing region determination based on single-frame fast-slow time processing, CFAR (Constant False-Alarm Rate) detection, and point cloud obtained by angle measurement, etc., or based on algorithms such as Capon used to perform DoA (direction of arrival estimation) and other operations after a single-frame fast-time processing, thereby forming an RA (Range-Azimuth) map, and then extracting features such as power in a predetermined region for classification and determination to achieve target detection, or the target detection is achieved by detecting human breathing and heartbeat based on filtering the received echo phase. For example, when target detection is realized by performing region determination based on single-frame fast-slow time processing, CFAR detection, and point cloud obtained by angle measurement, after the echo signal is processed by 1D-FFT (such as range FFT), time correlation analysis may be performed based on a complex echo data, then a human target discrimination is achieved, and then location determination may be performed by combining point cloud information, etc., so as to ultimately achieve the purpose of in-cabin target detection. For example, when target detection is realized by performing region determination based on single-frame fast-slow time processing, CFAR detection, and point cloud obtained by angle measurement, after the echo signal is processed by 1D-FFT (such as range FFT), time correlation analysis may be performed based on a complex echo data, then a human target discrimination is achieved, and then location determination may be performed by combining point cloud information, etc., so as to ultimately achieve the purpose of in-cabin target detection.

In some optional embodiments of the present application, another scheme of target detection and/or classification, i.e., a scheme of realizing target detection in a closed space region such as in a cabin, indoor, factory building, etc., through an inter-frame integration mode is further proposed, so as to effectively improve the detection rate, reduce the number of false alarm targets, and further greatly improve the accuracy of angle estimation and the like, thereby enabling special targets or weak targets such as infants and young children in the cabin to be accurately detected to implement applications such as CPD (Child Presence Detection) and SBR (Safety Belt Reminder). In the implementation process, accurate detection of targets in the cabin may be achieved based on one scheme or a combination of at least two schemes such as multi-frame joint processing technology, and detection, positioning and classification technology of deep learning.

The multi-frame joint processing scheme may be: after obtaining a range-Doppler spectrum by performing sliding window FFT on multi-frame data, or after using FIR (Finite Impulse Response) or other complex time-frequency transform processing, performing processing operations such as CFAR and DOA (Direction Of Arrival estimation) on the region of interest, and the processing operations may be combined with a set region determination logic and region parameters and the like to realize the detection and positioning operation of living targets such as adults, children, pets, or other non-living targets. The CFAR may be an NR-CFAR of the Doppler dimension, or an RD-CFAR, or a DAE (Doppler-Azimuth-Elevation)-CFAR, or the like. At the same time, after CFAR and DoA, post-processing such as clustering, false alarm suppression, and multiple processing of point cloud correlation may be used. A region parameter may be determined by using at least one scheme or a combination of at least two schemes of performing a clustering operation on the point cloud detected after a certain sliding window multi-frame processing, performing outlier detection and culling operation on the point cloud detected after the certain sliding window multi-frame processing, and the like.

Herein, when applied to a closed space region or a relatively closed space region, the space region can be divided in advance, and then the definition and detection of different regions of interest (divided regions) can be realized. For example, when detecting targets in a cabin of a vehicle, the region monitored by a radar may be divided into seat sections and aisle sections, etc., and then corresponding parameter types and thresholds may be preset for different types of regions, and corresponding processing steps may be combined, and then accurate detection of targets of interest in specific regions can be achieved.

In some optional embodiments, for a family vehicle, the space region in the vehicle may generally be simply divided into a head space, a rear space and a trunk space, and if the rear space is a key monitoring region, the rear space may be continuously divided into a seat section and an aisle section, etc. At the same time, the seat section may also be divided into a corresponding number of seat section units based on the seats. Similarly, the aisle section may also be divided into a corresponding number of aisle section units corresponding to the above seat section units, for example, for a five-seat family vehicle, for three seats in the rear space, three seat section units and three corresponding aisle section units may be divided. At the same time, corresponding parameter types and thresholds and the like may be preset for different types of regions (or sections or section bins), and adaptive signal data processing methods and steps may be adopted, to achieve accurate detection of targets of interest (specific targets) in regions such as specific sections or section bins. Here, adjacent section bins may have partial overlapping regions, or may be adjacent to each other or spaced apart by a gap of a predetermined width.

When combining the detection, positioning and classification scheme of deep learning, the implementation may be as follows: after performing 1D-FFT (e.g. range FFT) processing on the echo signal, continuing multi-frame sliding window FFT, and performing 2D-DBF processing of N points to form an N-channel RD (Range-Doppler) spectrum, and then using a deep learning model to perform monitoring, positioning and classification operations based on the N-channel RD spectrum; or, after performing 1D-FFT processing on the echo signal, continuing 2D-DBF processing of N points, then performing monitoring, positioning and classification operations based on the N-channel RD spectrum by using a deep learning model; or, performing 1D-FFT processing on the echo signal at first, then continuing 2D-DBF processing of N points, after continuing multi-frame sliding window FFT to form an N-channel RD spectrum, using the deep learning model to perform monitoring, positioning and classification operations based on the N-channel RD spectrum. Herein, the above N may be a number of the section bins; for example, for a rear section with three seats in the rear row, it is correspondingly divided into three seat section units and three corresponding aisle section units, then N may be equal to 6 at this time.

Based on the above scheme, after testing with actual collected data, it can effectively achieve a high detection rate, as well as extremely low missed detection rate and false alarm rate for the application scenarios of the top-mounted radar installation method. For example, when detecting in-cabin targets, a detection rate of at least 99%, a missed detection rate of at most 0.5%, and a false alarm rate of at most 1% can be achieved.

Hereinafter, taking the frame-level Fourier transform as an example, the technical schemes of the present application will be described in detail with reference to the drawings, that is, after the chirp in the echo signal is Fourier transformed in the fast time dimension, at least two frames of Fourier transform data in the fast time dimension are integrated, and then the at least two frames of data are Fourier transformed in the frame dimension, a Range-Doppler (RD) spectrum is obtained to perform estimation of a target range and/or velocity based on the RD spectrum.

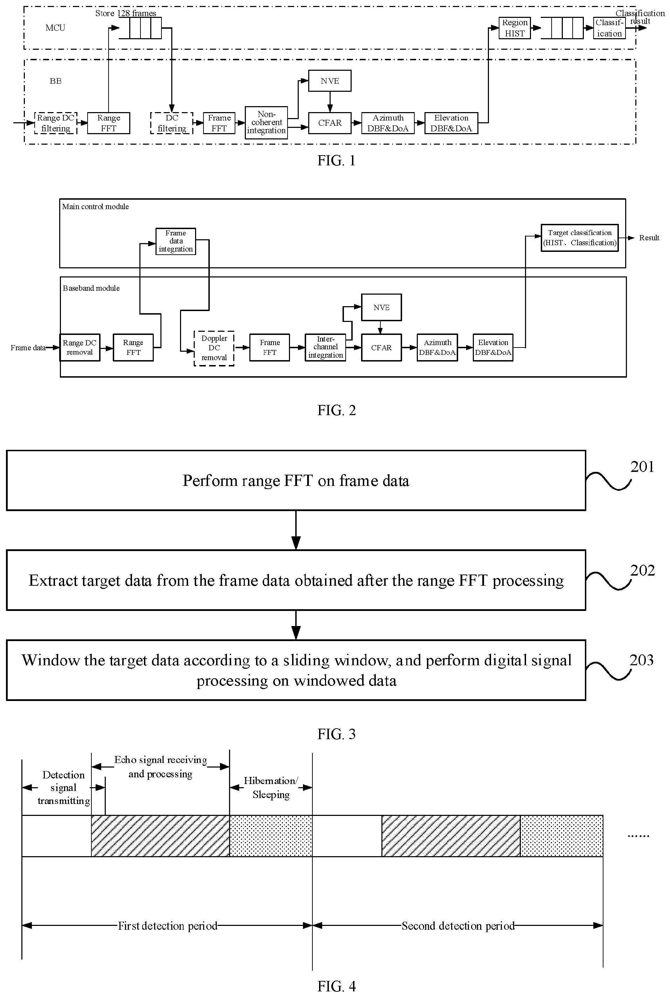

FIG. 1 is a schematic flow diagram of implementing target detection based on SISO-combine according to an embodiment of the present application. As shown in FIG. 1, after operations such as ADC (analog-to-digital) conversion and sampling processing are performed on the echo signal, and after Range DC removal and Range FFT (which may also be represented as 1D-FFT or fast-time FFT) are sequentially performed, multi-frame integration (for example, storing 128 frames) is performed, and Doppler DC removal, inter-frame FFT, Non-coherent integration are continued, and then constant false alarm rate detection (e.g. peak-based CFAR (detection with peak selection)) is performed based on noise obtained by noise estimation (e.g. using a noise variance estimator); finally, angle detection, target classification and the like are performed to obtain a range, velocity and/or angle information and the like of the target; for example, Azimut estimation and Elevation estimation may be performed first based on the CFAR result, for example, implemented by technologies such as DBF (digital beam forming) and DoA estimation (direction of arrival estimation).

In some optional embodiments, for an electromagnetic wave sensor having a BB (Baseband) unit and an MCU (Microcontroller Unit), operations such as accumulating frame data (storing 128 frames) and target classification (Region HIST, classification) may be performed in the MCU module based on the flow chart of target detection shown in FIG. 1, and Range DC removal and Doppler DC removal may be performed in the BB module or the MCU module. In the flow shown in FIG. 1, DC filtering of downlink ADC data in the BB module is taken as an example to illustrate.

As shown in FIG. 1, after 1D-FFT processing is performed in the BB module to obtain range dimension information, the 1D-FFT data may be cached into the MCU module, and when the data is cached to a preset amount of data (such as 128, 56, or 32 frames), the data cached to a preset number of frames may be DC removed and subjected to frame-dimension FFT in the frame dimension to obtain an RD (Range-Doppler) spectrum.

When the system contains multiple channels, as shown in FIG. 1, non-coherent integration may also be performed on the multi-channel RD spectrum, and an NVE module may be used to estimate a noise floor of each range bin in the integrated data. For example, the noise floor estimation may be performed based on a preset formula, and subsequent NR (Noise Reference)-CFAR processing may be performed based on the value of the noise floor estimation. The above preset formula may be: nᵢ= min (nᵢ, α * n_{g}), nᵢ is a noise floor estimation of an i-th range bin, n_{g} is a noise floor estimation of a last range bin of interest, and α is a coefficient which may be set and updated based on requirements or engineering data, experience, etc.

For angle estimation, as shown in FIG. 1, azimuth directional beamforming (DBF) and DoA, and elevation DBF and DoA may be performed for CFAR detection point data, respectively, to obtain azimuth and elevation estimation data of each CFAR detection point. In addition, the target points detected in each frame (or a preset amount of data, i.e., each time) may be counted according to a predefined (or divided) region bin design, and the counting result may be determined according to a preset rule to obtain whether a physical target of interest (e.g. an adult, a child, an infant or a pet, etc.) exists in the present processing, and determine the location region of the physical target of interest.

In the above embodiment, the frame-level FFT is used instead of a traditional chirp-level Doppler FFT to determine velocity of the target to achieve accurate detection of the target of interest, and a sliding window FFT processing may be performed on multiple frames to further improve the update frequency of the result, thereby improving the real-time performance of the system. At the same time, it is also possible to reduce the amount of data processed by processing only ranges and/or Doppler regions of interest. In addition, when estimating a background noise, a minimum value may be taken based on an estimation of a current range bin combined with an estimation of a last range bin of interest to obtain a background noise, such operation is more suitable for application scenarios in relatively closed environments such as a cabin of a vehicle. When judging the region logic, by counting target points detected in each frame according to a predefined zone design, and determining the counting result according to a preset rule, it is possible to accurately determine whether there is a target in each predefined zone.

FIG. 2 is an example of digital signal processing implemented by another target detection method applied to SISO-combine radar.

After the radar receives an echo signal through an antenna and performs analog-to-digital conversion, sampling and other processing, a digital signal will be obtained, and the digital signal will be processed by a main control module (for example, may be implemented based on a Microcontroller Unit (MCU), etc.) and a baseband (BB) module (for example, may be implemented based on implementing a baseband chip or the like) as shown in FIG. 2 (using a target detection of Frequency Modulated Continuous Wave (FMCW) radar as an example) to realize target detection. For example, in FIG. 2, the digital signal is sequentially subjected to Range DC removal and Range FFT (which may also be expressed as 1D-FFT or fast-time FFT) on the baseband module, and then the baseband module may further process the data, for example, merging a plurality of chirp data in a frame by complex averaging, or the like, but of course, no further processing may be performed. After that, the signal processed by the baseband module will be sent to the main control module and integrated in multiple frames. When the data is integrated to a preset number of frames, e.g. integrated to 128 frames, the main control module will transfer the integrated data in multiple frames from the static random access memory (SRAM) of the CPU to the baseband module, and continue to perform Doppler DC removal, inter-frame FFT, inter-channel integration (Non-coherent integration processing, channel integration, etc.) based on the baseband module. Then, in the baseband module, constant false alarm rate detection (e.g. peak-based CFAR (detection with peak selection)) is performed based on the noise obtained by noise estimation (e.g. using a noise variance estimator), and finally angle detection and target classification and the like are performed to obtain a range, velocity and/or angle information and the like of the target. Herein, when performing angle detection, Azimut estimation and Elevation estimation may be performed first based on CFAR results, for example, may be implemented through technologies such as Digital Beam Forming (DBF) and direction of arrival estimation.

Hereinafter, an implementation of the digital signal processing shown in FIG. 2 will be described.

The range DC removal is for data corresponding to each chirp in each frame of data. At this time, the range DC removal is: the data collected for each chirp and each receive (RX) channel is averaged along the fast-time dimension, and then for all sampling points of each RX channel, DC component of a sampling point is subtracted.

The range FFT may be implemented by windowing data after the range DC removal and then performing 1D-FFT.

It should be emphasized that in the process of target detection, each chirp data in each frame of data obtained by the radar performing ADC or sampling processing or the like based on an echo signal is sent to the main control module through the above operation. Herein, sending the data to the main control module is implemented by transferring the data from Direct Memory Access (DMA) to the CPU SRAM.

The Doppler DC removal may be implemented by calculating a complex average along the frame dimension of the multi-frame data integrated on the main control module (that is, averaging the data of different frames on the same range bin of the same channel), obtaining the average value of different ranges of different channels as a DC component of the corresponding range bin of the corresponding channel, and subtracting the respective DC component for each range bin of each channel.

The inter-frame FFT may be implemented by windowing the data after Doppler DC removal along the frame dimension and performing 2D-FFT along the frame dimension, and at this time, the range-Doppler information of each transmitting and receiving channel will be obtained.

Inter-channel integration, taking non-coherent integration as an example, may be realized by the following expression: $P \left(r, v\right) = {\sum }_{t = 0}^{{N}_{t}} {\sum }_{a = 0}^{{N}_{r}} {\left|s\left(r, t, a, v\right)\right|}^{2} / {N}_{t} / {N}_{r} ;$ or, $A \left(r, v\right) = {\sum }_{t = 0}^{{N}_{t}} {\sum }_{a = 0}^{{N}_{r}} \left|s\left(r, t, a, v\right)\right| / {N}_{t} / {N}_{r} ,$

Herein, P(r, v) and *A*(r, v) are respectively power and amplitude of a r-th range bin and a *v-th* Doppler bin, *s*(*r, t, a, v*) is a complex value obtained by Frame Fourier transforming a signal echo of the r-th range bin and the v-th Doppler bin on the t-th transmission channel and the *a*-th receiving channel.

Constant false alarm rate detection based on a noise floor obtained by noise estimation has been described in the existing CFAR technology, the detailed description thereof will not be repeated here.

The angle detection may be implemented by performing Azimuth Digital Beam Forming (DBF) and DOA, and Elevation DBF and DOA on the target points obtained by CFAR. Herein, DBF and DOA have been described in the existing DBF and DOA technologies, the detailed description thereof will not be repeated here.

The target classification may be implemented by counting the target points detected in each frame (or a preset amount of data, i.e., each time) according to the preset (or divided) region bin design, and may be implemented further by determining the counting result according to a preset rule to obtain whether there is a physical target of interest (e.g. an adult, a child, an infant or a pet, etc.) in this processing, and determining the location region of the physical target of interest.

It should be noted that for a radar with a baseband module and a main control module, FIG. 2 is only an example, that is, operations such as accumulating frame data and target classification (which may be implemented using Region HIST, classification, etc.) are performed in the main control module, and other operations such as range DC removal and Doppler DC removal are performed in the baseband module. In other embodiments, accumulating frame data may be performed in the baseband module, Doppler DC removal and the like may be performed in the main control module.

It should further be noted that in FIG. 2, only 128 frames of data are cached as an example, and 56 frames of data, 32 frames, etc. may be cached in other embodiments, but this is not limited in the embodiments of the present application, and it may be determined according to requirements or hardware support capabilities, etc., so that the cached multi-frame data will continue to be subjected to Doppler DC removal and inter-frame FFT, to obtain a Range-Doppler (RD) spectrum.

In the flow shown in FIG. 2, some steps such as range DC removal or range DC removal or windowing may be skipped; some steps may be simplified, for example, non-coherent integration may be skipped and CFAR detection is performed on only a certain one of the channels, or for example, only some channels are selected for non-coherent integration and CFAR detection is performed on the selected channels only.

In order to facilitate those skilled in the art to better understand the flow shown in FIG. 2, the flow of different target detection methods will be described hereinafter.

In some embodiments, the flow of the target detection method is shown in FIG. 3, and includes the following steps 201 to 203.

Step 201: performing range FFT on frame data.

Step 202: extracting target data from the frame data obtained after the range FFT processing.

Step 203: windowing the target data according to a sliding window, and performing digital signal processing on windowed data.

Herein, correspondingly, a duration of sliding window is commensurate with an order of magnitude of a target's periodic motion cycle.

In order to facilitate those skilled in the art to better understand the target detection method described in the embodiment shown in FIG. 3, the steps thereof will be explained below.

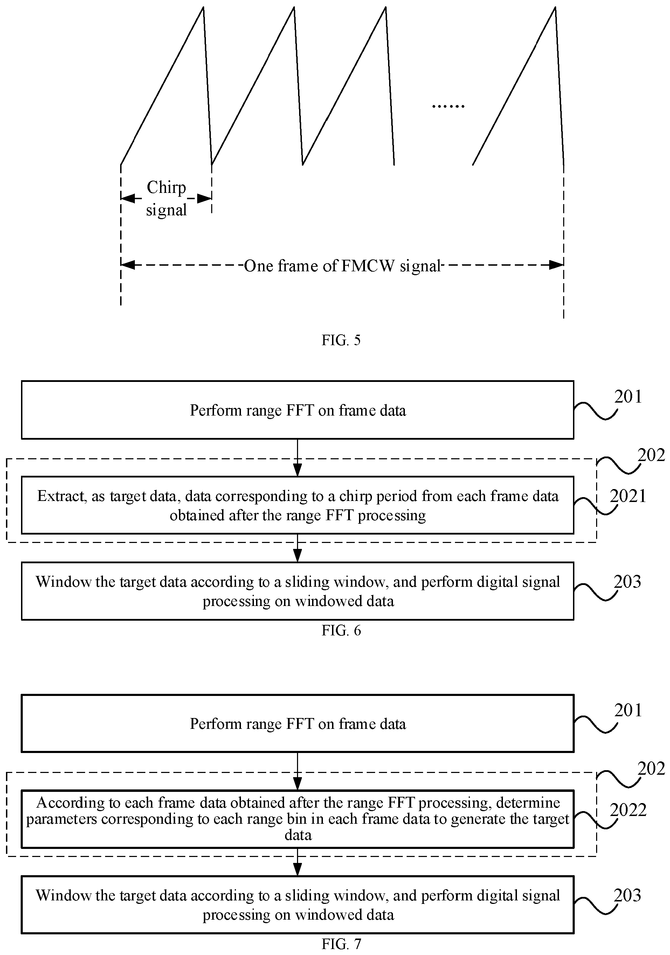

In step 201, frame data refers to data corresponding to an echo signal formed after a frame of detection signal is reflected by a target. It can be understood that the target detection behavior performed by radar usually has a certain period as shown in FIG. 4. Within a detection period, the radar first sends a frame of detection signal. Taking the detection of frequency modulated continuous wave (FMCW) radar as an example, as shown in FIG. 5, a frame of detection signal will be composed of multiple chirp signals (no idle period between chirp signals shown in FIG. 5 is just an example, and in some cases, there may be a certain idle period between chirp signals). At the same time as the detection signal starts to be transmitted, the radar is ready to start receiving the echo signal of the detection signal reflected by the target, and the echo signal corresponding to a frame of detection signal will undergo the aforementioned analog-to-digital conversion, range DC removal and other processing to obtain the above frame data. Furthermore, each frame data is subjected to range FFT as described above, and step 201 is implemented, the detailed description thereof will not be repeated here.

In step 202, the target data is not limited in the embodiments of the present application, and the target data may be any data content belonging to corresponding frame data, for example, when storage resources are sufficient and computing power is sufficient, extracting the target data may be extracting all the data. Or, when it is desired to achieve the highest real-time performance of target detection, at least part of the data or data obtained by preprocessing at least part of the data (e.g. data obtained after preprocessing operations such as complex averaging, complex weighted averaging, complex summing, etc.) or the like may be extracted, and the target data may be extracted according to requirements and hardware conditions, etc. For convenience of understanding, different implementations of extracting a portion of data as target data will be described below.

In some embodiments, as shown in FIG. 6, the target data is extracted from the frame data obtained after the range FFT processing by the following step 2021.

Step 2021: extracting, as target data, data corresponding to a period of any chirp or a chirp of specified index (e.g., the second chirp of each frame) from each frame data obtained after the range FFT processing; here, when it is confirmed that the data corresponding to a predetermined rule chirp period is adopted as the target data, the rules of the selected chirp in each frame are the same.

That is to say, the data corresponding to a chirp period is used as a representation of frame data, which makes the processing of target data simple and easy to implement, and the selected data is more real, which can carry out target detection more efficiently, and is conducive to improving the real-time performance of target detection.

In some embodiments, as shown in FIG. 7, extracting the target data from the frame data obtained after the range FFT processing may also be implemented by the following step 2022.

Step 2022: according to each frame data obtained after the range FFT processing, determining parameters corresponding to each range bin in each frame data to generate the target data.

That is, a plurality of data of each range bin in the frame data are compressed into corresponding parameters, so that the overall characteristics of the frame data can be retained, such that the information which can be referred to when subsequently detecting the target according to the target data is more comprehensive, and the obtained result is more accurate.

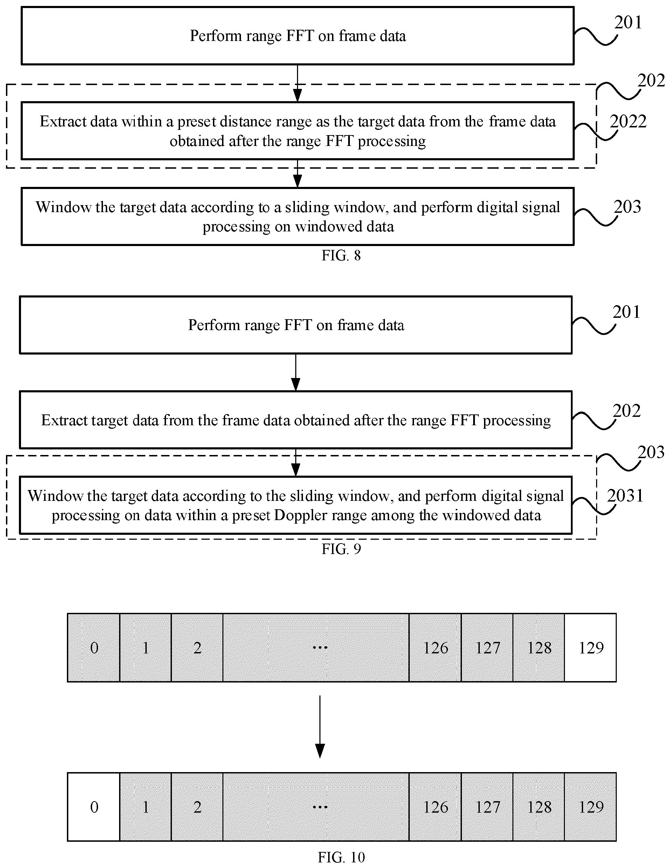

Of course, the above embodiments are only examples provided to reduce the resources occupied by the target data, and in some embodiments, the target data may also be extracted in consideration of validity of the data. For example, radar signals can usually cover a large range, but not all regions covered by radar are regions which need attention. Processing the data corresponding to such regions will cause a waste of resources. Therefore, in some embodiments, as shown in FIG. 8, extracting the target data from the frame data obtained after the range FFT processing may also be realized by the following step 2023.

Step 2023: extracting data within a preset distance range, as the target data, from the frame data obtained after the range FFT processing.

That is, the target data is extracted from frame data according to the range of interest (represented by a preset distance range), so that accurate target detection information can be retained, and the storage resources, computing resources, etc. occupied by the target data can be reduced, thus balancing the accuracy and real-time performance of target detection.

It should be noted that, in addition to the retention of the data within the range of interest implemented by the method shown in FIG. 8, in some embodiments, the retention may be implemented by windowing the target data according to the sliding window as shown in FIG. 9, and digital signal processing is performed on the windowed data by the following step 2031.

Step 2031: windowing the target data according to the sliding window, and performing digital signal processing on the data within a preset Doppler range among the windowed data.

It can also achieve the effect that can be achieved by the embodiment shown in FIG. 8, it is just that the dimensions of the two data are different, one is a range dimension and the other is a Doppler dimension, so the selection method of the range of interest is different. Of course, it is also possible to extract the target data in the range dimension as in the embodiment shown in FIG. 7, and extract the data in the Doppler dimension as in the embodiment shown in FIG. 9 for digital signal processing, etc., the detailed description thereof will not be repeated here.

Of course, the above descriptions are only examples of the implementation of step 202, and in some embodiments, it can also be implemented by other methods, such as the range bins may be modulo in pairs to get a larger range unit (similar to max-pooling) for target data extracting, the detailed description thereof will not be repeated here.

In step 203, the method of windowing is not limited, for example, the window may be a sliding window, or the sliding window may be a window of a fixed length or a window of a variable length, the method of windowing will not be listed here. And a sliding window of a fixed length may be described hereinafter as an example for convenience of understanding, but this does not mean that only a sliding window of a fixed length can be used, for example, a window of a variable length may be used, the window of the variable length may change according to a change in a periodic motion such as respiratory rate of the target.

In some embodiments, as shown in FIG. 10, for the 1st to 129th frame data generated after the radar is started, taking the window length of the sliding window as 128 frames as an example, the data obtained by the sliding window for the first time is the 1st to 128th frame data (the data of the shaded portion of the first row in FIG. 10). The data obtained for the second time is the 2nd to 129th frame data (the data of the shaded portion of the second row in FIG. 9), that is, the sliding step length of the sliding window is 1 frame.

Of course, the above is merely an example, and in other embodiments, other parameters such as the window length and sliding step length of the sliding window may be adopted, and will not be listed here.

And, in step 203, the data signal processing is not limited, and it can be understood that the data to be processed and obtained is different for different requirements. For example, in some embodiments, a target detection effect may be achieved when a target point is detected, and in some embodiments, it may be necessary to perform target classification as shown in FIG. 2 to obtain more accurate, more reliable, and more referential targets, etc., which will not be listed here. Hereinafter, the target detection flow illustrated in FIG. 2 will be described as an example, but it does not mean that it is necessary to perform target classification after processing such as CFAR as illustrated in FIG. 2.

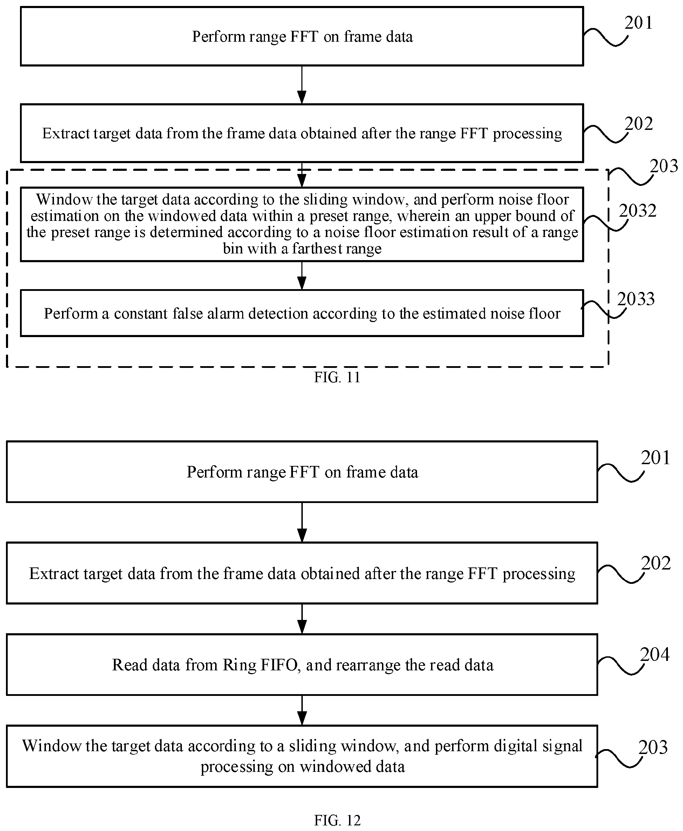

As shown in FIG. 2, the digital signal processing process of target detection usually includes constant false alarm rate detection, a typical constant false alarm rate detection is a constant false alarm rate detection based on noise floor. It can be understood that at this time, the more accurate the estimation of the noise floor is, the better the constant false alarm rate detection effect is, and the more accurate the target detection result is. Therefore, in some embodiments, as shown in FIG. 11, windowing the target data according to the sliding window and performing digital signal processing on the windowed data can be implemented by the following steps 2032 and 2034.

Step 2032: windowing the target data according to the sliding window, and performing a noise floor estimation on the windowed data within a predefined range, wherein an upper bound of the predefined range is determined according to a noise floor estimation result of a range bin with a farthest distance.

Step 2034: performing a constant false alarm rate detection according to the estimated noise floor.

That is, the noise floor estimation is constrained by setting a predefined range, so that the upper bound is constructed according to the noise floor estimation result of the range bin with the farthest distance, which is beneficial to improving the accuracy of noise estimation.

In some examples, performing the noise floor estimation within a predefined range is achieved by the following expression: ${n}_{i} ′ = min \left({n}_{i}, α*{n}_{g}\right) ;$ where nᵢ' is a noise floor estimation result of an i-th range bin within the predefined range, nᵢ is an initial noise floor estimation result of the i-th range bin, α is a preset parameter, α ≥ 1, n_{g} is an initial noise floor estimation result of a range bin with a farthest distance.

It should be noted that, the preset parameter α may be set and updated based on requirements, engineering data, experience, and the like.

It should be further noted that, the above is only an exemplary description of the noise floor estimation. In some embodiments, an initial noise floor estimation result of the i-th range bin may be directly selected as the noise floor result used in CFAR, or the minimum value, maximum value, quantile, average value, median value, etc. of initial noise floor estimation results of last few regions of interest may be further selected as the noise floor estimation, or only some Doppler bins of a certain range bin instead of all Doppler bins or the like may be selected when estimating the noise floor, here the methods of noise floor estimation are not listed.

In addition, the radar shown in FIG. 2 is a SISO-combine system, and when the radar contains multiple channels, a multi-channel RD spectrum may further be integrated between channels, e.g. by Non-coherent integration, correlation integration, etc., and the NVE module may be used to estimate the noise floor of each range bin in the integrated data, wherein the noise floor estimation method may adopt any of the above noise floor estimation schemes, the detailed description thereof will not be repeated here.

In step 203, when the data is windowed, the data has a certain order. If the order is disturbed, the temporal correlation of the data is destroyed, and the motion integrated for a certain period of time may no longer be reflected, which is not conducive to subsequent target detection. As shown in FIG. 2, the data is integrated in the main control module. When a certain amount of data is integrated, the baseband module reads the data and continues to process the data. In this process, the data writing order and the data reading order may be different due to the storage mode. Therefore, in some embodiments, it is necessary to rearrange the data to ensure its correct order. For example, in some embodiments, assuming that the data is cached using Ring FIFO, at this time, as shown in FIG. 12, the target detection method further includes the following step:
step 204: reading data from the Ring FIFO, and rearranging the read data.

Thus subsequent processing efficiency is improved through data rearrangement.

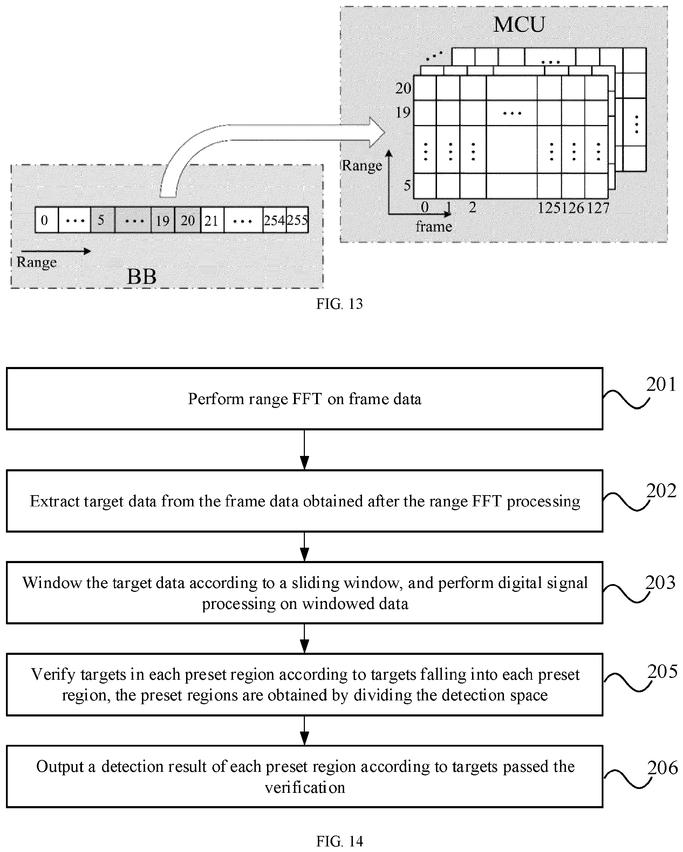

In order to facilitate those skilled in the art to better understand the above-described data storage and rearrangement, the following will be described with reference to FIG. 13.

As shown in FIG. 13, assuming that the data in the range of interest (for example, within the 5th range bin to the 20th range bin) obtained after the baseband module (shown by BB in the FIG.13) performing range FFT on the frame data belongs to the target data, a two-dimensional matrix as shown in FIG. 13 will need to be integrated in the main control module (shown by MCU in the FIG.13), rows of the matrix correspond to frame numbers and columns of the matrix correspond to range bins.

Assuming that the main control module uses Ring FIFO to store the two-dimensional matrix, the storage process may be as follows:
initializing a slice of CPU SRAM with N_{f} columns, each column may store a number of Nᵣ × Nᵣₓ × Nₜₓ, wherein N_{f} is a maximum number of frames designed for inter-frame FFT, and Nᵣ, Nᵣₓ and Nₜₓ are the number of range bins of interest, the number of transmit (Tx) channels and the number of receive (Rx) channels, respectively. The cache may all be initialized to 0 at an initialization, and a FIFO header pointer p_head points to a beginning of the 0th column of the Ring-FIFO.

The corresponding data (of each range bin, each Tx channel, and each Rx channel) is cached in the 0th column of the Ring-FIFO at the 0th frame, and then p_head points to the 1st column.

Similarly, for a k-th frame data (k = 1, 2,..., 126), corresponding data thereof is cached in a k-th column of the Ring-FIFO, and then p_head points to a (k + 1)-th column.

In the 127th frame, the corresponding data is cached in the 127th column of the Ring-FIFO; since all of 128 frames of data have been stored at this time, it is necessary to transfer the data in the Ring-FIFO to the baseband module to execute the aforementioned inter-frame FFT processing. At this time, data from the column to which pointed by the p_head to the 127th column is first transferred, and then data from the 0th column to the column to which pointed by the p_head is transferred. Obviously, at this time, the order of reading data is different from the order of writing, and a recover is needed, i.e., the data needs to be rearranged so that it is continuously distributed according to a frame dimension, thereby improving computing efficiency on the baseband module.

After the transfer is completed, the pointer p_head of the Ring-FIFO points to the 0th column again. Similarly, in the process of new data storage and transfer, the data of the 128th frame is stored in the 0th column of the Ring-FIFO. When transferring, the data of the 1st to 127th frames will be transferred to the baseband module first, and then the data of the 0th column will be transferred to the baseband module, and the following frames will be similar, which will not be repeated here.

It should be noted that, in the above example, the inter-frame processing is performed after all valid data is stored in the Ring-FIFO, and in other embodiments, the data in the Ring-FIFO may be transferred to the baseband module for inter-frame FFT and the like in a similar manner as described above after partial frame data such as data of the 0th frame to the 63th frame is stored, wherein, since at this time, in the 64th column to the 127th column, there are all initial values assigned at the time of initialization, therefore, it may be equivalent to inter-frame FFT in which the baseband module fills zero in front of the data sequence, of course, the above is merely an example, and it does not mean that data of 128 frames must be used for inter-frame FFT, or Ring-FIFO storage must be used, which will not be repeated here.

In addition, still as shown in FIG. 2, if the user wants to obtain a more intuitive and accurate result, at this time, in some embodiments, as shown in FIG. 14, after obtaining the target, the following steps 205-206 are further performed.

Step 205: validating targets within each predefined zone based on targets located therein, wherein the predefined zones are obtained by partitioning the surveillance area.

Step 206: disseminating the detection outcomes for each predefined zone based on validated targets.

That is, considering a situation that for a target with a certain volume and occupying a certain space, usually multiple target points are detected, the currently detected target is continuously used in combination with region division to perform target verification to improve the detection result, and based on division of the detection space in an application scenario, the detection result of each predefined zone is outputted, so that the target will show a distribution in the predefined zones, which is more intuitive and accurate, more conducive to the user's decision-making based on the output result, and the user experience is better.

In order to facilitate those skilled in the art to better understand the embodiment shown in FIG. 14, the steps thereof will be explained below.

In step 205, the detection space and the predefined zone are not limited, and may be different according to different application scenarios, application requirements, and the like. For example, in an application scenario of vehicle-mounted radar, the detection space may be inside the vehicle. At this time, the predefined zone at least includes any of the following regions: a region corresponding to a seat and a region corresponding to an aisle (floor mat), so that objects such as drivers or adults can better perceive the situation inside the vehicle. For another example, in a factory work scenario, the detection space may be a factory building, and at this time, the predefined zone may include workstation of each employee, so as to avoid production risks and the like caused by employees not being able to monitor working conditions of machines due to employees not being at their workstations. The detailed description thereof will not be repeated here. For the convenience of understanding, the above space inside the vehicle will be used as an example for illustration hereinafter, but this does not mean that the corresponding scheme can only be implemented inside the vehicle.

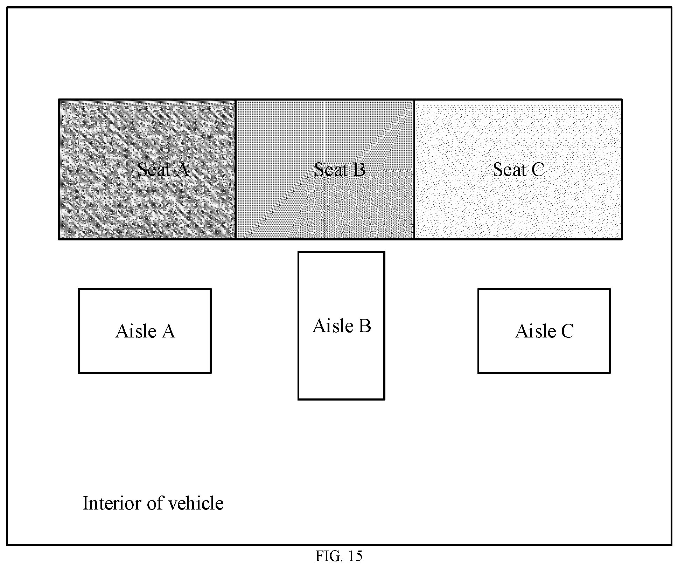

Taking the interior of the vehicle as the detection space as an example, the interior space of the vehicle is abstracted into a coordinate system as shown in FIG. 15, wherein the abscissa of the coordinate system represents the azimuth with respect to the radar installed in the vehicle, and the abscissa of the coordinate system represents the elevation with respect to the radar installed in the vehicle. At this time, the interior of the vehicle is divided into six predefined zones including three rear seats and three aisles in front of the three seats, i.e., different filling regions as shown in FIG. 15. Herein, according to different requirements, as shown in FIG. 15, different predefined zones may overlap with each other or may not overlap. Some regions in the vehicle may belong to a plurality of predefined zones at the same time, or may not belong to any region.

It should be noted that FIG. 15 is only an example of an abstract method of detecting space, and in some embodiments, the detection region may be abstracted from all or a certain dimension of range, azimuth, and elevation, or all or a certain dimension along the x-y-z dimension of the rectangular coordinate system, and further divided into predefined zones, the detailed description thereof will not be repeated here.

In step 205, the verification method is not limited, and for example, the verification may be performed according to the number, ratio, and the like of the target points.

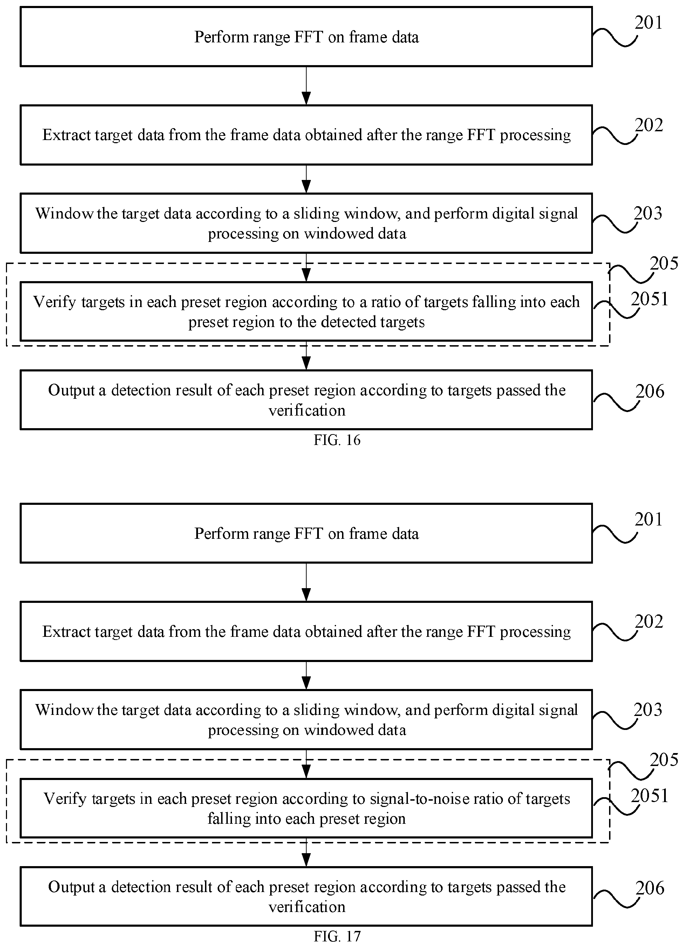

For example, in some embodiments, as shown in FIG. 16, verifying targets of each predefined zone according to targets falling into each predefined zone may be implemented by the following step 2051.

Step 2051: verifying targets in each predefined zone according to a ratio of targets falling into each predefined zone to the detected targets.

For another example, in some embodiments, as shown in FIG. 17, verifying targets of each predefined zone according to targets falling into each predefined zone may be implemented by the following step 2052.

Step 2052: verifying targets in each predefined zone according to signal-to-noise ratio of targets falling into each predefined zone.

Of course, the above are merely examples, and in some cases, targets at other locations may be verified based on the falling situation of target points at locations where targets are known, such as the driver's seat, and the detailed description thereof will not be repeated here.

In step 206, the output method is not limited, and the detection result of each predefined zone may be directly output in a point cloud, or the divided predefined zones may be simultaneously presented with the output of the point cloud of each predefined zone, etc., the detailed description thereof will not be repeated here.

In order to facilitate those skilled in the art to better understand the above embodiments, ratio verification will be described below as an example.

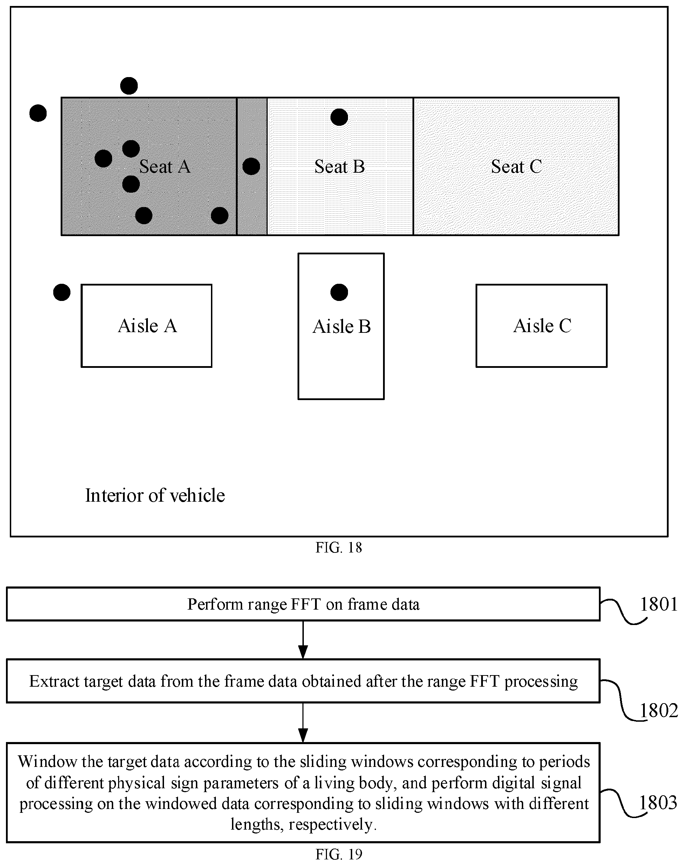

Assume that a total of 11 target points are detected in a certain target detection process, and the division of the predefined zones is shown in FIG. 15, at the same time, the distribution of the target points in the predefined zones is shown in FIG. 18, i.e., the region seat A (6 target points), seat B (2 target points), seat C (0 target points), aisle A (0 target points), aisle B (1 target point), and aisle C (0 target points), wherein the target points are represented by solid circles in FIG. 18.

At this time, the algorithm obtained according to the above embodiment will be as follows:
each flag bit flag_region_i that identifies whether there are people in six regions is initialized to 0, i.e., no one exists (i = 0, 1,..., 5);
if the total number of valid targets total_valid_tgt_num is 0, it is determined that no one is present in the vehicle and the process is terminated, otherwise, the following operations are performed;
traversing all regions, for region i, if a statistical value tgt_num_region_i of the number of valid targets belonging to the region exceeds 25% of the total number of valid targets total_valid_tgt _num, setting the modified region i flag bit flag_region_i to 1;
if a certain region exists, and if its flag_region_i is 1, flag_region_empty is set to 0, i.e., there is a person in the vehicle;
traversing the seat region, if a flag_region_i of any seat region is 1, it is determined that there is no one in the corresponding aisle region (for example, if it is determined that there is a person in seat A, then it is determined that there is no one in aisle A);
output a result.

At this time, the output result will be as shown in FIG. 18, six target points are displayed in region seat A, and there is no one in the remaining predefined zones.

It should be noted that the threshold value of 25% in the above example is merely an example, and other threshold values or other judging conditions may be adopted in other embodiments, and the detailed description thereof will not be repeated here.

It is also understood that in the process of detection, it may be necessary to combine different periodic motions for observation, so that the target detection result can be obtained more accurately. Based on this, in some embodiments, as shown in FIG. 19, the target detection method includes the following steps 1801 to 1803.

Step 1801: performing range FFT on frame data.

Step 1802: extracting target data from the frame data obtained after the range FFT processing.

Step 1803: windowing the target data according to the sliding windows corresponding to periods of various physiological parameters of a living subject, and performing digital signal processing on the windowed data corresponding to sliding windows of different lengths, respectively.

Herein, the periods of the physiological parameters associated with each sliding window vary, and the duration of each sliding window is commensurate with the period of its corresponding physiological parameter in terms order of magnitude.

Herein, step 1801 and step 1802 are substantially the same as step 201 and step 202, except that one of the subsequent step 1803 and step 203 detects based on a plurality of different periodic motions, and the other only describes one periodic motion. It can be understood that when detecting based on a plurality of different periodic motions, the data continues to integrate after the integration of data with a shorter period is completed, and the processing of the data is completed until the integration of data with a longer period is completed. For example, assuming that one periodic motion corresponds to 32 frames and another periodic motion corresponds to 128 frames, both of which need to be observed, then in the process of target detection, a detection result is outputted starting from the completion of the target data acquisition corresponding to the 32nd frame data, and a new detection result is outputted after the target data corresponding to the 33rd frame data is acquired... until the target data acquisition corresponding to the 128th frame data is completed, two detection results (one is the detection result corresponding to data of 32 frames, and the other is the detection result corresponding to data of 128 frames) will be outputted, or a result obtained after comprehensive processing of the two results will be outputted.

In this way, by acquiring the periods of physiological parameters of living subjects, a length of the sliding window can be adjusted in time, so as to better detect living subject targets and better monitor the state of living subjects (such as children, pets, etc.), and so as to monitor and perform corresponding processing on the performance of the living subjects in time.

It should be noted that the embodiments of the present application do not limit the physiological parameters. For example, the physiological parameter may include respiration. Further examples of the physiological parameters may include a heartbeat and/or a pulse. Of course, the above are only examples, and other physiological parameters that may be embodied as motion characteristics may also be used under other requirements, each of the other physiological parameters will not be listed here.

Thus, in the above embodiments, the frame-level FFT is adopted instead of the traditional chirp-level Doppler FFT to effectively integrate motion power of the target to achieve a purpose of accurate detection and measurement, and a result update frequency can be further improved by performing sliding window FFT processing on multiple frames; through post-processing methods such as regional statistics on the target points detected by the radar, determining whether there is someone in each region is realized.

In the above embodiments, the frame-level FFT is used instead of the traditional chirp-level Doppler FFT to determine velocity of the target to achieve accurate detection of the target of interest, and a sliding window FFT processing may be performed on multiple frames to further improve the update frequency of the result, thereby improving the real-time performance of the system. At the same time, it is also possible to reduce the amount of data processed by processing only ranges and/or Doppler regions of interest. In addition, when estimating a background noise, a minimum value may be taken based on an estimation of a current range bin combined with an estimation of a last range bin of interest to obtain a background noise, such operation is more suitable for application scenarios in relatively closed environments such as a cabin of a vehicle. When judging the region logic, by counting target points detected in each frame according to a predefined zone design, and determining the counting result according to a preset rule, it is possible to accurately determine whether there is a target in each predefined zone.

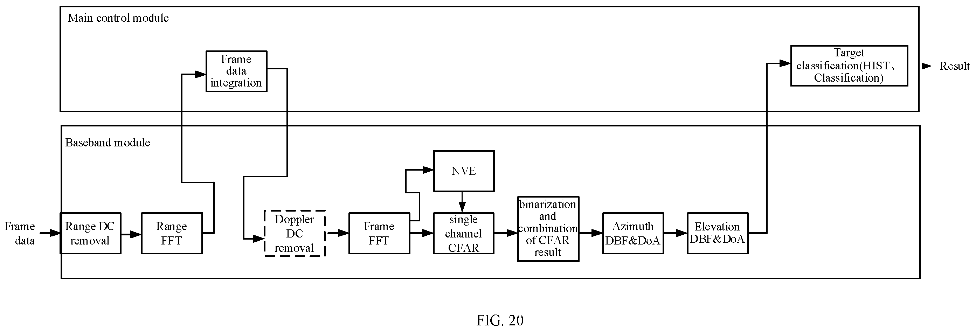

FIG. 20 is a schematic flow diagram of implementing target detection based on per-channel in an embodiment of the present application. As shown in FIG. 20, based on the flow structure shown in FIG. 1, which shows a scheme of non-coherently integrating multi-channel RD spectrum into a single-channel data for CFAR, in the present embodiment, after CFAR processing is performed on all channels one by one, binary integration in channel domain processing is continued, and then horizontal/azimuth and elevation estimation are performed. For example, for a system having M channels, after multiple channels are subjected to inter-frame FFT as shown in FIG. 1, and after multiple channels undergo CFAR processing respectively to obtain M bit masks, each element thereof may be of bool type, whether there is a target in each range bin and/or Doppler bin is determined (if 1, it is defined as existence, if 0, it is defined as non-existence). At the same time, the M bit masks may be integrated, where a range of each element may be defined as 0 ~ (M-1). Subsequently, the integrated mask may be detected a second time, that is, each element of the integrated mask may be compared with a preset threshold M0. If a value xᵢ (e.g. a certain element of the integrated mask) of a bin to be detected satisfies xᵢ > M0, it can output that there is a target in the bin to be detected. If xᵢ≤ M0, then it can output that there is no target in the bin to be detected. Herein xᵢ, M, M0 are positive integers.

In the flow of the detection method shown in FIG. 20, because each channel is used to perform CFAR processing separately, the performance loss of channel imbalance can be effectively reduced, and it has better robustness in practical applications.

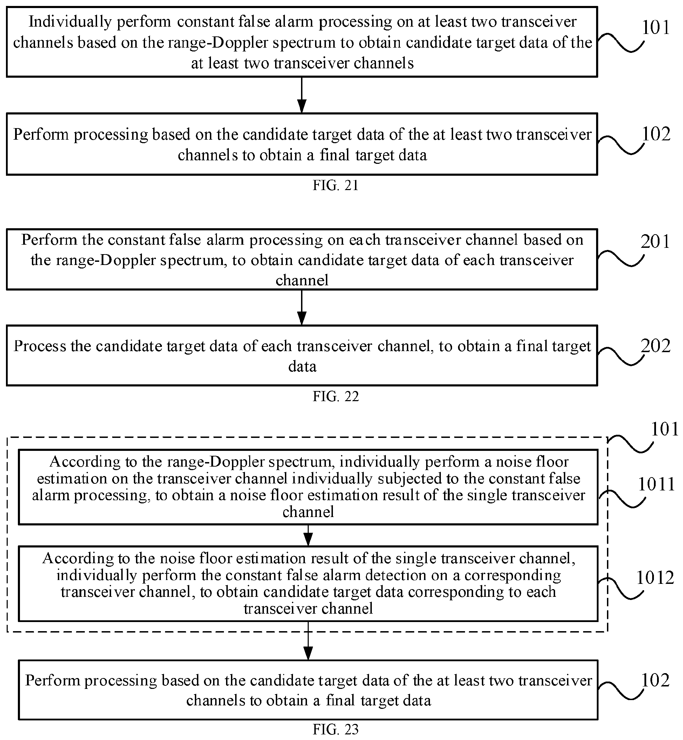

An embodiment of the present disclosure further provides another target detection method, as shown in FIG. 21, including the following steps 101 and 102.

Step 101: individually performing constant false alarm rate processing on at least two transceiver channels based on the range-Doppler spectrum to obtain candidate target data of the at least two transceiver channels.

Step 102: performing processing based on the candidate target data of the at least two transceiver channels to obtain a final target data.

In this way, at least two transceiver channels individually undergo constant false alarm rate detection, so that the results of the at least two transceiver channels do not interfere with each other and do not affect other channels. Therefore, even if there is abnormal channel or abnormal data in the at least two transceiver channels, the processing of other normal channels will not be affected, and then candidate target data of the at least two transceiver channels are processed to obtain the final target data, which supports the detection of the two transceiver channels, not only guarantees the normal processing of the abnormal channel or the channel to which the abnormal data belongs, but also avoids the interference of the abnormal channel or the channel to which the abnormal data belongs to the other channel, alleviates the problem of imbalance between the channels, and the problem of different delays and different phases caused by the inconsistent length of wiring from the transceivers to the antenna and inaccurate compensation, and improves the detection accuracy.

In order to help understand the target detection method provided by the above embodiments, the steps thereof will be explained and illustrated below.

In step 101, the number of transceiver channels on which the constant false alarm rate processing is individually performed is not limited, and may be, for example, two, or may be three or five, or all of the transceiver channels, etc.

For example, in some embodiments, constant false alarm rate processing may be performed on all transceiver channels individually, so that interference between each channel is completely avoided and accuracy is improved. At this time, as shown in FIG. 22, the constant false alarm rate processing is performed on at least two transceiver channels based on the range-Doppler spectrum, and is implemented by the following: step 201: performing the constant false alarm rate processing on each transceiver channel based on the range-Doppler spectrum. Correspondingly, processing is performed based on candidate target data of at least two transceiver channels by the following: step 202: processing the candidate target data of each transceiver channel.

Of course, the above is merely an example. In some embodiments, constant false alarm rate detection may be performed on some transceiver channels individually to avoid adverse effects of channels with relatively poor signal-to-noise ratio or serious TRX leakage, and the detailed description thereof will not be repeated here.

In step 101, the method of constant false alarm rate processing on a single transceiver channel is also not limited. For example, in some embodiments, it may be implemented based on the existing constant false alarm rate processing, except that an original data integration of multiple transceiver channels is changed to data integration of a single transceiver channel.

In some embodiments, as shown in FIG. 23, performing constant false alarm rate processing on at least two transceiver channels based on the range-Doppler spectrum may be implemented by the following steps 1011-1012.

Step 1011: according to the range-Doppler spectrum, individually conduting a noise floor estimation for the transceiver channel individually undergoing the constant false alarm rate processing, to obtain a noise floor estimation result of the single transceiver channel.

Step 1012: according to the noise floor estimation result of the single transceiver channel, individually performing the constant false alarm rate detection on a corresponding transceiver channel, to obtain candidate target data corresponding to each transceiver channel.

That is, when the constant false alarm rate processing is performed for a single transceiver channel, the noise floor estimation of the single transceiver channel is performed as an independent whole, and CFAR based on the noise floor estimation is realized based on the noise floor obtained by each estimation.

Herein, in step 1011, the estimation method of the noise floor is also not limited. For example, in some embodiments, according to the range-Doppler spectrum, the noise floor estimation of the transceiver channel undergoing the individual constant false alarm rate processing may be individually performed by the following: according to the range-Doppler spectrum, conducting the noise floor estimation for the transceiver channel undergoing the individual constant false alarm rate processing individually within a predefined range, to obtain the noise floor estimation result of a single transceiver channel; wherein an upper bound of the predefined range is determined according to a noise floor estimation result of a range bin with a farthest distance. That is, the noise floor estimation is constrained by setting a predefined range, so that the upper bound is constructed according to the noise floor estimation result of the range bin with the farthest distance, which is beneficial to improving the accuracy of noise estimation.

In some examples, the noise floor estimation is performed individually for each transceiver channel, which is achieved by the following expression: ${n}_{i} ′ = min \left({n}_{i}, α*{n}_{g}\right) ;$ where nᵢ' is a noise floor estimation result of an i-th range bin of a single transceiver channel within a predefined range, nᵢ is an initial noise floor estimation result of the i-th range bin on a single transceiver channel determined according to the range-Doppler spectrum, α is a preset parameter, α ≥ 1, n_{g} is an initial noise floor estimation result of a range bin with a farthest distance on a single transceiver channel.

The preset parameter α may be set and updated based on, for example, requirements, engineering data, experience, and the like.

It should be further noted that, the above are merely exemplary descriptions of the noise floor estimation. In some embodiments, the initial noise floor estimation result of the i-th range bin on a single transceiver channel may be directly selected as the noise floor result used when performing CFAR processing on the transceiver channel, or a minimum value, maximum value, quantile, average value, median value, etc. of an initial noise floor estimation results of the last few regions of interest on a single transceiver channel may be selected as the noise floor estimation, or only some Doppler bins of a certain range bin instead of all Doppler bins or the like may be selected when estimating the noise floor, here the methods of noise floor estimation are not listed.

In step 1012, the constant false alarm rate detection may be implemented by threshold detection, for example, the noise floor estimation result of a single transceiver channel is composed of the noise floor estimation result of each range bin on a single transceiver channel, and the constant false alarm rate detection result corresponding to a single transceiver channel is composed of the constant false alarm rate detection result of each Doppler bin of each range bin on a single transceiver channel.

According to the noise floor estimation result of a single transceiver channel, the constant false alarm rate detection is individually performed on the corresponding transceiver channel, which is realized by the following expression: $F \left(c, r, v\right) = \left\{\begin{matrix}0 , & if P \left(c, r, v\right) < β n ′ \left(c, r\right) \\ 1 , & if P \left(c, r, v\right) \geq β n ′ \left(c, r\right)\end{matrix},\right.$ wherein F(c, r, v) is a constant false alarm rate detection result of a v-th Doppler bin of the r-th range bin of the c-th channel, P(c, r, v) is echo power on the v-th Doppler bin of the r-th range bin of the c-th channel, *n'*(*c, r*) is a noise floor estimation result of the r-th range bin of the c-th channel, β is a preset parameter, β≥1.

It can be seen from the above expression that, when F(c, r, v) is 1, it indicates that there is a target in the v-th Doppler bin of the r-th range bin, when F(c, r, v) is 0, it indicates that there is no target in the v-th Doppler bin of the r-th range bin. That is to say, the detection result of a single transceiver channel is binarized, so that the detection process is no longer interfered by specific values (especially extreme data), and the detection accuracy is improved.

The above expression is only used to illustrate an example of binarizing the result to obtain candidate target data. In some embodiments, the results may be quantified by a quantization method other than the binarization processing, and the interference of extreme data can be reduced to a certain extent, and it is not necessary to implement by the binary method, and the quantization method will not be listed here.

It should also be noted that the above is only an example of the constant false alarm rate processing of a single transceiver channel provided in combination with CFAR based on noise floor estimation, but it does not mean that the constant false alarm rate processing of a single transceiver channel in an embodiment of the present application can only be implemented as described above, for example, it can be implemented in combination with CA (Cell Averaging)-CFAR, OS-CFAR, GO-CFAR, etc., when implemented, the processing of a plurality of transceiver channels in the original algorithm may be regarded as a single transceiver channel, and the detailed description thereof will not be repeated here.

In step 102, processing candidate target data of at least two transceiver channels may actually be regarded as further performing a target verification again based on candidate target data of the transceiver channel on the basis of performing a target verification based on data of a single transceiver channel in step 101, so that the accuracy and reliability of the target can be improved. Therefore, step 102 may be implemented by the idea of constant false alarm rate detection.

For example, in some embodiments, as shown in FIG. 23, processing candidate target data of each transceiver channel to obtain a final target data may be implemented by the following step:
performing a constant false alarm rate detection according to a currently obtained candidate target data and a preset threshold value to obtain the final target data.

That is, in a case where the conventional constant false alarm rate processing performs threshold determination by the result of non-correlation integration, similar threshold processing is applied to the candidate target data to obtain the final detection result. It is easy to implement and has high efficiency, and it can improve the accuracy of detection, and the real-time performance is good, thus improving the user experience.

At this time, the constant false alarm rate detection is performed according to the currently acquired candidate target data and the preset threshold value, which is realized by the following expression: ${F}_{sum} \left(r, v\right) = {\sum }_{c = 1}^{{N}_{c}} F \left(c, r, v\right) ,$ $F ′ \left(r, v\right) = \left\{\begin{matrix}0 , if {F}_{sum} \left(r, v\right) < {F}_{0} \\ 1 , if {F}_{sum} \left(r, v\right) \geq {F}_{0}\end{matrix},\right.$ wherein F(c, r, v) is a constant false alarm rate detection result of a v-th Doppler bin of a r-th range bin of a c-th channel, N_{c} is a total number of channels, F₀ is a preset threshold, F'(r, v) is a constant false alarm rate detection result of a v-th Doppler bin of a r-th range bin.

That is to say, the candidate target data of the transceiver channel is integrated, and then the final target data is determined by determining whether the integrated result exceeds a preset threshold value, so that the final target data is used to characterize whether a target exists in the v-th Doppler bin of the r-th range bin, thereby avoiding false monitoring and missed detection caused by multi-path and the like, and improving the accuracy and reliability of the target detection.

Of course, the above is only an exemplary description of obtaining the final target data by a method of threshold, and in some embodiments, if the constant false alarm rate detection result of the v-th Doppler bin of the r-th range bin of the c-th channel is expressed by a quantized numerical value, then the acquisition of the final target data may be judged by determining on how many channels the constant false alarm rate detection result of the v-th Doppler bin of the r-th range bin exceeds a preset value, etc.; or, the above integration process is replaced with a process such as weighted summation or averaging, the detailed description thereof will not be repeated here.

It should be noted that the above embodiments only limit the target verification in the target detection process (that is, obtaining a result similar to the existing CFAR process), and do not limit other data processing processes in the target detection process. It can be understood that the embodiments shown in FIG. 21 to FIG. 23 may be further implemented in combination with other data processing implementations, for example, in combination with a multi-frame joint processing scheme.

In order to facilitate understanding of the above target detection scheme and the multi-frame joint processing scheme, the multi-frame joint processing scheme will be described below first.

As shown in FIG. 2, the radar (taking Frequency Modulated Continuous Wave (FMCW) radar as an example) receives the echo signal through the antenna and performs analog-to-digital conversion, sampling and other processing to obtain the digital signal, and the digital signal will be processed by a main control module (for example, may be implemented based on a Microcontroller Unit (MCU), etc.) and a baseband (BB) module (for example, may be implemented based on implementing a baseband chip or the like) as shown in FIG. 2 to realize target detection. For example, in FIG. 2, the digital signal will be sequentially subjected to Range DC removal and Range FFT (which may also be represented as 1D-FFT or fast-time FFT) on the baseband module. After that, the signal processed by the baseband module will be sent to the main control module and integrated in multiple frames. When the data is integrated to a preset number of frames, e.g. integrated to 128 frames, the main control module will transfer the integrated data in multiple frames from the static random access memory (SRAM) of the CPU to the baseband module, and continue to perform Doppler DC removal, inter-frame FFT, inter-channel integration (e.g. Non-coherent integration, correlation integration, etc.) processing based on the baseband module. Then, in the baseband module, constant false alarm rate detection (e.g. peak-based CFAR (detection with peak selection)) is performed based on the noise obtained by noise estimation (e.g. using a noise variance estimator), and finally angle detection and target classification and the like are performed to obtain a range, velocity and/or angle information and the like of the target. Herein, when performing angle detection, Azimut estimation and Elevation estimation may be performed first based on CFAR results, for example, may be implemented through technologies such as Digital Beam Forming (DBF) and direction of arrival estimation.

The implementation of digital signal processing shown in FIG. 2 is described previously.

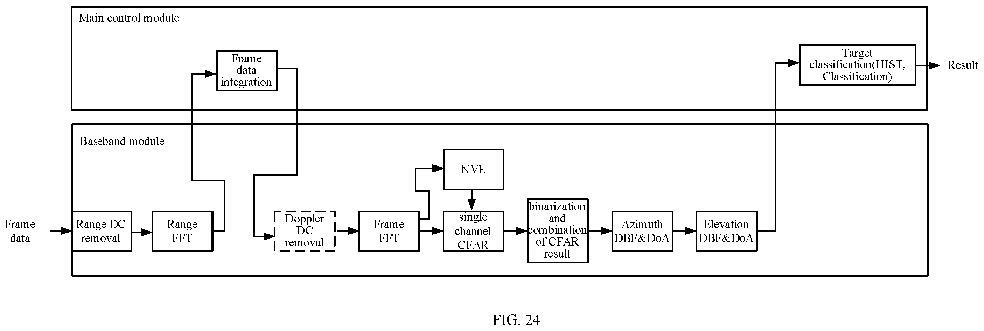

Therefore, it can be seen from the above that, in combination with the multi-frame joint processing scheme described above, that is, the multi-frame joint technology is adopted when acquiring the RD spectrum, or the non-correlation integration and CFAR processing in the multi-frame joint technical scheme described above are replaced with the target detection method provided by the embodiment shown in FIG. 21 to FIG. 23, that is, the scheme provided by the embodiment described above is combined with the flow shown in FIG. 2, and the flow of the target detection will be as shown in FIG. 24. At this time, after CFAR processing is performed on all channels one by one, binary integration in channel-domain processing is continued, and then an horizontal/azimuth and elevation estimations are performed. For example, for a system having M channels, after each channel is subjected to inter-frame FFT as shown in FIG. 2, after each channel undergoes CFAR processing respectively to obtain M bit masks, each element thereof may be of bool type, whether there is a target in each range bin and/or Doppler bin is determined (if 1, it is defined as existence, if 0, it is defined as non-existence). At the same time, the M bit masks may be integrated, where a range of each element may be defined as 0 ~ (M-1). Subsequently, the integrated mask may be detected a second time, that is, each element of the integrated mask may be compared with a preset threshold M0. If a value xi (such as a certain element of the integrated mask) of a bin to be detected satisfies xi> M0, an existence of a target in the bin to be detected may be outputted (that is, a final target data represents an existence of a target), if xᵢ ≤ M0, then it can be outputted that there is no target in the bin to be detected (that is, the final target data indicates that there is no target). Wherein xi, M, and M0 are positive integers. Herein, in the flow of the detection method shown in FIG. 24, because each channel is used to perform CFAR processing separately, the performance loss of channel imbalance can be effectively reduced, and it has better robustness in practical applications.

In order to facilitate those skilled in the art to better understand the flow shown in FIG. 24, the flow of different target detection methods will be described hereinafter.

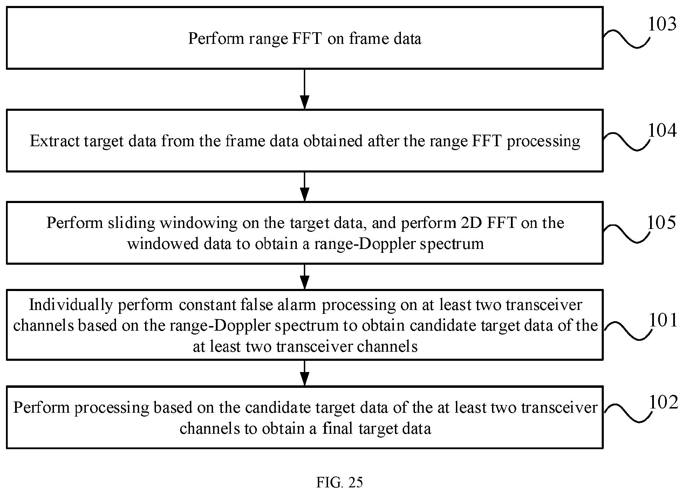

In some embodiments, the flow of the target detection method is shown in FIG. 25, and includes the following steps.

Step 103: performing range FFT on frame data.

Step 104: extracting target data from the frame data obtained after the range FFT processing.

Step 105: applying sliding windowing to the target data, and performing 2D FFT on the windowed data to obtain a range-Doppler spectrum.

Herein, correspondingly, a duration of sliding window is commensurate with an order of magnitude of a target's periodic motion cycle.

In order to facilitate those skilled in the art to better understand the target detection method described in the embodiment shown in FIG. 25, the steps thereof will be explained below.

In step 103, frame data refers to data corresponding to an echo signal formed after a frame of detection signal is reflected by a target. It can be understood that the target detection behavior performed by a radar usually has a certain period as shown in FIG. 4. Within a detection period, the radar first sends a frame of detection signal. Taking the detection of frequency modulated continuous wave (FMCW) radar as an example, as shown in FIG. 5, a frame of detection signal will be composed of multiple chirp signals. At the same time as the detection signal starts to be transmitted, the radar is ready to start receiving the echo signal of the detection signal reflected by the target, and the echo signal corresponding to a frame of detection signal will undergo the aforementioned analog-to-digital conversion, range DC removal and other processing to obtain the above frame data. Furthermore, each frame data is subjected to range FFT as described above, and step 103 is implemented, the detailed description thereof will not be repeated here.

In step 104, the target data is not limited in the embodiments of the present application, and the target data may be any data content belonging to corresponding frame data, for example, when storage resources are sufficient and computing power is sufficient, extracting the target data may be extracting all the data. Or, in a case where it is desired to achieve the highest real-time performance of target detection, a portion of data or the like may be extracted, and the target data may be extracted according to requirements, hardware conditions, and the like. For convenience of understanding, different implementations of extracting a portion of data as target data will be described below.

In some embodiments, extracting the target data from the frame data obtained after the range FFT processing is implemented by the following step: extracting data corresponding to a chirp period from each frame data obtained after the range FFT processing as the target data.

That is to say, the data corresponding to a chirp period is used as a representation of frame data, which makes the processing of target data simple and easy to implement, and the selected data is more real, which can carry out target detection more efficiently, and is conducive to improving the real-time performance of target detection.

In some embodiments, extracting the target data from the frame data obtained after the range FFT processing may be further implemented by the following step: determining parameters corresponding to each range bin in each frame data according to each frame data obtained after the range FFT processing, to generate the target data.

That is, a plurality of data of each range bin in the frame data are compressed into corresponding parameters, so that the overall characteristics of the frame data can be retained, such that the information which can be referred to when subsequently detecting the target according to the target data is more comprehensive, and the obtained result is more accurate.

Of course, the above embodiments are only examples provided to reduce the resources occupied by the target data, and in some embodiments, the target data may also be extracted in consideration of validity of the data. For example, radar signals can usually cover a large range, but not all regions covered by radar are regions which need attention. Processing the data corresponding to such regions will cause a waste of resources. Therefore, in some embodiments, extracting the target data from the frame data obtained after the range FFT processing may be further implemented by the following step: extracting data within a preset distance range from the frame data obtained after the range FFT processing as the target data.

That is, the target data is extracted from frame data according to the range of interest (represented by a preset distance range), so that accurate target detection information can be retained, and the storage resources, computing resources, etc. occupied by the target data can be reduced, thus balancing the accuracy and real-time performance of target detection.

It should be noted that, in addition to the above methods, the retention of data within the range of interest may further be implemented by the following: in some embodiments, windowing the target data according to the sliding window, and windowing the target data according to the sliding window is implemented by steps of performing digital signal processing on data within a preset Doppler range among the windowed data and performing digital signal processing on the windowed data, and the effect of retaining data of the region of interest can also be realized, but the dimensions of the two data are different, one is the range dimension and the other is the Doppler dimension, so the selection method of the range of interest is different, and of course, it is also possible to extract the target data in both the range dimension and the Doppler dimension to perform digital signal processing and the like, the detailed description thereof will not be repeated here.

Of course, the above descriptions are only examples of the implementation of step 104, and in some embodiments, it can also be implemented by other methods, such as the range bins may be modulo in pairs to get a larger range unit (similar to max-pooling) for target data extracting, the detailed description thereof will not be repeated here.

In step 105, the method of sliding windowing is not limited, for example, the sliding window may be a window of a fixed length; and for example, the sliding window may be a window of variable length, etc., the method of sliding windowing will not be listed here. And a sliding window of a fixed length may be described hereinafter as an example for convenience of understanding, but this does not mean that only a sliding window of a fixed length can be used, for example, a window of a variable length may be used, the window of the variable length may change according to a change in a periodic motion such as respiratory rate of the target.

In some embodiments, as shown in FIG. 10, for the 1st to 129th frame data generated after the radar is started, taking the window length of the sliding window as 128 frames as an example, the data obtained by the sliding window for the first time is the 1st to 128th frame data (the data of the shaded portion of the first row in FIG. 9). The data obtained for the second time is the 2nd to 129th frame data (the data of the shaded portion of the second row in FIG. 10), that is, the sliding step length of the sliding window is 1 frame.

Of course, the above is merely an example, and in other embodiments, other parameters such as the window length and sliding step length of the sliding window may be adopted, and will not be listed here.

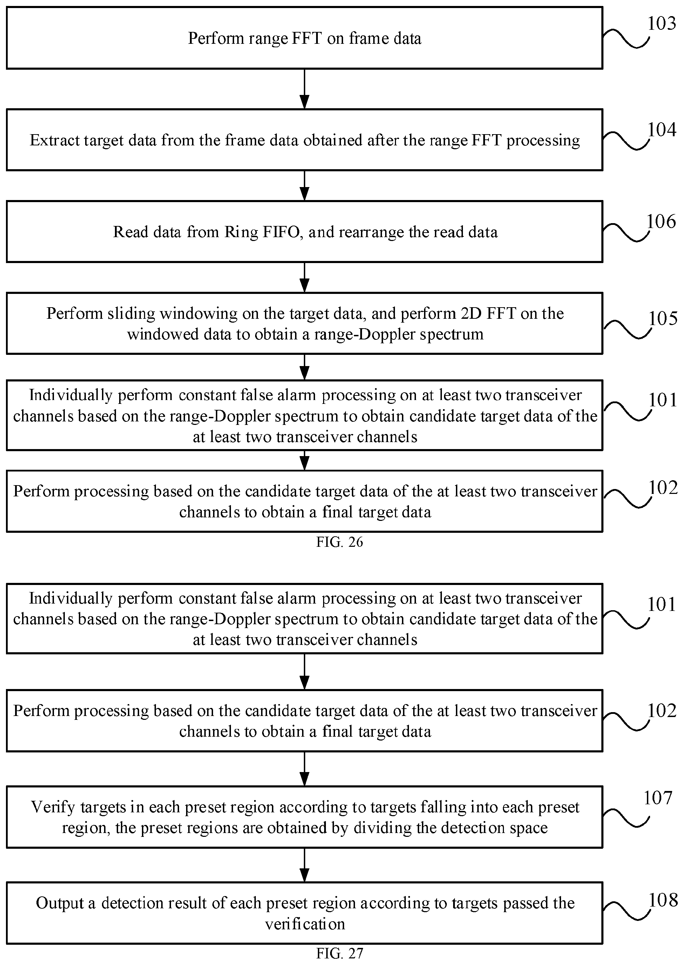

In step 105, when windowing the data, the data needs to have a certain order. If the order is disturbed, the temporal correlation of the data is destroyed, and the motion integrated for a certain period of time may no longer be reflected, which is not conducive to subsequent target detection. As shown in FIG. 24, the data is integrated in the main control module. When a certain amount of data is integrated, the baseband module reads the data and continues to process the data. In this process, the data writing order and the data reading order may be different due to the storage mode. Therefore, in some embodiments, it is necessary to rearrange the data to ensure its correct order. For example, in some embodiments, assuming that the data is cached using Ring FIFO, at this time, as shown in FIG. 26, the target detection method further includes the following step:
step 106: reading data from the Ring FIFO, and rearranging the read data.

Thus subsequent processing efficiency is improved through data rearrangement.

In order to facilitate those skilled in the art to better understand the above-described data storage and rearrangement, the following will be described with reference to FIG. 13.

As shown in FIG. 13, assuming that the data in the range of interest (for example, within the 5th range bin to the 20th range bin) obtained after the baseband module (shown by BB in the FIG. 13) performing range FFT on the frame data belongs to the target data, a two-dimensional matrix as shown in FIG. 13 will need to be integrated in the main control module (shown by MCU in the FIG. 13), rows of the matrix correspond to frame numbers and columns of the matrix correspond to range bins. Detailed storage procedures are described in the previous description and will not be repeated here.

In step 105, it can be further understood that in the detection process, the multi-frame joint detection may need to be observed in combination with different periodic motions, so that the target detection result can be obtained more accurately. Based on this, applying sliding windowing to the target data and performing 2D FFT on the windowed data may be implemented by the following: applying sliding windowing to the target data according to the periods of different physiological parameters of the living subjects, and performing digital signal processing on the windowed data corresponding to the sliding windows of different lengths, respectively.

Herein, the periods of the physiological parameters associated with each sliding window vary, and the duration of each sliding window is commensurate with the period of its corresponding physiological parameter in terms order of magnitude. At this time, it can be understood that when detecting based on a plurality of different periodic motions, the data continues to integrate after the integration of data with a shorter period is completed, and the processing of the data is completed until the integration of data with a longer period is completed. For example, assuming that one periodic motion corresponds to 32 frames and another periodic motion corresponds to 128 frames, both of which need to be observed, then in the process of target detection, a detection result is outputted starting from the completion of the target data acquisition corresponding to the 32nd frame data, and a new detection result is outputted after the target data corresponding to the 33rd frame data is acquired... until the target data acquisition corresponding to the 128th frame data is completed, two detection results (one is the detection result corresponding to data of 32 frames, and the other is the detection result corresponding to data of 128 frames) will be outputted, or a result obtained after comprehensive processing of the two results will be outputted.

In this way, by acquiring the periods of physiological parameters of living subjects, a length of the sliding window can be adjusted in time, so as to better detect living subject targets and better monitor the state of living subjects (such as children, pets, etc.), and so as to monitor and perform corresponding processing on the performance of the living subjects in time.

It should be noted that the embodiments of the present application do not limit the physiological parameters. For example, the physiological parameter may include respiration. Further examples of the physiological parameters may include a heartbeat and/or a pulse. Of course, the above are only examples, and other physiological parameters that may be embodied as motion characteristics may also be used under other requirements, each of the other physiological parameters will not be listed here.

In addition, it can also be understood that if the user wants to obtain more intuitive and accurate results, at this time, further processing may be performed after obtaining the target data, for example, target classification, further verification, and the like.

Based on this, in some embodiments, as shown in FIG. 27, after obtaining the target, the following steps are further executed.

Step 107: verifying targets in each predefined zone according to targets falling into each predefined zone, the predefined zones are obtained by dividing the detection space.

Step 108: outputting a detection result of each predefined zone according to targets passed the verification.

That is, considering a situation that for a target with a certain volume and occupying a certain space, usually multiple target points are detected, a currently detected target is continuously used in combination with region division to perform target verification to improve the detection result, and based on a division of the detection space in an application scenario, the detection result of each predefined zone is outputted, so that the target will show a distribution in the predefined zones, which is more intuitive and accurate, more conducive to the user's decision-making based on the output result, and the user experience is better.

In order to facilitate those skilled in the art to better understand the embodiment shown in FIG. 27, the steps thereof will be explained below.

In step 107, the detection space and the predefined zone are not limited, and may be different according to different application scenarios, application requirements, and the like. For example, in the application scenario of vehicle-mounted radar, the detection space may be inside the vehicle, and at this time, the predefined zone at least includes any of the following regions: a region corresponding to a seat and an region corresponding to a floor mat, so that objects such as drivers or adults can better perceive the situation inside the vehicle. For another example, in a factory work scenario, the detection space may be a factory building, and at this time, the predefined zone may include workstation of each employee, so as to avoid production risks and the like caused by employees not being able to monitor working conditions of machines due to employees not being at their workstations. The detailed description thereof will not be repeated here. For the convenience of understanding, the above space inside the vehicle will be used as an example for illustration hereinafter, but this does not mean that the corresponding scheme can only be implemented inside the vehicle.

Taking the interior of the vehicle as the detection space as an example, the interior space of the vehicle is abstracted into a coordinate system as shown in FIG. 15, wherein the abscissa of the coordinate system represents the azimuth with respect to the radar installed in the vehicle, and the abscissa of the coordinate system represents the elevation with respect to the radar installed in the vehicle. At this time, the interior of the vehicle is divided into six predefined zones including three rear seats and three aisles in front of the three seats, i.e., different filling regions as shown in FIG. 15. Herein, according to different requirements, as shown in FIG. 15, different predefined zones may overlap with each other or may not overlap. Some regions in the vehicle may belong to a plurality of predefined zones at the same time, or may not belong to any region.

It should be noted that FIG. 15 is only an example of an abstract method of detecting space, and in some embodiments, the detection region may be abstracted from all or a certain dimension of range, azimuth, and elevation, or all or a certain dimension along the x-y-z dimension of the rectangular coordinate system, and further divided into predefined zones, the detailed description thereof will not be repeated here.

In step 108, the verification method is not limited, and for example, the verification may be performed according to the number, ratio, and the like of the target points.

For example, in some embodiments, verifying the targets in each predefined zone based on the targets falling into each predefined zone may be implemented by the following step: verifying the targets in each predefined zone based on a ratio of the targets falling into each predefined zone to the detected targets.

For another example, in some embodiments, verifying the targets in each predefined zone according to the targets falling into each predefined zone may be implemented by the following step: verifying the target in each predefined zone according to the signal-to-noise ratio of the target falling into each predefined zone.

Of course, the above are merely examples, and in some cases, targets at other locations may be verified based on the falling situation of target points at locations where targets are known, such as the driver's seat, and the detailed description thereof will not be repeated here.

In step 108, the output method is not limited, and the detection result of each predefined zone may be directly output in a point cloud, or the divided predefined zones may be simultaneously presented with the output of the point cloud of each predefined zone, etc., the detailed description thereof will not be repeated here.

In order to facilitate those skilled in the art to better understand the above embodiments, ratio verification will be described below as an example.

Assume that a total of 11 target points are detected in a certain target detection process, and the division of the predefined zones is shown in FIG. 15, at the same time, the distribution of the target points in the predefined zones is shown in FIG. 18, i.e., the region seat A (6 target points), seat B (2 target points), seat C (0 target points), aisle A (0 target points), aisle B (1 target point), and aisle C (0 target points), wherein the target points are represented by solid circles in FIG. 18.

At this time, the algorithm obtained according to the above embodiment will be as follows:
each flag bit flag_region_i that identifies whether there are people in six regions is initialized to 0, i.e., no one exists (i = 0, 1,..., 5);
if the total number of valid targets total_valid_tgt_num is 0, it is determined that no one is present in the vehicle and the process is terminated, otherwise, the following operations are performed;
traversing all regions, for region i, if a statistical value tgt_num_region_i of the number of valid targets belonging to the region exceeds 25% of the total number of valid targets total_valid_tgt_num, setting the modified region i flag bit flag_region_i to 1;
if a certain region exists, and if its flag_region_i is 1, flag_region_empty is set to 0, i.e., there is a person in the vehicle;
traversing the seat region, if a flag_region_i of any seat region is 1, it is determined that there is no one in the corresponding aisle region (for example, if it is determined that there is a person in seat A, then it is determined that there is no one in aisle A);
outputting a result.

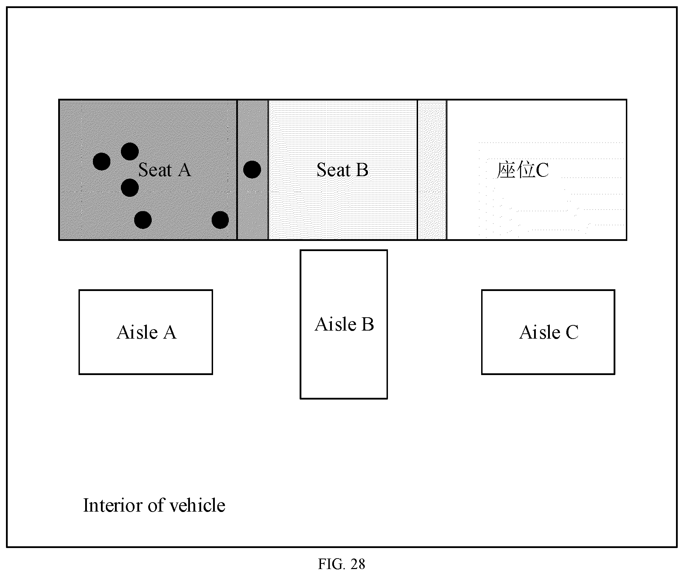

At this time, the output result will be as shown in FIG. 28, only in the region seat A, six target points are displayed, and there is no one in the remaining predefined zones.

It should be noted that the threshold value of 25% in the above example is merely an example, and other threshold values or other judging conditions may be adopted in other embodiments, and the detailed description thereof will not be repeated here.

In this way, on the basis of individually performing constant false alarm rate processing through a single transceiver channel to avoid the interference of abnormal channels or abnormal data, the frame-level FFT is further adopted instead of a traditional chirp-level Doppler FFT to effectively integrate motion power of the target to achieve a purpose of accurate detection and measurement, and a result update frequency can be further increased by performing sliding window FFT processing on multiple frames; through post-processing ways such as regional statistics on the target points detected by the radar, determining whether there is a person in each region is realized. For example, the frame-level FFT is used instead of the traditional chirp-level Doppler FFT to determine a velocity of the target to achieve accurate detection of the target of interest, and a sliding window FFT processing may be performed on multiple frames to further improve the update frequency of the result, thereby improving the real-time performance of the system. At the same time, it is also possible to reduce the amount of data processed by processing only ranges and/or Doppler regions of interest. In addition, when estimating a background noise, a minimum value may be taken based on an estimation of a current range bin combined with an estimation of a last range bin of interest to obtain a background noise, such operation is more suitable for application scenarios in relatively closed environments such as a cabin of a vehicle. When judging the region logic, by counting target points detected in each frame according to a predefined zone design, and determining the counting result according to a preset rule, it is possible to accurately determine whether there is a target in each predefined zone.

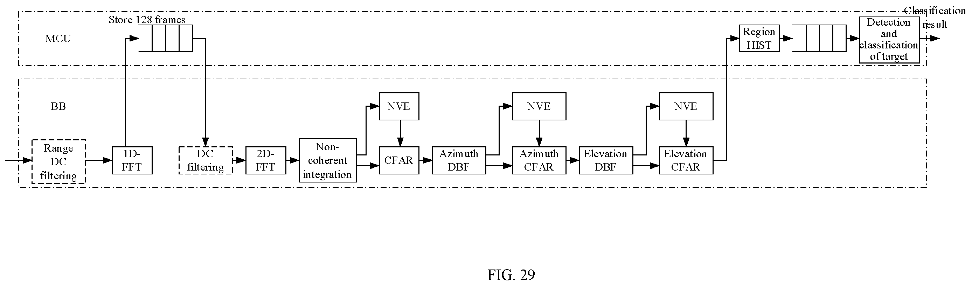

FIG. 29 is a schematic flow diagram of implementing target detection based on frame-level FFT combined with DAE CFAR in an embodiment of the present application. As shown in FIG. 29, on the basis of the flow structure shown in FIG. 1, and on the basis of the inter-frame FFT, an azimuth and an elevation are estimated using DAE CFAR (Constant False Alarm Rate) for the CFAR data. For example, after a Doppler CFAR detection is performed on inter-frame FFT data, an Azimuth DBF is performed, and an Azimuth CFAR is continued in combination with an NVE (noise variance estimator) noise floor estimation; Similarly, an Elevation DBF is performed on the Azimuth CFAR data, and an Elevation CFAR is continued in combination with the NVE noise floor estimation to obtain the azimuth and elevation information of the target.

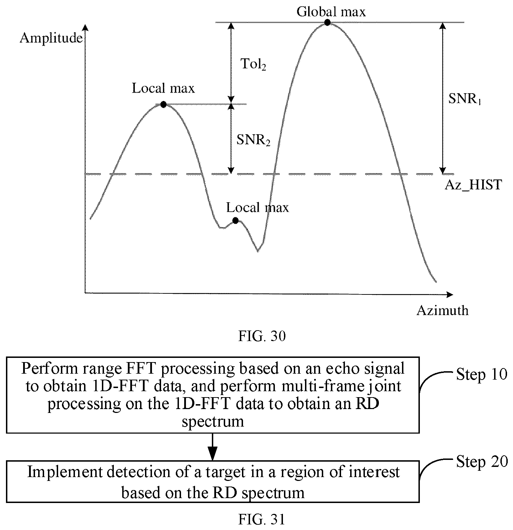

FIG. 30 is a schematic diagram of an azimuth or elevation CFAR in the target detection flow based on an illustration in FIG. 29. As shown in FIG. 30, power data of azimuth/elevation may be calculated by an NVE engine in a HIST manner, and a noise floor estimation of azimuth/elevation DBF may be performed by selecting a certain rank value (e.g., average, median, etc.). Herein, when performing DBF, it is possible to determine whether to output as a peak by acquiring all peaks in a DBF spectrum, and by all peak points and a SNR of the estimated noise floor.

For example, when performing DBF, it is possible to determine whether to output as a peak by acquiring magnitude of all peak points in the DBF spectrum, i.e., including a peak of a global maximum value (Global max) and a peak of the local maximum value (Local max); for any peak, if its SNR exceeds a predetermined threshold, and a difference between its amplitude (local max) and a global maximum amplitude (Global max) is within a predetermined range, and a number of outputted peaks is less than the predetermined number, the peak is outputted, that is, outputted as a real target point. Compared with the scheme of directly using a peak value in the Doppler spectrum to find a target, because the Doppler spectrum may contain clutter, so that the peak value may not reflect a real target, so it is more accurate to find real target points through the above scheme; secondly, there may be a plurality of targets in the Doppler spectrum, and if only the peak value is used to find the target, it will lead to the lack of target classification. However, using this implementation method for target detection takes into account a relationship between a candidate target and a global maximum amplitude, so accurate multi-target detection can be realized.

An embodiment of the present disclosure provides a target detection method, as shown in FIG. 31, including:
Step 10: performing range FFT processing based on an echo signal to obtain 1D-FFT data, and performing multi-frame joint processing on the 1D-FFT data to obtain an RD spectrum; the RD spectrum is, for example, a range-Doppler two-dimensional spectrum, and the spectrum contains range Doppler data, i.e., contains two-dimensional range-velocity data.
Step 20: implementing detection of a target in a region of interest based on the RD spectrum.

The above detection of the target in the region of interest includes at least one of the following operations: determination, positioning, classification, and the like.

In a target detection method and a related apparatus provided by an embodiment of the present disclosure, through the multi-frame joint processing technology, i.e., through the inter-frame integration mode, a target detection scheme in a closed space region such as in a cabin, an indoor, a factory building, etc. can be realized, so that the detection rate can be effectively improved, the number of false alarm targets can be reduced, and the accuracy of angle estimation can be greatly improved, and the like, thereby enabling special targets or weak targets such as infants and young children in the cabin to be accurately detected to implement applications such as CPD (Child Presence Detection) and SBR (Safety Belt Reminder).

An embodiment of the present disclosure further provides a target detection method, which may include: performing an intra-chirp range FFT and an inter-frame velocity FFT on an echo signal to obtain range velocity data; performing a primary constant false alarm rate processing on the range velocity data to obtain candidate target detection points; performing a secondary constant false alarm rate processing on the candidate target detection points, and performing the target detection based on a secondary constant false alarm rate processing result.

In the embodiment of the present application, through twice constant false alarm rate processing, false alarms can be effectively eliminated, and detection and measurement performance can be effectively improved. Furthermore, special targets or weak targets such as infants and young children in the cabin can be accurately detected, and applications such as CPD and SBR can be realized.

In an exemplary embodiment, the step of obtaining the range velocity data includes: obtaining 1D-FFT data after performing range FFT processing in a chirp on an echo signal; integrating frame data on the 1D-FFT data until a preset data amount is integrated, and then performing inter-frame velocity FFT processing to obtain an RD spectrum, i.e., the range velocity data. For example, a sliding window method may be used to read 1D-FFT data of a preset number of frames at a time to perform inter-frame FFT processing.

During the implementation process, accurate detection of targets in the cabin can be achieved based on multi-frame joint processing technology. The multi-frame joint processing scheme may be: after obtaining a range-Doppler spectrum by performing sliding window FFT on multi-frame data, or after using FIR (Finite Impulse Response) or other complex time-frequency transform processing, performing processing operations such as CFAR and DOA on the region of interest, and the processing operations may be combined with a set region determination logic and region parameters and the like to realize the detection and positioning operation of living targets such as adults, children, pets, or other non-living targets. The CFAR may be an NR-CFAR of the Doppler dimension, or an RD-CFAR, or a DAE (Doppler-Azimuth-Elevation)-CFAR, or the like. After CFAR and DoA, post-processing such as clustering, false alarm suppression, and multiple processing of point cloud correlation may be used. A region parameter may be determined by using at least one scheme or a combination of at least two schemes of performing a clustering operation on the point cloud detected after a certain sliding window multi-frame processing, performing outlier detection and culling operation on the point cloud detected after the certain sliding window multi-frame processing, and the like.

In an exemplary embodiment, the step of performing the primary constant false alarm rate processing includes: performing non-coherent integration on the RD spectrum of the multiple channels, performing the primary constant false alarm rate processing on the basis of noise estimation based on the non-coherent integration result, and obtaining candidate target detection points.

Optionally, the primary constant false alarm rate processing includes: performing the non-coherent integration on the RD spectrum of multiple channels, estimating a noise floor of each range bin after the non-coherent integration, obtaining a noise floor estimation value of each range bin, and performing a non-coherent constant false alarm rate processing by using the noise floor estimation value, wherein when estimating the noise floor of each range bin after the non-coherent integration, the noise floor of each range bin is adjusted by using a global noise floor.

An optional adjustment method includes: adjusting the noise floor of each range bin in the following manner:*nᵢ'* = *min*(*nᵢ, α * n_{g}*), wherein is an original noise floor estimation value of an *i* -th range bin, and *n_{g}* is an average value of noise floor estimation value of a plurality of range bins, *nᵢ'* is the estimated noise floor value of the *i* -th range bin after the adjustment, *α*>1. By adjusting the noise floor, the target can be avoided from being undetected due to excessively high noise floor.

In an exemplary embodiment, the secondary constant false alarm rate processing includes performing DAE (Doppler-Azimuth-Elevation) CFAR processing on the azimuth and the elevation, respectively.

In an exemplary embodiment, the step of performing the secondary constant false alarm rate processing includes performing digital beam forming processing on the candidate target detection points, and then performing the secondary constant false alarm rate processing based on noise estimation to obtain a screening result in which the false target points are filtered out.

One implementable method is: performing azimuth DBF on the candidate target detection points, and performing an azimuth constant false alarm rate processing in combination with a noise floor estimation result, to obtain an azimuth dimension target screening result; and/or performing elevation DBF on the candidate target detection points, and performing an elevation constant false alarm rate processing in combination with the noise floor estimation result, to obtain an elevation dimension target screening result.

Another implementable method is: performing azimuth elevation two-dimensional DBF on the candidate target detection points, and performing an azimuth elevation constant false alarm rate processing in combination with the noise floor estimation result, to obtain an azimuth elevation two-dimensional target screening result.

The noise floor estimation result may be statistics on the DBF spectrum of the current dimension, and a quantile value (such as a median value or an average value) is selected as the noise floor estimation result of the current dimension.

For example, the above azimuth constant false alarm rate processing, the elevation constant false alarm rate processing, or the azimuth elevation constant false alarm rate processing may perform the constant false alarm rate processing in the following manner:
During the constant false alarm rate processing, one or more of the following operations are performed on the candidate target detection points to obtain the target detection result: global maximum value screening, first threshold value screening, and second threshold value screening, wherein the global maximum value screening is used for screening whether a global maximum value is a target detection point, the first threshold value screening determines whether a current candidate screening result is a target detection point according to a relationship between a candidate screening result and a noise floor estimation value, so as to filter out a false target, and the second threshold value screening determines whether the current candidate screening result is a target detection point according to a relationship between the candidate screening result and the global maximum value, so as to realize multi-target detection.

Herein, the global maximum value screening includes: determining whether a difference between a digital beam forming spectral amplitude value corresponding to a current global maximum value and the noise floor estimation value is within a first predefined range, and if it is within the first predefined range, a candidate screening result corresponding to the global maximum value is a target detection point; the first threshold value screening includes: determining whether a difference between a current candidate screening result and a noise floor estimation value is within a second predefined range, and if it is within the second predefined range, the current candidate screening result is a target detection point; the second threshold value screening includes: determining whether a difference between a power value of the current candidate screening result and a power of the global maximum value is within a third predefined range, and if it is within the third predefined range, the current candidate screening result is a target detection point.

In an exemplary embodiment, when detecting a target in the region of interest is implemented based on the RD spectrum, the region of interest is divided into a plurality of target sub-regions in advance, the number and locations of target points in each sub-region are determined according to the locations of the target detection points obtained after the target detection, and whether there is a target to be detected in a current sub-region is determined according to whether a ratio of the number of target points in each sub-region to a total number of targets is greater than a preset ratio value.

Herein, when applied to a closed space region or a relatively closed space region, the space region can be divided in advance, and then the definition and detection of different regions of interest (divided regions) can be realized. For example, when detecting targets in a cabin of a vehicle, the region monitored by a radar may be divided into seat sections and aisle sections, etc., and then corresponding parameter types and thresholds may be preset for different types of regions, and corresponding processing steps may be combined, and then accurate detection of targets of interest in specific regions can be achieved.

In some optional embodiments, for a family vehicle, the space region in the vehicle may generally be simply divided into a head space, a rear space and a trunk space, and if the rear space is a key monitoring region, the rear space may be continuously divided into a seat section and an aisle section, etc., and the seat section may also be divided into a corresponding number of seat section units based on the seats. Similarly, the aisle section may also be divided into a corresponding number of aisle section units corresponding to the above seat section units, for example, for a five-seat family vehicle, for three seats in the rear space, three seat section units and three corresponding aisle section units may be divided. Corresponding parameter types and thresholds and the like may be preset for different types of regions (or sections or section bins), and adaptive signal data processing methods and steps may be adopted, to achieve accurate detection of targets of interest (specific targets) in regions such as specific sections or section bins. Here, adjacent section bins may have partial overlapping regions, or may be adjacent to each other or spaced apart by a gap of a predetermined width.

An embodiment of the present disclosure further provides a target detection method, which is applied to target detection in a specific target region, and the method includes: after performing a primary constant false alarm rate processing on range-Doppler data, continuing to perform a secondary constant false alarm rate processing in an angle dimension to obtain target data.

In the above embodiment, the frame-level FFT is used instead of a traditional chirp-level Doppler FFT to determine velocity of the target to achieve accurate detection of the target of interest, and a sliding window FFT processing may be performed on multiple frames to further improve the update frequency of the result, thereby improving the real-time performance of the system. Furthermore, it is also possible to reduce the amount of data processed by processing only ranges and/or Doppler regions of interest. In addition, when estimating a background noise, a minimum value may be taken based on an estimation of a current range bin combined with an estimation of a last range bin of interest to obtain a background noise, such operation is more suitable for application scenarios in relatively closed environments such as a cabin of a vehicle. When judging the region logic, by counting target points detected in each frame according to a predefined zone design, and determining the counting result according to a preset rule, it is possible to accurately determine whether there is a target in each predefined zone.

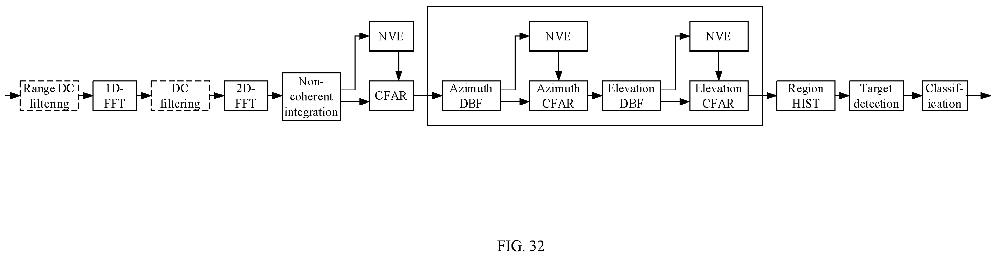

Hereinafter, the target detection method of the present disclosure will be described in detail through an application example, and the processing process may be shown in FIG. 32.

Step 1: when the target detection system detects a target in its detectable region, a transmitting antenna in the target detection system may transmit a Frequency Modulated Continuous Wave (FMCW) signal containing several chirps, and the FMCW signal is refracted and/or reflected by the target to form an echo signal, and the echo signal may be received by a receiving antenna in the target detection system.

Herein, the target detection system may be an antenna array system or a radar system having at least one transceiver channel, such as a Multiple Input Multiple Output (MIMO) radar system, or a Single Input Multiple Output (SIMO) system and the like. The target detection system usually has N transmitting antennas (TX) and M receiving antennas (RX), N and M are both positive integers greater than or equal to 1, and a virtual antenna array of N × M transceiver channels may be formed by reasonably arranging the location of each antenna.

Step 2: performing range FFT (1D-FFT) processing on each chirp data of each frame of the received echo signal to obtain range-dimensional data, that is, 1D-FFT data;
before 1D-FFT processing, optionally, the chirp data may be subjected to analogue-to-digital conversion (ADC) first, the obtained ADC data is then subjected to a first DC filtering process, the first DC filtering process includes: calculating an average along a fast time dimension of data collected on each RX channel of each chirp, and subtracting DC components thereof from all sampling points of each RX channel, wherein, the fast time dimension refers to a data sequence formed by sampling the radar echo signal along a range (or angle) direction, a DC offset in the signal can be eliminated by DC filtering, and a center line of the signal is made to be zero.

Optionally, before 1D-FFT processing, windowing the DC-filtered data may be performed first, and then 1D-FFT processing may be performed to obtain range dimension information. Spectrum leakage can be reduced and the performance of spectrum estimation can be improved by the windowing process. The windowing operation is to multiply an original signal by a window function, the window function may be a weight function with a specific shape, and the window function has a window length such as 64, 96 or 128. Common window functions include rectangular windows, Hamming windows, Hanning windows, Blackman windows, etc.

1D-FFT data may be transfered from the BB to the SRAM cache in the CPU or MPU by DMA.

Step 3: performing velocity FFT (2D-FFT) processing on 1D-FFT data of a preset number of frames to obtain range -velocity data, that is, 2D-FFT data (or RD spectrum).

The velocity dimension may also be referred to as Doppler dimension, and the above range-velocity information may also be referred to as range-Doppler information.

This step is different from a slow time processing between chirps of radar signal processing in the related art. By integrating multiple frames of data and performing inter-frame 2D-FFT processing on 1D-FFT data of a preset number of frames in a sliding window manner each time, the update frequency of the result can be improved, and the real-time performance of the system can be improved. The 2D-FFT process of this step may also be referred to as an inter-frame FFT process. Taking the preset number of frames as 128 and the sliding window step as 1 as an example, 2D-FFT processing is performed on frames 0-127 for the first time, and 2D-FFT processing is performed on frames 1-128 for the second time. Here, taking the sliding window step as 1 as an example, the sliding window step may be configured to be a positive integer greater than or equal to 1, and the preset number of frames may also be configured.

Before performing the 2D-FFT processing, optionally, a second DC filtering process may be performed on the 1D-FFT data, and the second DC filtering process includes: calculating a complex average along a frame dimension for the 1D-FFT data of a preset number of frames, obtaining an average value of different channels and different ranges as a DC component thereof, and subtracting the DC component thereof for each range bin of each channel. Herein, the range bin refers to a small range section divided by the radar system along a radial direction (that is, a direction of transmitting signals and receiving echoes). The range bin is used to represent the discrete range intervals used by the radar system in detecting and tracking targets.

Optionally, before the 2D-FFT processing, the data after the second DC filtering may be windowed first, and then the 2D-FFT processing may be performed along the frame dimension to obtain the range-velocity data. Spectrum leakage can be reduced and the performance of spectrum estimation can be improved by the windowing process. The windowing operation is to multiply an original signal by a window function, the window function may be a weight function with a specific shape, and the window function has a window length such as 64, 96 or 128, etc.

For each chirp data of each frame, when the data cached in SRAM obtains a certain number of frames, such as 128 frames, the 128 frames of data may be transfered from SRAM to BB through DMA, and the following steps can be continued.

Step 4: performing non-coherent integration (NCI) on multi-channel 2D-FFT data to obtain integrated data;
a non-coherent integration may be performed using any one of the following equations: $P \left(r, v\right) = {\sum }_{t = 0}^{{N}_{t}} {\sum }_{a = 0}^{{N}_{r}} {\left|s\left(r, t, a, v\right)\right|}^{2} / {N}_{t} / {N}_{r}$ $A \left(r, v\right) = {\sum }_{t = 0}^{{N}_{t}} {\sum }_{a = 0}^{{N}_{r}} \left|s\left(r, t, a, v\right)\right| / {N}_{t} / {N}_{r}$
wherein P(r, v) is power of a r-th range bin and a v-th Doppler bin, A(r, v) is an amplitude on the r-th range bin and the *v*-th Doppler bin, *s*(*r, t, a, v*) is a complex value of an echo signal on a t-th transmit channel and a *a*-th receiving channel of the v-th Doppler bin of the r-th range bin after being processed by the above 2D-FFT.

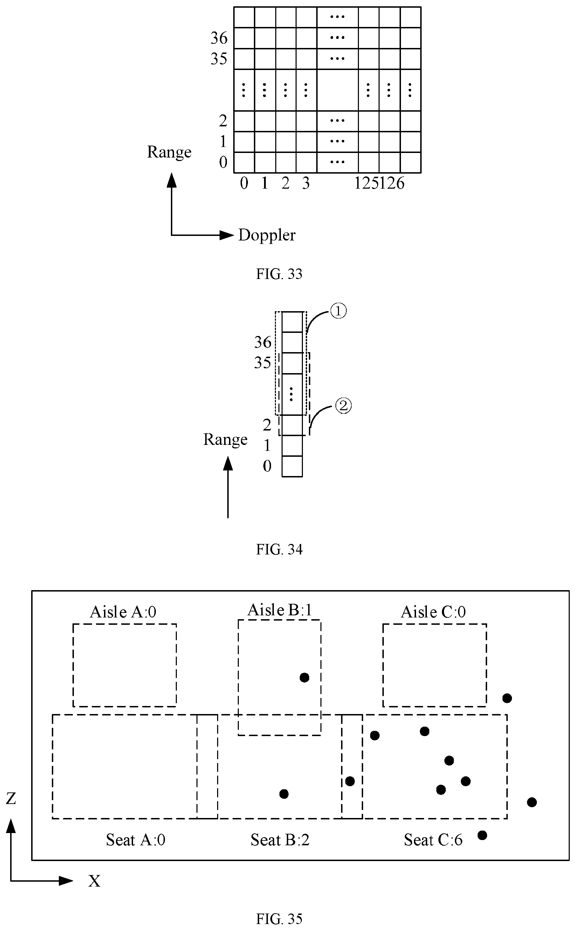

After non-coherent integration, the results shown in FIG. 33 are obtained. In the FIG.33, the abscissa illustrates Doppler, i.e., velocity, and the ordinate illustrates range. The 0th range bin includes all Doppler values with ordinate 0 in the FIG. 33.

Step 5: estimating a noise floor of each range bin in the integrated data, i.e., the background noise;
in this example, the following noise floor estimation method is used: *nᵢ'* = *min*(*nᵢ, α* * *n_{g}*), wherein *nᵢ* is an original noise floor estimation value of an *i* -th range bin, and *n_{g}* is an average value of noise floor estimation values of the plurality of range bins or a noise floor estimation value of a last range bin of interest, *nᵢ'* is an estimated noise floor value of an *i* -th ange bin after adjustment, *α*>1. As shown in FIG. 34, each cell in the figure represents an original noise floor estimation value of one range bin, for example, a median value or average value of each range bin may be calculated as the original noise floor estimation value. For example, a noise floor estimation value of the plurality of range bins may be calculated as an median value of a plurality of original noise floor estimation values shown in the dashed box ① in the figure. As a global noise floor estimation value, the noise floor estimation value of the plurality of range bins may help for noise floor adjustment (or referred to as noise floor saturation processing). When a target (for example, an adult) moves greatly, entire Doppler spectrum value will be high as a whole, resulting in the original noise floor estimation values being high, and the target will not be detected in the subsequent target screening. Adjusting the noise floor through the global noise floor estimation value can make the subsequent target detection more accurate. The quantities and locations of the range bins used for calculating the global noise floor estimation value may be configured, and for example, an noise floor estimation value of a plurality of range bins shown in the dashed box ② in the figure may be used as the global noise floor estimation value.

Step 6: performing NR-CFAR (Non-Coherent Constant False Alarm Rate, short for non-coherent CFAR) to obtain candidate target detection points.

Step 7: performing a first target screening on the candidate target detection points;
for each candidate target detection point *Tᵢ*, performing operations are as follows: extracting 2D-FFT data corresponding to the detection point, selecting azimuth antenna and carrying out azimuth DBF (digital beam forming) according to azimuth guide vector, obtaining DBF spectrum which shows a situation that signal power or strength varies with frequency in different directions, estimating a noise floor of the DBF spectrum and performing Azimuth CFAR (may be referred to as Az-CFAR), judging a result of Azimuth CFAR by preset conditions, and selecting candidate target points meeting the preset conditions as a first target screening result to form a first candidate target point set, wherein the first candidate target point set includes all the candidate target points T_{i,j} meeting the preset conditions and corresponding azimuths θ_{i,j} thereof.

The azimuth DBF spectrum is subject to statistics to obtain an azimuth CFAR results as shown in FIG. 3 (the elevation CFAR is similar). In the figure, the x-axis represents the azimuth, and the y-axis represents the DBF spectral amplitude. If only DBF amplitude peaks are used as target points, the Global max and Local max in the figure are both used as the target points, which may lead to a false alarm. Therefore, in order to suppress false targets, the following operations are performed:
performing statistics on the azimuth DBF spectrum, and selecting a certain quantile value thereof (such as a median value or an average value) as a noise floor estimation of azimuth DBF, that is, Az_HIST as shown in the figure, which is denoted as nₐ; finding a global maximum (global max) in the DBF spectrum, whose value is denoted as Pₘₐₓ, judging that if Pₘₐₓ < β₁ · nₐ , *β*₁ > 1, the candidate target point corresponding to the global maximum value in the DBF spectrum is determined to be a false target point, and finishing the processing of the target point, in the formula, β₁ is one of thresholds of azimuth CFAR, and whether the global maximum value is the target detection point can be determined through the above formula; if Pₘₐₓ ≥ β₁ · nₐ , the following processing is performed:
traversing all points on the DBF spectrum, if any point satisfies the following conditions, it is determined to be a candidate target point meeting the preset conditions: ${P}_{i} \geq {β}_{2} ⋅ {n}_{a}$ ${P}_{i} \geq γ ⋅ {P}_{max}$ ${P}_{i} \geq {ρ}_{1} ⋅ {P}_{i - 1}$ ${P}_{i} \geq {ρ}_{2} ⋅ {P}_{i + 1}$
in the formulas, i is a sequence number of the candidate target point in the azimuth dimension, 0 < i < Nₐ - 1, Nₐ is a number of points in the azimuth DBF spectrum, *Pᵢ* is power of the candidate target point, *β*₂, *γ*, *ρ*₁, *ρ*₂ are settable parameters, 0 < γ < 1, *β*₂ > 1. The above Formula 1 represents that a ratio of the power of the candidate target point to a noise floor needs to be above a preset threshold value *β*₂, the Formula 1 is equivalent to setting a first threshold, the first threshold is the estimated noise floor value plus a first threshold value, and a false target point can be filtered out by setting the first threshold. The Equation 2 represents that a ratio of the power of the candidate target point to a global maximum value of the power needs to be above a preset threshold valueγ, the Equation 2 is equivalent to setting a second threshold, the second threshold is the global maximum value of power minus a second threshold value, and by setting the second threshold, multi-target detection can be realized, which is beneficial to identifying targets such as children or infants. When *ρ*₁ *= ρ*₂ = 1, Formulas 3 and 4 above show that this point is of a local maximum value.

Step 8: performing a second target screening;
for each candidate target point T_{i,j}, generating an elevation direction steering vector sv(i, j) thereof according to azimuth θ_{i,j} and array arrangement thereof respectively, for each candidate target point T_{i,j}, performing elevation DBF to obtain its elevation DBF spectrum, estimating a noise floor of the elevation DBF spectrum and perform a second CFAR (referred to as El-CFAR), determining a result of elevation CFAR under a preset condition, and taking candidate target points meeting the preset condition as a second target screening result to form a second candidate target point set, which includes all candidate target points T_{i,j,k} meeting the condition and includes corresponding elevations φ_{i,j,k∘}

The second target screening process is similar to the first target screening process, firstly, the elevation DBF is subjected to statistics to obtain the elevation CFAR result, and after the result is determined, the formula Pₘₐₓ < β₁ · nₐ is used to determine whether the global maximum value is the target detection point, if Pₘₐₓ ≥ β₁ · nₐ , all points on the DBF spectrum are traversed, and if any point satisfies the above Formulas 1-4, it is determined that the point is a candidate target point meeting the preset condition.

In the embodiment of the present disclosure, after NR-CFAR is performed, the second CFAR is performed on the azimuth and elevation DBF, thereby suppressing false targets and realizing multi-target resolution at a same range and velocity to a certain extent.

When the DBF spectrum of the elevation dimension is generated in the El-CFAR described above, an elevation dimension steering vector is regenerated according to the azimuth information obtained in the Az-CFAR. In an exemplary embodiment, the elevation dimension steering vector with a preset azimuth (such as 0° azimuth) may also be directly used.

The order of the first target screening in the above step 7 and the second target screening in the above step 8 may be exchanged. And in some embodiments only step 7 or only step 8 may be performed.

Step 9: extracting target information;
for all candidate target points T_{i,j,k} that meet the conditions, extracting information including but not limited to range bin indexes and Doppler bin indexes of the target points. In addition, SNR (signal-to-noise ratio) of RD-CFAR (Range-Doppler CFAR, range-velocity CFAR), SNR of Az-CFAR, SNR of El-CFAR(Elevation CFAR), and the like may be included. The above SNR may be used for subsequent judging of a target person, for example, a corresponding target detection point weight may be assigned according to the SNR value, and the weight is related to the SNR value.

Step 10: all target points detected in each frame are counted according to the predefined zone design, and the counting result is determined according to the preset rule to obtain whether there is a person in the present processing, and a location of the person is determined, that is, the target information is obtained.

An embodiment of the predefined zone is shown in the following FIG. 35, in which the abscissa is the X direction (i.e. vehicle width direction), and the Z direction represents a vehicle rear-front direction. All or some dimensions of the interior space of a vehicle along X-Y-Z, or all or some dimensions along the distance-azimuth-elevation dimension of the polar coordinate system are divided into regions to be judged. As an example shown in FIG. 35, the interior space of a vehicle is divided into a total of six regions, including three rear seats and three aisles in front of the three seats. These regions may or may not overlap with each other; some regions in the vehicle may belong to a plurality of predefined zones at the same time, or may not belong to any region.

Assuming that a total of 11 target points are detected in a certain process, the number of these target points in these predefined zones is counted. In this example, the counts of these target points in the regions seat A, seat B, seat C, aisle A, aisle B and aisle C are respectively: 0, 2, 6, 0, 1 and 0.

Taking the region division and counting results as an example, determining whether there is a person in the present process and determining the location of the person includes the following steps:
step 10.1: initializing a flag bit flag_region_i of each of the six regions which indicates whether there is any person to 0, that is, no one exist in each of the six regions(i = 0, 1, . . . ,5) ;
step 10.2: determining whether a total number of valid targetstotal_valid_tgt_num is 0, if yes, it is determined that there is no one in the vehicle and the process is terminated, if not, step 10.3 is executed;
step 10.3: traversing all regions, for region i, if it is determined that a ratio of a statistical value tgt_num_region_i of the number of valid targets belonging to the region to a total number of valid targets total_valid_tgt_num exceeds a preset ratio value (for example, 25%, the ratio value may be set empirically), the flag bitflag_region_i of the region i is set to 1;
step 10.4: if there is a region of which the flag_region_iis 1, then setting the flag_region_empty to 0, that is, there is someone in the vehicle;
step 10.5: traversing the seat regions, if a flag_region_i of any seat region is 1, it is determined that there is no one in the corresponding aisle region (for example, if it is determined that there is a person in the seat A, then it is determined that there is no one in the aisle A);
step 10.6: outputting the result.

By dividing regions and setting a preset ratio value, a target person can be determined more accurately, and mis-determination can be prevented.

In an exemplary embodiment, on the basis of the first CFAR result (i.e., the result of step 6), the azimuth and elevation DBF may be directly performed and a second CFAR may be directly performed on the azimuth and elevation DBF spectrum. The main steps include:
extracting 2D-FFT data, select an antenna and perform two-dimensional DBF according to the azimuth and elevation two-dimensional steering vector to obtain the DBF spectrum, estimating a noise floor of the DBF spectrum, wherein a method for estimating the noise floor refers to the above step 5, performing a second CFAR (called AE-CFAR), and obtaining all candidate target points *T_{i,j}* satisfying the conditions and corresponding azimuth θ*_{i,j}* and elevation *φ_{i,j}* thereof; one possible way is to traverse all points on the DBF spectrum, and determine that any point is a candidate target point if the point meets the following conditions: ${P}_{i , j} \geq {β}_{2} ⋅ {n}_{a}$ ${P}_{i , j} \geq γ ⋅ {P}_{max}$ ${P}_{i , j} \geq {ρ}_{1} ⋅ {P}_{i - 1 , j}$ ${P}_{i , j} \geq {ρ}_{2} ⋅ {P}_{i + 1 , j}$ ${P}_{i , j} \geq {ρ}_{3} ⋅ {P}_{i , j - 1}$ ${P}_{i , j} \geq {ρ}_{4} ⋅ {P}_{i , j + 1}$
in the formulas, i is a serial number of the point in the azimuth dimension, 0 < i < Nₐ - 1 Nₐ is a number of points in the azimuth DBF spectrum, j is a serial number of the point in the elevation dimension, 0 < j < Nₑ - 1 , Nₑ is a number of points in the elevation DBF spectrum, P_{i,j} is power of this point, *β*₂, γ, *ρ*₁, *ρ*₂, *ρ₃*, *ρ*₄ are settable parameters, 0 < γ < 1, *β*₂ > 1. The above Formula 5 represents that a ratio of the power of the candidate target point to a noise floor needs to be above a preset threshold value *β*₂, the Formula 6 is equivalent to setting a first threshold, the first threshold is the estimated noise floor value plus a first threshold value, and a false target point can be filtered out by setting the first threshold. The formula 6 represents that a ratio of the power of the candidate target point to a global maximum value of the power needs to be above a preset threshold valueγ, the Formula 6 is equivalent to setting a second threshold, the second threshold is the global maximum value of power minus a second threshold value, and by setting the second threshold, multi-target detection can be realized, which is beneficial to identifying targets such as children or infants. When *ρ*₁ = *ρ*₂ = ρ₃ = ρ₄ = 1, the above Formulas 7-10 show that this point is of a local maximum value.

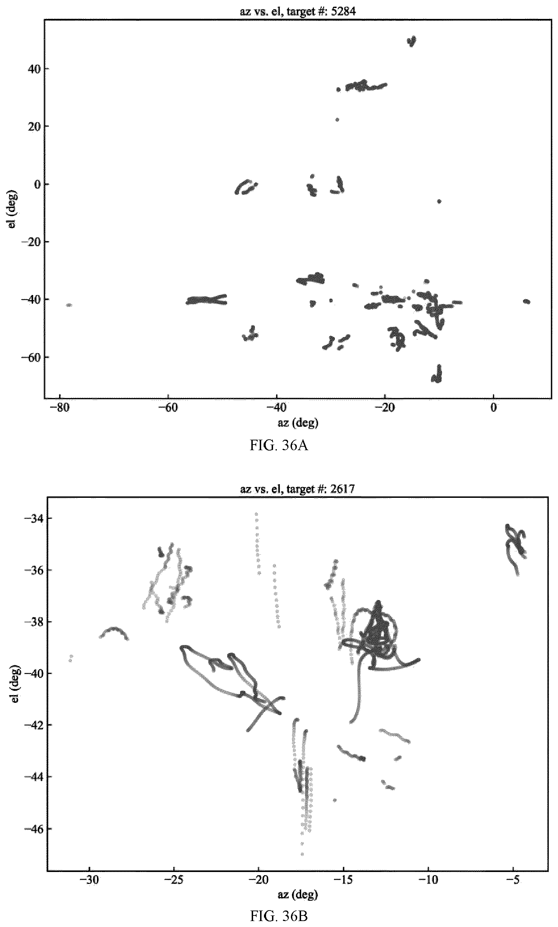

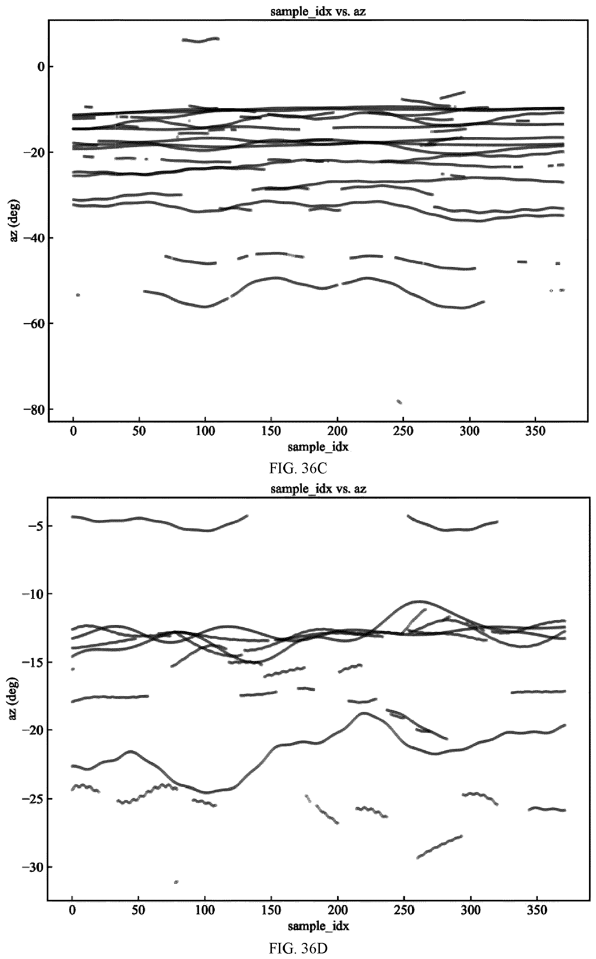

FIG. 36A-FIG. 36D show the processing results in a single-person scenario (a baby in aisle C). On a total of 500 frames of data, 128 frames of inter-frame FFT are performed, the sliding window length of the sliding window processing is 1, and the total processing is 373 times. FIG. 36A and FIG. 36C show results of azimuth elevation DoA (angle of arrival estimation) after using only NR-CFAR in the RD dimension; FIG. 36B and FIG. 36D show the processing results of azimuth elevation DBF and a second CFAR after using NR-CFAR in the RD dimension; FIG. 36A and FIG. 36B are elevation-azimuth results, and FIG. 36C and FIG. 36D are results of azimuth-processing sequence numbers. It can be seen that the method of the present embodiment (using the NR-CFAR in the RD dimension, then performing the azimuth elevation DBF and performing the second CFAR) can effectively reduce the number of invalid false target points, make the target points more aggregated, and effectively reduce the difficulty of post-processing and improve the detection effect.

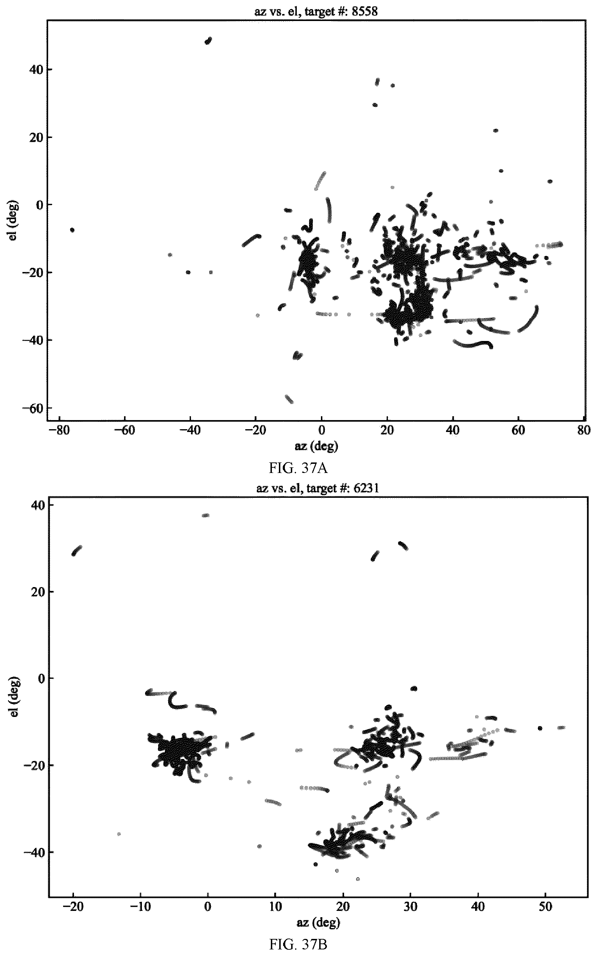

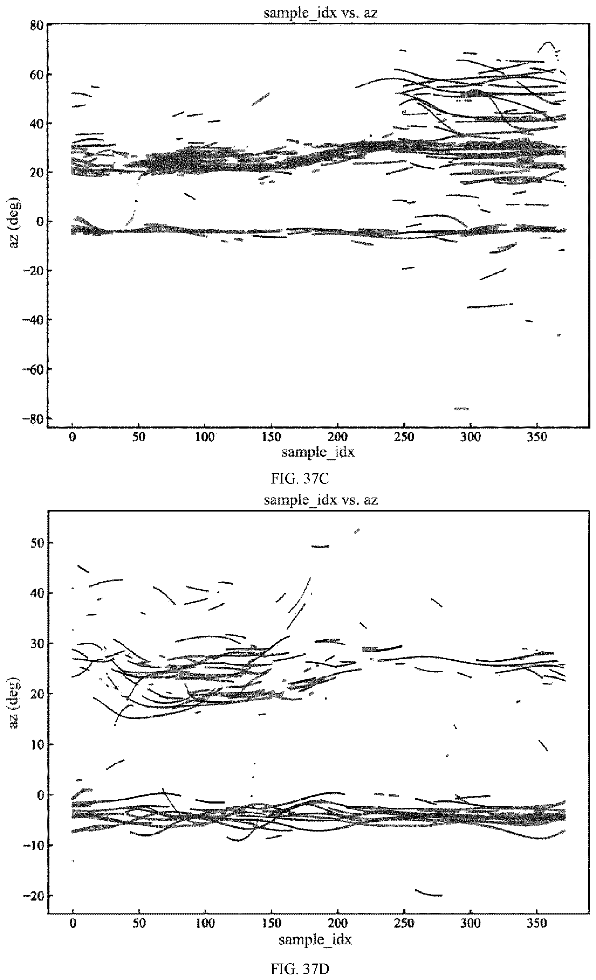

FIG. 37A-FIG. 37D show the processing results in a two-person scenario (a baby in seat B and an adult in seat A). FIG. 37A and FIG. 37C show results of azimuth elevation DoA after using only NR-CFAR in the RD dimension; FIG. 37B and FIG. 37D show the processing results of azimuth elevation DBF and a second CFAR after using NR-CFAR in the RD dimension; FIG. 37A and FIG. 37B are elevation-azimuth results, and FIG. 37C and FIG. 37D are results of azimuth-processing sequence numbers. It can be seen that the method of the present embodiment can effectively reduce the number of invalid false target points, make the target points more aggregated, and effectively reduce the difficulty of post-processing and improve the detection effect.

The embodiments of the present disclosure are not only applicable to in-vehicle human target detection and positioning scenarios, but also applicable to other similar application scenarios such as indoor personnel detection and factory personnel detection.

The above operations may be performed in the MCU or in a baseband accelerator, or may be performed in part by the MCU and in part by the baseband accelerator. For example, 1D-FFT, 2D-FFT, CFAR, and DBF are performed in the baseband accelerator, and target detection and location determination are performed in the MCU.

Compared with the prior art, the CPD processing method proposed in the embodiment of the present disclosure mainly adopts a new inter-frame integration method, which performs coherent integration on the frequency of human breathing, and improves the signal-to-noise ratio between the human target and the static strong clutter, thereby improving the detection performance; and a second CFAR detection is used again on the DBF spectrum generated by the CFAR results in the azimuth/elevation dimension, which effectively eliminates false alarms and effectively improves detection and measurement performance, thereby enabling special targets or weak targets such as infants and young children in the cabin to be accurately detected to realize applications such as in-vehicle child presence detection (CPD) and seat belt reminder (SBR).

The performance evaluation is carried out through a large number of actual measurement experiments, and the results show that the method proposed in the present disclosure can achieve a detection rate of above 99%, a missed detection rate and a false alarm rate of below 0.5% and below 1%, and has obvious advantages over the prior art.

An embodiment of the present disclosure further provides a method of target detection, which is applied to target detection in a specific target region, and the method includes: after performing a primary constant false alarm rate processing on range-Doppler data, continuing to perform a secondary constant false alarm rate processing in an angle dimension to obtain target data. The specific target region is, for example, a closed and/or semi-closed region, such as a cabin, an interior, or the like. The primary constant false alarm rate processing and the secondary constant false alarm rate processing may refer to the description in the above embodiments.

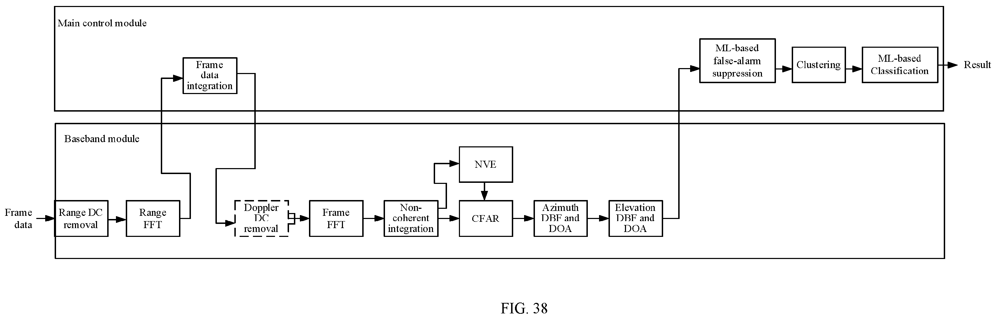

FIG. 38 is a schematic flow diagram of post-processing for a target according to an embodiment of the present application. As shown in FIG. 38, on the basis of the process structure shown in FIG. 1, after the azimuth/elevation angle estimation is performed, the output target point cloud data may be processed in combination with a machine learning model to accurately detect targets in the regions of interest. For example, for a single-pass target point cloud data obtained after azimuth/elevation estimation, a false alarm suppression technology of ML (Machine Learning, such as SVM or RF algorithm) may be combined to suppress non-ideal data such as false alarms generated by noise, stationary clutter or target multi-path, that is, for the target point cloud data outputted by such as elevation DBF & DoA, operations such as ML-based false-alarm suppression, Clustering, and/or ML-based classification are continued, wherein the clustering may be an algorithm such as agglomerative clustering or DBSCAN. At the same time, the point cloud data after clustering may also be input into the machine learning model (such as SVM or RF) that has been trained to determine whether there is someone in the current processing. If a person is exist, a location of the person may be further determined, and operations such as distinguishing and judging physical targets such as adults, children, and infants can be realized.

In the embodiment shown in FIG. 38, the influence of non-ideal factors such as noise, stationary clutter, and target multi-path can be effectively reduced, the difficulty of selecting region parameters and the difficulty of designing complex region determination logic can be effectively reduced, and the point cloud information can be more effectively utilized, especially for applications in complex scenarios, and better performance of target detection and distinction determination can be obtained.

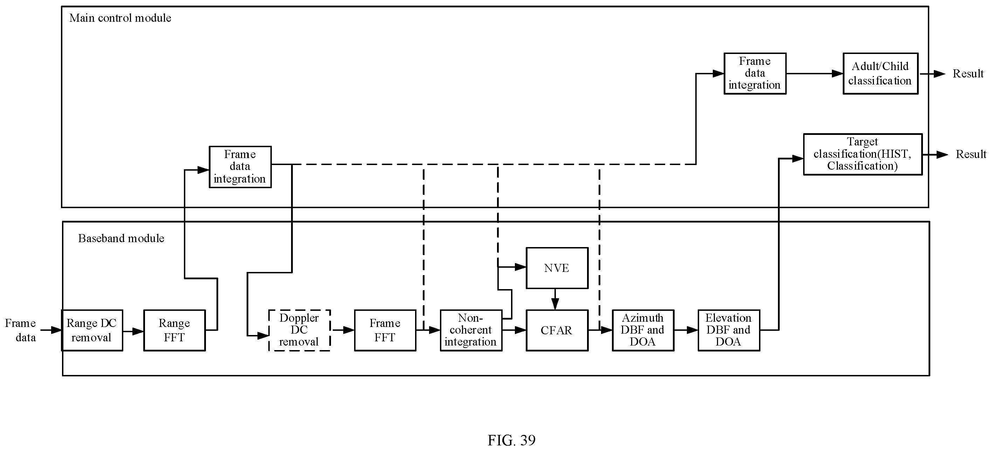

FIG. 39 is a schematic flow diagram of post-processing for a target in combination with a DL algorithm in an embodiment of the present application. As shown in FIG. 39, on the basis of the flow shown in FIG. 1, after extracting data obtained by performing at least one of operation steps such as store frame, inter-frame FFT, Non-coherent integration, and constant false alarm rate detection (CFAR), a Deep Learning algorithm (DL) may be used to determine, position, and classify a target, e.g., classify whether a physical target is an adult or a child (i.e perform an Adult/Child Classification).

For example, the following ways may be used to achieve the distinction and judging of subsequent goals: Accessing 1D-FFT data as raw input of DL, Accessing 2D-FFT data as raw input of DL, Accessing non-coherent integrated 2D-FFT data as raw input of DL and/or Accessing SNR data as raw input of DL and the like. Here, the raw data may be complex data, a modulo value of the complex data, a modulo square of the complex data, or other transformed forms, and may be a value in a linear domain, a value in a dB (log) domain, or other arithmetic forms.

Based on the flow shown in FIG. 39, by using at least one of the above ways to extract multi-frame 1D-FFT data, multi-channel data after multi-frame FFT, and power or SNR data after multi-frame FFT and SISO-combine, the extracted data is input into the constructed deep learning model (such as CNN or Transformer, etc.) to determine whether a target exists in the current processing, and to determine a current target type (e.g. determine that the target is an adult or a child, etc.).

It should be noted that the flow steps and related descriptions shown in FIG. 39 and the flow steps and related descriptions shown in FIG. 38 may be compatible with each other. For example, the Region HIST and its subsequent modules in the flow shown in FIG. 39 may be partially or entirely replaced with each other using the ML-based false alarm support and its subsequent modules in the flow shown in FIG. 38.

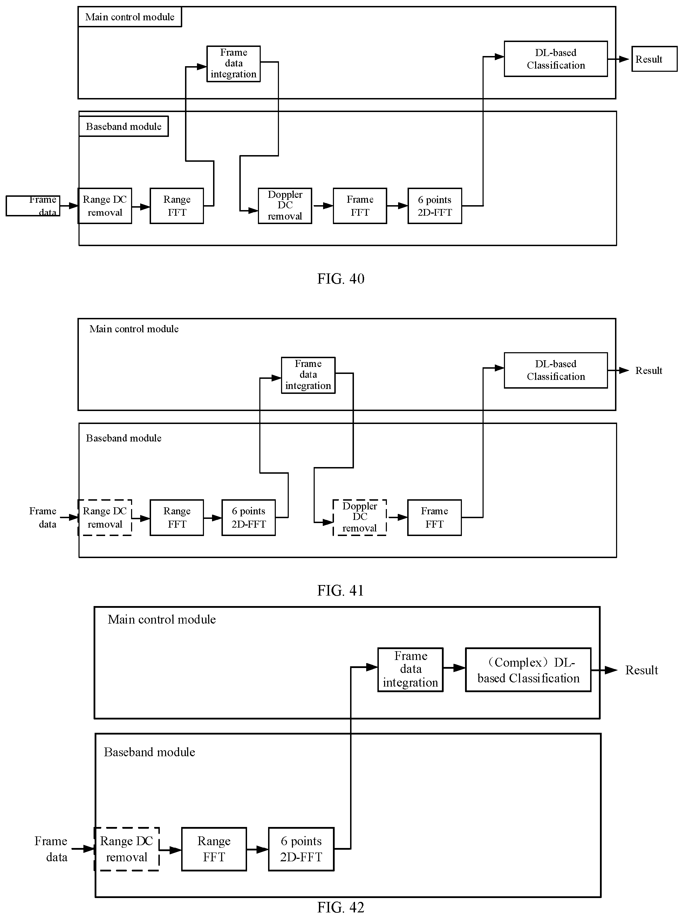

FIG. 40 is a schematic flow diagram of a target detection method combined with a DL algorithm according to an embodiment of the present application. As shown in FIG. 40, two-dimensional digital beam forming (2D-DBF) processing and DL-based classification operation may be directly performed after the inter-frame FFT processing on the basis of the flow shown in FIG. 1. That is, instead of performing CFAR processing after inter-frame FFT, 2D-DBF operation is performed on azimuth and elevation dimensions for each region of interest (or bin section) to obtain a number of RD spectrums, the number of RD spectrums corresponds to a number of the regions of interest; subsequently, the above RD spectrums are input into a constructed deep learning model (DL) for target determination. In some optional embodiments, the above region of interest and the RD spectrums may also have a one-to-many relationship, that is, at least two RD spectrums can be obtained based on one region of interest, and the detailed number can be adjusted according to actual needs.

Herein, the above DL input may be linear amplitude or power or dB domain amplitude or power, and operations such as normalization processing may also be performed simultaneously. At the same time, the deep learning model constructed above may perform classification according to the input, and the categories to be classified may be: whether there is a target, attributes of targets, specific bin section location (i.e. regional location) in which each target is located, etc.

For example, in an application scenario in which the rear row region in a vehicle is an region of interest or referred to as a target region, and the the rear row region is divided into three seat regions (section bins) and corresponding three aisle regions, after the inter-frame FFT, an azimuth and elevation 2D-DBF is performed on a center location of the six regions of interest (the regions of interest may also be set as three seat regions) to obtain six RD spectrums (or three RD spectrums) corresponding to the interest, and at least some regions of the six RD spectrums (or three RD spectrums) are input to the constructed deep learning model to perform operations such as target determination and distinguishing. For example, the constructed deep learning model classifies according to the input to determine whether there is a living target in the above region of interest. If there is a living target, it can further determine whether the living target is an adult, a child, an infant, a pet, etc., and can further determine specific location information such as which seat or aisle region or the like the living target is in.

In an embodiment shown in FIG. 40, the target detection method is a processing flow based on dual drive of model data, which has low requirements for signal processing, and can effectively avoid steps such as selection of signal processing parameters and region parameters, and design of CFAR logic and region determination logic, thereby greatly reducing the difficulty of implementation and design of the scheme.

In some optional embodiments, in all of the aforementioned target detection methods, all of the FFT processings may be windowed FFT, or may be implemented by SVA in combination with FFT at the same time.

FIG. 41 is a schematic flow diagram of another target detection method combined with a DL algorithm according to an embodiment of the present application. As shown in FIG. 41, on the basis of the flow shown in FIG. 40, after a range Fourier transform (short for Range FFT, also referred to as 1D-FFT), and after a 2D-DBF is performed, operations such as integration of frame data and inter-frame FFT processing may be performed. That is, before the inter-frame FFT, an azimuth and elevation 2D-DBF is performed on the center location of the region of interest (such as three seat regions). This embodiment can realize the scheme of parallel processing of data processing and wave transmission, and thus effectively reduce the processing time.

In some optional embodiments, in all of the above target detection methods, the multi-frame sliding window FFT may also be replaced with other time-frequency transform processing, e.g. short-time Fourier transform, fractional Fourier transform, etc., and effective detection of the target of interest can also be realized.

In some optional embodiments, in all of the above target detection methods, the multi-frame sliding window FFT may be replaced with a high-pass FIR, a Comb FIR, or a filter optimized and synthesized according to an optimization function, so as to achieve a purpose of obtaining a similar or even higher performance of the multi-frame FFT processing with less hardware resources.

As shown in FIG. 40 - FIG. 41, through the combination of 2D-FFT and DL-based Classification, an accuracy of target classification can be effectively improved. At the same time, the steps of 2D-FFT and DL classification can be flexibly set between various steps in signal processing according to actual needs, for example, after setting the 2D-FFT step after Range FFT or after inter-frame FFT or the like.

FIG. 42 is a schematic flow diagram of a further target detection method combined with a DL algorithm according to an embodiment of the present application. As shown in FIG. 42, on the basis of the flow shown in FIG. 40 or FIG. 41, that is, after the Range FFT (also referred to as 1D-FFT), and after the 2D-DBF is continued, the integration of frame data is completed, and the DL-based target classification operation (e.g. Complex DL-based Classification) is directly performed. That is, in the entire target detection process, no inter-frame FFT processing operation is performed. After 1D-FFT, 2D-DBF is directly performed on the center location of the region of interest (e.g. 3 seating regions) to obtain a corresponding number of range-frame spectrums (e.g. 3 range-frame spectrums), and then input the above range-frame spectrums into a constructed deep learning model (e.g. complex deep learning model) for operations such as target distinction and determination.

In the embodiment shown in FIG. 42, since the inter-frame FFT processing step is avoided, the amount of calculation can be effectively reduced; at the same time, if a complex deep learning model is used, higher processing gain can be obtained than FFT, thereby achieving a purpose of improving target detection performance; in addition, this embodiment also effectively shortens the processing level, and can facilitate the efficient scheduling operation and the like.

In some optional embodiments, the DBF in the target detection method shown in FIG. 40 to FIG. 42 may be replaced by Capon, MUSIC, ESPRINT, etc. and variant algorithms thereof, and at the same time, the beam forming at the center position of the region of interest may be optimized and synthesized and/or the antenna arrangement may be optimized and synthesized to further improve the performance of the target detection of the system.

It should be noted that the embodiments of the present application can be used for reference and be compatible with each other without conflict, and at the same time, the order of each functional module and whether or not to set each functional module can be adjusted according to the requirements. At the same time, for a system having a BB module and an MCU module, when the system performs target detection by transmitting electromagnetic waves and receiving corresponding echo signals, each step in each target detection method according to the embodiments of the present application can be configured to operate in the BB module and/or the MCU module according to the actual demand, the data processing capability and timeliness of the system operation, and the related examples in the figures may be used as a reference for some of the options.

In some optional embodiments, all of the above target detection methods are applicable to TDM-MIMO systems, and for systems that do not use TDM-MIMO, on the basis of presetting different phase shifts at different Tx transmissions, operations such as target detection and determination may be performed by performing a transmit beam digital synthesis (e.g by phased array) on the region of interest and then in combination with the processing flow shown in FIG. 40-FIG. 42.

In other embodiments, for a system using a pulse scheme, a pulse compression scheme, or an ultra-wideband, the method flow shown in FIG. 40 to FIG. 42 is directly used for target detection, and at the same time, a performance of target detection may be further improved by using a transmit beam digital synthesis technology. Herein, in the system with the pulse compression scheme, it is not necessary to perform the 1D-FFT process, but it is necessary to add a step of pulse compression.

It should be noted that the above processing methods corresponding to different systems with different specifications may also be applied to a system with a hybrid specification, for example, in a system having six transmitting antennas (i.e., six Tx), three of the transmitting antennas (i.e. three Tx) may be of the TDM specification, and the other three transmitting antennas may be of other specifications, and subsequent processing may just correspond to the processing methods of the above specifications.

Two-dimensional digital beam forming (2D-DBF) processing and DL-based classification operation may be directly performed after the inter-frame FFT processing. That is, instead of performing CFAR processing after inter-frame FFT, 2D-DBF operation is performed on azimuth and elevation for each region of interest (or bin section) to obtain a number of RD spectrums, the number of RD spectrums corresponds to a number of the regions of interest; subsequently, the above RD spectrums are input into a constructed deep learning model (DL) for target determination. In some optional embodiments, if the inter-frame FFT method is not adopted, but FIR, STFT (Short-Time Fourier Transform) or the like is used for processing, the above RD spectrum is outputted as a one-dimensional spectrum corresponding to FIR, and is outputted as a three-dimensional spectrum corresponding to STFT as a time-frequency transform.

It should be noted that the electromagnetic waves in the present application may include radio waves and light waves, the radio waves include short waves, medium waves, long waves, microwaves, etc., and the microwaves include centimeter waves (i.e., electromagnetic waves of 3 GHz to 30 GHz, such as electromagnetic waves of 3.1 GHz to 10.6 GHz, electromagnetic waves of 24 GHz, and the like) and millimeter waves (i.e., electromagnetic waves of 30 GHz to 300 GHz, Such as electromagnetic waves in the 60GHz frequency band, electromagnetic waves in the 77GHz frequency band (such as 77GHz ~ 81GHz, etc.)), light waves may include ultraviolet rays, visible light, infrared rays, and lasers, etc., wherein the electromagnetic wave frequency band of lasers is (3.846~7.895)*10^5GHz, i.e., lasers are included in some frequency bands of ultraviolet and visible light.

An embodiment of the present disclosure further provides an integrated circuit, which may include: a signal transmitting module configured for electromagnetic waves for target detection; a signal receiving module configured to receive an echo formed by reflection and/or scattering of the electromagnetic wave; and a processing module configured to perform signal and data processing on the echo to achieve target detection.

Optionally, the target detection is, for example, at least one of the following operations on targets in the regions of interest: determination, positioning and/or classification.

In an exemplary embodiment, the processing module includes at least a baseband unit, and the baseband unit may be configured to implement a range FFT processing, a velocity FFT processing, a primary constant false alarm rate processing, and a secondary constant false alarm rate processing as in the method of any embodiment of the present disclosure.

Optionally, in an exemplary embodiment, the processing module may further include an MCU unit, when the method includes digital beam forming and accumulating frame data, the baseband unit is configured to implement the digital beam forming, the MCU unit is configured to implement accumulating frame data.

In an exemplary embodiment, an integrated circuit may be a millimeter wave radar chip or a millimeter wave radar die.

An embodiment of the present application also provides an integrated circuit, which may include a radio frequency module, an analog signal processing module, and a digital signal processing module connected in sequence; the radio frequency module is used for generating radio frequency transmitting signals and receiving echo signals; the analog signal processing module is used for down-conversion processing of the echo signal to obtain an intermediate frequency signal; the digital processing module is used for performing analog-to-digital conversion on the intermediate frequency signal to obtain a digital signal; and processing the digital signal based on the target detection methods in the embodiments of the present application to achieve the purpose of target detection, for example, the integrated circuit may be a millimeter wave radar chip or a millimeter wave radar die. Herein, the digital processing module may include sub-units such as a BB unit and an MCU unit, and each sub-unit may be configured to execute each corresponding step in the target detection methods in the above embodiments.

In some option embodiments, the integrated circuit may be of an AiP (Antenna-In-Package) chip structure, an AoP (Antenna-On-Package) chip structure, or an AoC (Antenna-On-Chip) chip structure.

According to other embodiments of the present application, an electromagnetic wave sensor is further provided. The electromagnetic wave sensor may include an antenna, as well as an integrated circuit as previously described. Herein the integrated circuit is electrically connected with the antenna and is used for transmitting and receiving electromagnetic wave signals. For example, the electromagnetic wave sensor may include: a carrier; an integrated circuit and an antenna as described in any of the above embodiments, the integrated circuit may be provided on the carrier; the antenna may be provided on the carrier, or the antenna is integrated with the integrated circuit as an integral device provided on the carrier (i.e., at this situation, the antenna may be an antenna provided in an AiP, AoP or AoC structure); wherein, the integrated circuit is connected with the antenna (i.e., at this situation, the sensor chip or the integrated circuit is not integrated with the antenna, such as a conventional SoC, etc.), and the antenna is used for receiving and transmitting electromagnetic signals. Herein, the carrier may be a printed circuit board (PCB), and corresponding transmission wires may be PCB wires.

An embodiment of the present application provides a terminal device, which may include: a device body; and an electromagnetic wave sensor as described above provided on the device body; wherein, the electromagnetic wave sensor is used for target detection and/or communication to provide reference information to the operation of the device body.

An embodiment of the present application further provides an electronic device (which may be understood as a terminal device), and the electronic device may be represented in a form of a general purpose computing device. The components of the electronic device may include, but are not limited to: at least one processing unit, at least one storage unit, a bus connecting different system components (including the storage unit and the processing unit), a display unit, and the like. Here, the storage unit stores a program code, and the program code may be executed by the processing unit so that the processing unit executes the methods according to various exemplary embodiments of the present application described in the present specification. The memory unit may include a readable medium in a form of a volatile memory unit, such as a random access memory unit (RAM) and/or a cache memory unit, and may further include a read-only memory unit (ROM).

The storage unit may also include a program/utility having a set (at least one) of program modules, such program modules include, but are not limited to: an operating system, one or more application programs, other program modules, and program data, which may include implementations of a network environment in each or some combination of these examples.

The bus may represent one or more of several types of bus structures, including: a memory unit bus or a memory unit controller, a peripheral bus, a graphics acceleration port, a processing unit, or a local bus using any bus structure of a variety of bus structures.

The electronic device may also communicate with one or more external devices (e.g., keyboards, pointing devices, bluetooth devices, etc.), and may also communicate with one or more devices that enable a user to interact with the electronic device, and/or with any device (e.g., a router, modem, etc.) that enables the electronic device to communicate with one or more other computing devices. Such communication may perform through an input/output (I/O) interface. Moreover, the electronic device may also communicate with one or more networks (e.g. a local area network (LAN), a wide area network (WAN), and/or a public network, e.g., Internet) through a network adapter. The network adapter may communicate with other modules of the electronic device over the bus. It should be understood that, although not shown in the figures, other hardware and/or software modules may be used in connection with the electronic device, other hardware and/or software modules include, but are not limited to: microcode, device drivers, redundant processing units, external disk drive arrays, RAID systems, tape drives, and data backup storage systems, etc.

For example, the electronic device in the embodiments of the present application may further include: a device body; and an electromagnetic wave sensor as described in any of the above embodiments disposed on the device body; wherein the electromagnetic wave sensor may be used to realize functions such as target detection and/or wireless communication.

Specifically, on the basis of the above embodiments, in an optional embodiment of the present application, the electromagnetic wave sensor may be disposed outside the device body or inside the device body, and in other optional embodiments of the present application, the electromagnetic wave sensor may be partially disposed inside the device body and partially disposed outside the device body. The embodiments of the present disclosure are not limited thereto, it all depends specifically.

In an optional embodiment, the above device body may be components and products applied to fields such as smart cities, smart houses, transportation, smart homes, consumer electronics, security monitoring, industrial automation, in-cabin detection (such as smart cockpits), medical devices and health care. For example, the device body can be smart transportation device (such as automobiles, bicycles, motorcycles, ships, subways, trains, etc.), security device (such as cameras), liquid level/flow rate detection device, smart wearable device (such as bracelets, glasses, etc.), smart home device (such as sweeping robots, door locks, televisions, air conditioners, smart lights, etc.), various communication devices (such as mobile phones, tablet computers, etc.), as well as road gates, smart traffic lights, smart signs, traffic cameras and various industrial mechanical arms (or robots), etc. The device body can be various instruments for detecting vital signs parameters and various devices equipped with the instruments, such as vital signs characteristics detection in automobile cabins, indoor personnel monitoring, intelligent medical devices, consumer electronic devices, etc.

An embodiment of the present application also provides a non-transitory computer-readable storage medium storing computer-readable instructions thereon, and when the instructions are executed by the processor, the processor executes the feeder unequal length compensation method as described above.

From the above description of the embodiments, it will be readily understood by those skilled in the art that the example embodiments described herein may be implemented by software, or may be implemented by software in combination with necessary hardware. The technical scheme according to the embodiments of the present application may be embodied in the form of a software product, and the software product may be stored in a non-volatile storage medium (which may be a CD-ROM, a U disk, a mobile hard disk, etc.) or on a network, and include several instructions to cause a computing device (which may be a personal computer, a server, or a network device, etc.) to perform the above methods according to the embodiments of the present application.

The software product may employ any combination of one or more readable media. The readable medium may be a readable signal medium or a readable storage medium. The readable storage medium may be, for example, but is not limited to, an electrical, magnetic, optical, electromagnetic, infrared, or semiconductor system, apparatus, or device, or a combination of any of the above. More specific examples (with a non-exhaustive list) of readable storage media include: an electrical connection having one or more wires, a portable disk, a hard disk, a random access memory (RAM), a read-only memory (ROM), an erasable programmable read-only memory (EPROM or flash memory), an optical fiber, a portable compact disk read-only memory (CD-ROM), an optical storage device, a magnetic storage device, or any suitable combination of the above.

The computer-readable storage medium may include data signal propagated in a baseband or as part of a carrier wave, the data signal carries readable program code. Such propagated data signals may take a variety of forms, including, but not limited to, electromagnetic signals, optical signals, or any suitable combination of the above. The readable storage medium may also be any readable medium other than a readable storage medium, the storage medium may transmit, propagate, or transmit a program for use by or in connection with an instruction execution system, apparatus, or device. The program code contained on the readable storage medium may be transmitted using any suitable medium, including, but not limited to, wireless, wired, fiber optic cable, RF, etc., or any suitable combination of the above.

The program code for performing the operations of the present application may be written in any combination of one or more programming languages, the programming languages include object-oriented programming languages, such as Java, C++, and the like, and also include conventional procedural programming languages, such as the "C" language or similar programming languages. The program code may be executed entirely on the user computing device, executed partially on the user device, executed as a stand-alone software package, executed partially on the user computing device and partially on a remote computing device, or executed entirely on a remote computing device or server. In a case that a remote computing device is involved, the remote computing device may be connected with the user computing device over any kind of network, including a local area network (LAN) or a wide area network (WAN), or may be connected with an external computing device (e.g., using an Internet service provider to connect over the Internet).

The above computer-readable medium carries one or more programs, and when the one or more programs are executed by one of the devices, the computer-readable medium is enable to implement the aforementioned functions.

Those skilled in the art can understand that the above modules may be distributed in an apparatus according to the description of the embodiments, or may be changed accordingly to locate in one or more apparatuses different from the present embodiment. The modules of the above embodiments may be merged into one module, or may be further split into a plurality of sub-modules.

According to an embodiment of the present application, a computer program including computer programs or instructions that, when the computer programs or instructions are executed by a processor, the above-described method can be performed. In an optional embodiment, the above integrated circuit may be a millimeter wave radar chip. The types of digital function modules in the integrated circuit can be determined according to the actual requirements. For example, in the millimeter wave radar chip, the data processing module may be used for operations such as range-dimensional Doppler transform, velocity-dimensional Doppler transform, constant false alarm rate detection, direction of wave arrival detection, point cloud processing, etc., for obtaining information such as range, angle, velocity, height, micro-Doppler motion characteristics, shape, size, surface roughness and dielectric characteristics of the target.

It should be noted that, the radio device may achieve functions such as target detection and/or communication by transmitting and receiving radio signals to provide detection target information and/or communication information to the device body, thereby assisting or even controlling the operation of the device body.

For example, when the above-mentioned device body is applied to advanced driving assistance system (ADAS), the radio device (such as millimeter wave radar) as on-board sensor can assist ADAS system to achieve application scenarios such as adaptive cruise, autonomous emergency braking (AEB), blind spot detection (BSD), lane change assist (LCA), rear cross traffic alert (RCTA), parking assistance, rear vehicle warning, collision avoidance, pedestrian detection, cabin living target detection (CPD), etc.

Those of ordinary skill in the art will appreciate that all or some of the steps in the methods, systems, functional modules/units in the apparatus disclosed above may be implemented as software, firmware, hardware, and suitable combinations thereof. In a hardware embodiment, the division between functional modules/units mentioned in the above description does not necessarily correspond to a division of physical components; for example, one physical component may have multiple functions, or one function or step may be performed by several physical components in cooperation. Some components or all of the components may be implemented as software executed by a processor, such as a digital signal processor or microprocessor, or implemented as hardware, or implemented as an integrated circuit, such as an application specific integrated circuit. Such software may be distributed on a computer-readable medium, computer-readable medium may include a computer storage medium (or non-transitory medium) and a communication medium (or transitory medium). As is well known to those of ordinary skill in the art, the term computer storage medium includes volatile and non-volatile, removable and non-removable medium implemented in any method or technique for storing information, such as computer-readable instructions, data structures, program modules, or other data. Computer storage media include, but are not limited to, RAM, ROM, EEPROM, flash memory or other memory technology, CD-ROM, digital versatile disk (DVD) or other optical disk storage, magnetic cartridges, magnetic tape, magnetic disk storage or other magnetic storage apparatuses, or any other medium that can be used to store desired information and can be accessed by a computer. Furthermore, it is well known to those of ordinary skill in the art that communication media typically contain computer-readable instructions, data structures, program modules, or other data in modulated data signals such as carrier waves or other transmission mechanisms, and may include any information delivery medium.

## Claims

1. A method of target detection, performed by a processor, the method comprising:
accumulating frame data from 1D-Fourier Transform, 1D-FFT, data obtained through range FFT processing of an echo signal; wherein the frame data refers to data corresponding to the echo signal formed after a frame of detection signal is reflected by a target, and the frame of detection signal comprises a plurality of chirp signals;
wherein the accumulating frame data from 1D-FFT data obtained through range FFT processing of an echo signal comprises:
performing 1D-FFT processing on chirp data of each frame to obtain the 1D-FFT data; and
accumulating the 1D-FFT data to obtain accumulated multi-frame 1D-FFT data;
conducting inter-frame FFT processing on the accumulated multi-frame 1D-FFT data to generate a Range-Doppler, RD, spectrum; and
detecting target within a region of interest based on the RD spectrum.

2. The method of target detection according to claim 1, wherein detecting the target in the region of interest based on the RD spectrum comprises:
conducting a primary CFAR processing on the RD spectrum to identify candidate target detection points; and
applying a secondary CFAR processing to the candidate target detection points to facilitate target detection based on the processing results.

3. The method of target detection according to claim 2, wherein conducting the primary CFAR processing on the RD spectrum to identify the candidate target detection points comprises:
conducting non-coherent integration on range-velocity dimension data from multiple channels and subsequently applying the primary CFAR processing based on noise estimation using the results of the non-coherent integration;
or
performing non-coherent integration on range-velocity dimension data of multiple channels, and based on the non-coherent integration result, performing the primary CFAR processing based on the noise estimation comprising:
performing the non-coherent integration on the range velocity dimension data of the multiple channels, estimating a noise floor of each range bin after the non-coherent integration, obtaining noise floor estimation value of each range bin, and performing a non-coherent CFAR processing by using the noise floor estimation value, wherein when estimating the noise floor of each range bin after the non-coherent integration, the noise floor of each range bin is adjusted by using a global noise floor;
or
wherein performing the target detection based on the secondary CFAR processing result comprises:
pre-dividing the region of interest into multiple target sub-regions, determining the number and locations of target points in each sub-region based on the locations of the detected target points, and determining the presence of a target in the current sub-region based on whether the ratio of the number of target points in each sub-region to the total number of targets exceeds a preset ratio value.

4. The method of target detection according to claim 2, wherein applying the secondary CFAR processing to the candidate target detection points comprises:
performing azimuth directional beamforming, DBF, on the candidate target detection points, and performing azimuth CFAR processing in combination with noise floor estimation, to obtain an azimuth dimension target screening result; and/or performing elevation DBF on the candidate target detection points, and performing an elevation CFAR processing in combination with the noise floor estimation result, to obtain an elevation dimension target screening result; and
performing azimuth-elevation two-dimensional DBF on the candidate target detection points, and performing an azimuth elevation CFAR processing in combination with the noise floor estimation result, to obtain an azimuth elevation two-dimensional target screening result.

5. The method of target detection according to claim 4, wherein,
during the CFAR processing, one or more of the following operations are performed on the candidate target detection points to obtain the target detection result: global maximum value screening, first threshold value screening, and second threshold value screening, wherein the global maximum value screening is used for screening whether a global maximum value is a target detection point, the first threshold value screening determines whether a current candidate screening result is a target detection point according to a relationship between a candidate screening result and a noise floor estimation value, and the second threshold value screening determines whether the current candidate screening result is a target detection point according to a relationship between the candidate screening result and the global maximum value.

6. The method of target detection according to claim 5, wherein,
the global maximum value screening comprises: determining whether a difference between a digital beamforming spectral amplitude value corresponding to a current global maximum value and the noise floor estimation value is within a first predefined range, and if the difference is within the first predefined range, the candidate screening result corresponding to the global maximum value is a target detection point;
the first threshold value screening comprises: determining whether a difference between the current candidate screening result and the noise floor estimation value is within a second predefined range, and if the difference is within the second predefined range, the current candidate screening result is a target detection point; and
the second threshold value screening comprises: determining whether a difference between a power value of the current candidate screening result and power of the global maximum value is within a third predefined range, and if the difference is within the third predefined range, the current candidate screening result is a target detection point.

7. The method of target detection according to claim 1, wherein conducting the inter-frame FFT processing on the accumulated multi-frame 1D-FFT data to generate the RD spectrum and implementing detection of the target in the region of interest based on the RD spectrum comprises:
extracting target data from the accumulated multi-frame 1D-FFT data;
applying sliding windowing to the target data; and
conducting digital signal processing on windowed data to derive target information;
wherein a duration of sliding window is commensurate with an order of magnitude of a target's periodic motion cycle.

8. The method of target detection according to claim 7, wherein,
extracting target data from the accumulated multi-frame 1D-FFT data comprises:
extracting at least a portion of data or processed data from each frame data obtained after range FFT processing as the target data; or
determining parameters corresponding to each range bin in each frame data based on the range FFT processing results to generate the target data;
and conducting digital signal processing on the windowed data to derive the target information comprises:
performing a noise floor estimation within a predefined range, where an upper bound of the predefined range is determined based on a noise floor estimation result of a range bin with a farthest distance; and
performing constant false alarm rate, CFAR, detection based on an estimated noise floor to obtain the target information;
or
extracting the target data from the frame data obtained after the range FFT processing comprises:
extracting data within a preset distance range from the frame data obtained after the range FFT processing as the target data; and/or,
conducting the digital signal processing on the windowed data, comprises:
performing the digital signal processing on data within a preset Doppler range from the windowed data;
or
wherein the data is cached utilizing a Ring FIFO, the method further comprises: extracting data from the Ring FIFO and reordering the extracted data;
or
wherein applying sliding windowing to the target data and conducting the digital signal processing on the windowed data to derive the target information comprises:
applying sliding windowing to the target data in accordance with periods of various physiological parameters of a living subject, and processing the windowed data associated with each distinct sliding window length via digital signal processing;
wherein the periods of the physiological parameters associated with each sliding window vary, and the duration of each sliding window is commensurate with the period of its corresponding physiological parameter in terms order of magnitude.

9. The method of target detection according to claim 1 or 7, wherein detecting the target in the region of interest based on the RD spectrum comprises:
applying individual constant false alarm rate, CFAR, processing to at least two transceiver channels based on the RD spectrum, to acquire candidate target data for each of the at least two transceiver channels; and
processing the acquired candidate target data from at least two transceiver channels to yield final target data.

10. The method of target detection according to claim 9, wherein applying individual constant false alarm rate, CFAR, processing to at least two transceiver channels based on the RD spectrum comprises:
separately applying CFAR processing to at least two transceiver channels based on the RD spectrum; or
conducting a noise floor estimation for each transceiver channel undergoing CFAR processing in accordance with the RD spectrum, to obtain a noise floor estimation result for each individual transceiver channel; and utilizing the noise floor estimation result of each individual transceiver channel to individually perform CFAR detection on the corresponding transceiver channel;
or
wherein processing the acquired candidate target data from at least two transceiver channels comprises:
performing CFAR detection on currently acquired candidate target data against a preset threshold to obtain the final target set.

11. The method of target detection according to any one of claims 7-10, the method further comprising:
validating targets within each predefined zone based on targets located therein, wherein the predefined zones are obtained by partitioning the surveillance area; and
disseminating the detection outcomes for each predefined zone based on validated targets.

12. The method of target detection according to claim 1, wherein for a multi-channel application scenario, implementing the detection of the target in the region of interest based on the RD spectrum comprises:
after performing non-coherent integration on the RD spectrum of multiple channels, continuing CFAR processing and Direction of Arrival, DoA, estimation based on noise estimation to achieve target detection; or
after performing CFAR processing respectively on the RD spectrum of multiple channels, continuing binary integration processing in the channel domain and DoA estimation, and then performing any one of the following operations on the target: determination, positioning, and classification;
or
wherein detecting the target in the region of interest based on the RD spectrum comprises:
based on the extraction of at least a portion of the integrated data from multiple frames, inter-frame Fourier Transform, FFT, data, non-coherent integrated data, and/or CFAR detection data, performing target identification /recognition.

13. An integrated circuit comprising:
a signal transmitting module configured to transmit electromagnetic waves for target detection;
a signal receiving module configured to receive echoes formed by reflection and/or scattering of the electromagnetic waves; and
a processing module configured to process the echoes according to any one of the target detection methods according to any one of claims 1-12 to detect targets.

14. An electromagnetic wave sensor, comprising:
a carrier;
an integrated circuit according to claim 13 mounted on the carrier;
an antenna, either mounted on the carrier or integrated with the integrated circuit as a unified device on the carrier;
wherein the integrated circuit is linked to the antenna and configured to transmit an electromagnetic wave signal and/or receive an echo signal.

15. A terminal device comprising:
a device body; and
the electromagnetic wave sensor according to claim 14 mounted on the device body;
wherein the electromagnetic wave sensor is utilized for target detection and/or communication to supply reference information for the operation of the device body.

## Patentansprüche

1. Verfahren zur Zielerkennung, durchgeführt durch einen Prozessor, wobei das Verfahren Folgendes umfasst:
Akkumulieren von Frame-Daten aus 1D-Fourier-Transformations-Daten, 1D-FFT-Daten, die durch Bereich-FFT-Verarbeitung eines Echosignals erlangt wurden; wobei sich die Frame-Daten auf Daten beziehen, die dem Echosignal entsprechen, das ausgebildet wird, nachdem ein Frame eines Erkennungssignals durch ein Ziel reflektiert wird, und der Frame des Erkennungssignals eine Vielzahl von Chirp-Signalen umfasst;
wobei das Akkumulieren von Frame-Daten aus 1D-FFT-Daten, die durch Bereich-FFT-Verarbeitung eines Echosignals erlangt wurden, Folgendes umfasst:
Durchführen von 1D-FFT-Verarbeitung an Chirp-Daten jedes Frames, um die 1D-FFT-Daten zu erlangen; und
Akkumulieren der 1D-FFT-Daten, um akkumulierte Multi-Frame 1D-FFT-Daten zu erlangen;
Ausführen von Inter-Frame-FFT-Verarbeitung an den akkumulierten Multi-Frame-1D-FFT-Daten, um ein Bereich-Doppler-Spektrum, RD-Spektrum, zu erzeugen; und
Erkennen von Ziel innerhalb einer Region von Interesse basierend auf dem RD-Spektrum.

2. Verfahren zur Zielerkennung nach Anspruch 1, wobei das Erkennen des Ziels in der Region von Interesse basierend auf dem RD-Spektrum Folgendes umfasst:
Ausführen einer primären CFAR-Verarbeitung an dem RD-Spektrum, um Kandidatenzielerkennungspunkte zu identifizieren; und
Anwenden einer sekundären CFAR-Verarbeitung auf die Kandidatenzielerkennungspunkte, um Zielerkennung basierend auf den Verarbeitungsergebnissen zu erleichtern.

3. Verfahren zur Zielerkennung nach Anspruch 2, wobei das Ausführen der primären CFAR-Verarbeitung an dem RD-Spektrum, um die Kandidatenzielerkennungspunkte zu identifizieren, Folgendes umfasst:
Ausführen von nicht kohärenter Integration an Bereich-Geschwindigkeit-Dimensionsdaten von mehreren Kanälen und anschließendes Anwenden der primären CFAR-Verarbeitung basierend auf einer Rauschschätzung unter Verwendung der Ergebnisse der nicht kohärenten Integration;
oder
Durchführen von nicht kohärenter Integration an Bereich-Geschwindigkeit-Dimensionsdaten mehrerer Kanäle und, basierend auf dem Ergebnis der nicht kohärenten Integration, Durchführen der primären CFAR-Verarbeitung basierend auf der Rauschschätzung, umfassend:
Durchführen der nicht kohärenten Integration an den Bereich-Geschwindigkeit-Dimensionsdaten der mehreren Kanäle, Schätzen eines Grundrauschens jedes Bereich-Bins nach der nicht kohärenten Integration, Erlangen eines Grundrauschen-Schätzwerts jedes Bereich-Bins und Durchführen einer nicht kohärenten CFAR-Verarbeitung unter Verwendung des Grundrauschen-Schätzwerts, wobei beim Schätzen des Grundrauschens jedes Bereich-Bins nach der nicht kohärenten Integration das Grundrauschen jedes Bereich-Bins unter Verwendung eines globalen Grundrauschens angepasst wird;
oder
wobei das Durchführen der Zielerkennung basierend auf dem sekundären CFAR-Verarbeitungsergebnis Folgendes umfasst:
Vorabteilen der Region von Interesse in mehrere Zielunterregionen, Bestimmen der Anzahl und Positionen von Zielpunkten in jeder Unterregion basierend auf den Positionen der erkannten Zielpunkte und Bestimmen des Vorhandenseins eines Ziels in der aktuellen Unterregion basierend darauf, ob das Verhältnis der Anzahl von Zielpunkten in jeder Unterregion zu der Gesamtanzahl von Zielen einen voreingestellten Verhältniswert überschreitet.

4. Verfahren zur Zielerkennung nach Anspruch 2, wobei das Anwenden der sekundären CFAR-Verarbeitung auf die Kandidatenzielerkennungspunkte Folgendes umfasst:
Durchführen von Azimut-Richtstrahlformung, DBF, an den Kandidatenzielerkennungspunkten und Durchführen von Azimut-CFAR-Verarbeitung in Kombination mit einer Grundrauschen-Schätzung, um ein Azimut-Dimensionszieluntersuchungsergebnis zu erlangen; und/oder Durchführen von Höhen-DBF an den Kandidatenzielerkennungspunkten und Durchführen einer Höhen-CFAR-Verarbeitung in Kombination mit dem Grundrauschen-Schätzungsergebnis, um ein Höhen-Dimensionszieluntersuchungsergebnis zu erlangen; und
Durchführen von zweidimensionaler Azimut-Höhen-DBF an den Kandidatenzielerkennungspunkten und Durchführen einer Azimut-Höhen-CFAR-Verarbeitung in Kombination mit dem Grundrauschen-Schätzungsergebnis, um ein zweidimensionales Azimut-Höhen-Zieluntersuchungsergebnis zu erlangen.

5. Verfahren zur Zielerkennung nach Anspruch 4, wobei
während der CFAR-Verarbeitung eine oder mehrere der folgenden Operationen an den Kandidatenzielerkennungspunkten durchgeführt werden, um das Zielerkennungsergebnis zu erlangen: globale Maximalwert-Untersuchung, erste Schwellenwert-Untersuchung und zweite Schwellenwert-Untersuchung, wobei die globale Maximalwert-Untersuchung zum Untersuchen verwendet wird, ob ein globaler Maximalwert ein Zielerkennungspunkt ist, die erste Schwellenwert-Untersuchung bestimmt, ob ein aktuelles Kandidatenuntersuchungsergebnis ein Zielerkennungspunkt ist, gemäß einer Beziehung zwischen einem Kandidatenuntersuchungsergebnis und einem Grundrauschen-Schätzwert, und die zweite Schwellenwert-Untersuchung bestimmt, ob das aktuelle Kandidatenuntersuchungsergebnis ein Zielerkennungspunkt ist, gemäß einer Beziehung zwischen dem Kandidatenuntersuchungsergebnis und dem globalen Maximalwert.

6. Verfahren zur Zielerkennung nach Anspruch 5, wobei
die globale Maximalwert-Untersuchung Folgendes umfasst: Bestimmen, ob eine Differenz zwischen einem digitalen Strahlformungs-Spektralamplitudenwert, der einem aktuellen globalen Maximalwert entspricht, und dem Grundrauschen-Schätzwert innerhalb eines ersten vordefinierten Bereichs liegt, und wobei, falls die Differenz innerhalb des ersten vordefinierten Bereichs liegt, das Kandidatenuntersuchungsergebnis, das dem globalen Maximalwert entspricht, ein Zielerkennungspunkt ist;
die erste Schwellenwert-Untersuchung Folgendes umfasst: Bestimmen, ob eine Differenz zwischen dem aktuellen Kandidatenuntersuchungsergebnis und dem Grundrauschen-Schätzwert innerhalb eines zweiten vordefinierten Bereichs liegt, und wobei, falls die Differenz innerhalb des zweiten vordefinierten Bereichs liegt, das aktuelle Kandidatenuntersuchungsergebnis ein Zielerkennungspunkt ist; und
die zweite Schwellenwert-Untersuchung Folgendes umfasst: Bestimmen, ob eine Differenz zwischen einem Leistungswert des aktuellen Kandidatenuntersuchungsergebnisses und einer Leistung des globalen Maximalwerts innerhalb eines dritten vordefinierten Bereichs liegt, und wobei, falls die Differenz innerhalb des dritten vordefinierten Bereichs liegt, das aktuelle Kandidatenuntersuchungsergebnis ein Zielerkennungspunkt ist.

7. Verfahren zur Zielerkennung nach Anspruch 1, wobei das Ausführen der Inter-Frame-FFT-Verarbeitung an den akkumulierten Multi-Frame-1D-FFT-Daten, um das RD-Spektrum zu erzeugen, und Implementieren von Erkennung des Ziels in der Region von Interesse basierend auf dem RD-Spektrum Folgendes umfasst:
Extrahieren von Zieldaten aus den akkumulierten Multi-Frame-1D-FFT-Daten;
Anwenden eines gleitenden Fensterns auf die Zieldaten; und
Ausführen von digitaler Signalverarbeitung an gefensterten Daten, um Zielinformationen abzuleiten;
wobei eine Dauer des gleitenden Fensters einer Größenordnung eines periodischen Bewegungszyklus eines Ziels angeglichen ist.

8. Verfahren zur Zielerkennung nach Anspruch 7, wobei
das Extrahieren von Zieldaten aus den akkumulierten Multi-Frame-1D-FFT-Daten Folgendes umfasst:
Extrahieren mindestens eines Teils von Daten oder verarbeiteten Daten aus allen Frame-Daten, die nach der Bereich-FFT-Verarbeitung erlangt werden, als die Zieldaten; oder
Bestimmen von Parametern, die jedem Bereich-Bin in allen Frame-Daten entsprechen, basierend auf den Bereich-FFT-Verarbeitungsergebnissen, um die Zieldaten zu erzeugen;
und das Ausführen von digitaler Signalverarbeitung an den gefensterten Daten, um die Zielinformationen abzuleiten, Folgendes umfasst:
Durchführen einer Grundrauschen-Schätzung innerhalb eines vordefinierten Bereichs, wobei eine obere Grenze des vordefinierten Bereichs basierend auf einem Grundrauschen-Schätzungsergebnis eines Bereich-Bins mit einem weitesten Abstand bestimmt wird; und
Durchführen von konstanter Falschalarmratenerkennung, CFAR-Erkennung, basierend auf einem geschätzten Grundrauschen, um die Zielinformationen zu erlangen;
oder
das Extrahieren der Zieldaten aus den Frame-Daten, die nach der Bereich-FFT-Verarbeitung erlangt werden, Folgendes umfasst:
Extrahieren von Daten innerhalb eines voreingestellten Abstandsbereichs aus den Frame-Daten, die nach der Bereich-FFT-Verarbeitung erlangt werden, als die Zieldaten; und/oder
das Ausführen der digitalen Signalverarbeitung an den gefensterten Daten Folgendes umfasst:
Durchführen der digitalen Signalverarbeitung an Daten innerhalb eines voreingestellten Dopplerbereichs aus den gefensterten Daten;
oder
wobei die Daten unter Nutzung eines Ring-FIFO zwischengespeichert werden, wobei das Verfahren ferner Folgendes umfasst: Extrahieren von Daten aus dem Ring-FIFO und Neuordnen der extrahierten Daten;
oder
wobei das Anwenden eines gleitenden Fensterns auf die Zieldaten und das Ausführen der digitalen Signalverarbeitung an den gefensterten Daten, um die Zielinformationen abzuleiten, Folgendes umfasst:
Anwenden eines gleitenden Fensterns auf die Zieldaten in Übereinstimmung mit Zeiträumen verschiedener physiologischer Parameter eines lebenden Subjekts und Verarbeiten der gefensterten Daten, die jeder unterschiedlichen gleitenden Fensterlänge zugeordnet sind, durch digitale Signalverarbeitung;
wobei die Perioden der physiologischen Parameter, die jedem gleitenden Fenster zugeordnet sind, variieren und die Dauer jedes gleitenden Fensters der Periode seines entsprechenden physiologischen Parameters in Bezug auf die Größenordnung angeglichen ist.

9. Verfahren zur Zielerkennung nach Anspruch 1 oder 7, wobei das Erkennen des Ziels in der Region von Interesse basierend auf dem RD-Spektrum Folgendes umfasst:
Anwenden von individueller konstanter Falschalarmratenverarbeitung, CFAR-Verarbeitung, auf mindestens zwei Transceiver-Kanäle basierend auf dem RD-Spektrum, um Kandidatenzieldaten für jeden der mindestens zwei Transceiver-Kanäle zu erfassen; und
Verarbeiten der erfassten Kandidatenzieldaten von mindestens zwei Transceiver-Kanälen, um endgültige Zieldaten zu erreichen.

10. Verfahren zur Zielerkennung nach Anspruch 9, wobei das Anwenden von individueller konstanter Falschalarmratenverarbeitung, CFAR-Verarbeitung, auf mindestens zwei Transceiver-Kanäle basierend auf dem RD-Spektrum Folgendes umfasst:
getrenntes Anwenden von CFAR-Verarbeitung auf mindestens zwei Transceiver-Kanäle basierend auf dem RD-Spektrum; oder
Ausführen einer Grundrauschen-Schätzung für jeden Transceiver-Kanal, der einer CFAR-Verarbeitung in Übereinstimmung mit dem RD-Spektrum unterzogen wird, um ein Grundrauschen-Schätzungsergebnis für jeden individuellen Transceiver-Kanal zu erlangen; und Nutzen des Grundrauschen-Schätzungsergebnisses jedes individuellen Transceiver-Kanals, um eine CFAR-Erkennung auf dem entsprechenden Transceiver-Kanal individuell durchzuführen;
oder
wobei das Verarbeiten der erfassten Kandidatenzieldaten von mindestens zwei Transceiver-Kanälen Folgendes umfasst:
Durchführen von CFAR-Erkennung an aktuell erfassten Kandidatenzieldaten gegen einen voreingestellten Schwellenwert, um den endgültigen Zielsatz zu erlangen.

11. Verfahren zur Zielerkennung nach einem der Ansprüche 7-10, wobei das Verfahren ferner Folgendes umfasst:
Validieren von Zielen innerhalb jeder vordefinierten Zone basierend auf darin befindlichen Zielen, wobei die vordefinierten Zonen durch Aufteilen der Überwachungsfläche erlangt werden; und
Verbreiten der Erkennungsresultats für jede vordefinierte Zone basierend auf validierten Zielen.

12. Verfahren zur Zielerkennung nach Anspruch 1, wobei für ein Mehrkanalanwendungsszenario das Implementieren der Erkennung des Ziels in der Region von Interesse basierend auf dem RD-Spektrum Folgendes umfasst:
nach Durchführen von nicht kohärenter Integration an dem RD-Spektrum mehrerer Kanäle, Fortsetzen von CFAR-Verarbeitung und Schätzung einer Einfallsrichtung, DoA, basierend auf einer Rauschschätzung, um eine Zielerkennung zu erhalten; oder
nach Durchführen von CFAR-Verarbeitung jeweils auf dem RD-Spektrum mehrerer Kanäle, Fortsetzen von binärer Integrationsverarbeitung in der Kanaldomäne und DoA-Schätzung und dann Durchführen einer beliebigen der folgenden Operationen an dem Ziel: Bestimmung, Positionierung und Klassifizierung;
oder
wobei das Erkennen des Ziels in der Region von Interesse basierend auf dem RD-Spektrum Folgendes umfasst:
basierend auf der Extraktion mindestens eines Teils der integrierten Daten aus mehreren Frames, Inter-Frame-Fourier-Transformations-Daten, FFT-Daten, nicht kohärenten integrierten Daten und/oder CFAR-Erkennungsdaten, Durchführen von Zielidentifizierung/-anerkennung.

13. Integrierte Schaltung, umfassend:
ein Signalübertragungsmodul, das dazu konfiguriert ist, elektromagnetische Wellen zur Zielerkennung zu übertragen;
ein Signalempfangsmodul, das dazu konfiguriert ist, Echos zu empfangen, die durch Reflexion und/oder Streuung der elektromagnetischen Wellen ausgebildet werden; und
ein Verarbeitungsmodul, das dazu konfiguriert ist, die Echos nach einem der Zieldetektionsverfahren nach einem der Ansprüche 1-12 zu verarbeiten, um Ziele zu erkennen.

14. Sensor für elektromagnetische Wellen, umfassend:
einen Träger;
eine integrierte Schaltung nach Anspruch 13, die an dem Träger montiert ist;
eine Antenne, die entweder auf dem Träger montiert oder mit der integrierten Schaltung als eine einheitliche Vorrichtung auf dem Träger integriert ist;
wobei die integrierte Schaltung mit der Antenne verbunden und dazu konfiguriert ist, ein elektromagnetisches Wellensignal zu übertragen und/oder ein Echosignal zu empfangen.

15. Endgerätevorrichtung, umfassend:
einen Vorrichtungskörper; und
den Sensor für elektromagnetische Wellen nach Anspruch 14, der an dem Vorrichtungskörper montiert ist;
wobei der Sensor für elektromagnetische Wellen zur Zielerkennung und/oder Kommunikation genutzt wird, um Referenzinformationen für den Betrieb des Vorrichtungskörpers zu liefern.

## Revendications

1. Procédé de détection de cible, réalisé par un processeur, le procédé comprenant :
l'accumulation de données de trame à partir de données de transformée de Fourier 1D, 1D-FFT, obtenues au travers d'un traitement FFT de portée d'un signal d'écho ; dans lequel les données de trame se réfèrent à des données correspondant au signal d'écho formé après qu'une trame de signal de détection est réfléchie par une cible, et la trame de signal de détection comprend une pluralité de signaux chirp ;
dans lequel l'accumulation de données de trame à partir de données 1D-FFT obtenues au travers du traitement FFT de portée d'un signal d'écho comprend :
la réalisation d'un traitement 1D-FFT sur des données chirp de chaque trame pour obtenir les données 1D-FFT ; et
l'accumulation des données 1D-FFT pour obtenir des données 1D-FFT multi-trames accumulées ;
l'exécution d'un traitement FFT inter-trame sur les données 1D-FFT multi-trame accumulées pour générer un spectre de portée Doppler, RD ; et
la détection d'une cible au sein d'une région d'intérêt sur la base du spectre RD.

2. Procédé de détection de cible selon la revendication 1, dans lequel la détection de la cible dans la région d'intérêt sur la base du spectre RD comprend :
l'exécution d'un traitement CFAR primaire sur le spectre RD pour identifier des points de détection de cible candidate ; et
l'application d'un traitement CFAR secondaire aux points de détection de cible candidate pour faciliter une détection de cible sur la base des résultats du traitement.

3. Procédé de détection de cible selon la revendication 2, dans lequel l'exécution du traitement CFAR primaire sur le spectre RD pour identifier les points de détection de cible candidate comprend :
l'exécution d'une intégration non cohérente sur des données de dimension de vitesse de portée à partir de plusieurs canaux et l'application ultérieure du traitement CFAR primaire sur la base d'une estimation de bruit à l'aide des résultats de l'intégration non cohérente ;
ou
la réalisation d'une intégration non cohérente sur des données de dimension de vitesse de portée de plusieurs canaux, et sur la base du résultat d'intégration non cohérente, la réalisation du traitement CFAR primaire sur la base de l'estimation de bruit comprenant :
la réalisation de l'intégration non cohérente sur les données de dimension de vitesse de portée des multiples canaux, l'estimation d'un plancher de bruit de chaque cellule de portée après l'intégration non cohérente, l'obtention d'une valeur d'estimation de plancher de bruit de chaque cellule de portée, et la réalisation d'un traitement CFAR non cohérent en utilisant la valeur d'estimation de plancher de bruit, dans lequel lors de l'estimation du plancher de bruit de chaque cellule de portée après l'intégration non cohérente, le plancher de bruit de chaque cellule de portée est ajusté en utilisant un plancher de bruit global ;
ou
dans lequel la réalisation de la détection de cible sur la base du résultat du traitement CFAR secondaire comprend :
la prédivision de la région d'intérêt en plusieurs sous-régions de cible, la détermination du nombre et d'emplacements de points de cible dans chaque sous-région sur la base des emplacements des points de cible détectés, et la détermination de la présence d'une cible dans la sous-région actuelle sur la base du fait que le rapport du nombre de points de cible dans chaque sous-région au nombre total de cibles dépasse ou non une valeur de rapport prédéfinie.

4. Procédé de détection de cible selon la revendication 2, dans lequel l'application du traitement CFAR secondaire aux points de détection de cible candidate comprend :
la réalisation d'une formation de faisceau directionnel en azimut, DBF, sur les points de détection de cible candidate, et la réalisation d'un traitement CFAR en azimut en combinaison avec une estimation de plancher de bruit, pour obtenir un résultat de filtrage de cible de dimension en azimut ; et/ou la réalisation d'une DBF d'altitude sur les points de détection de cible candidate, et la réalisation d'un traitement CFAR d'altitude en combinaison avec le résultat d'estimation de plancher de bruit, pour obtenir un résultat de filtrage de cible de dimension d'altitude ; et
la réalisation d'une DBF bidimensionnelle d'altitude-azimut sur les points de détection de cible candidate, et la réalisation d'un traitement CFAR d'altitude-azimut en combinaison avec le résultat d'estimation de plancher de bruit, pour obtenir un résultat de filtrage de cible bidimensionnel d'altitude-azimut.

5. Procédé de détection de cible selon la revendication 4, dans lequel :
au cours du traitement CFAR, une ou plusieurs des opérations suivantes sont réalisées sur les points de détection de cible candidate pour obtenir le résultat de détection de cible : filtrage de valeur maximale globale, filtrage de première valeur de seuil et filtrage de seconde valeur de seuil, dans lequel le filtrage de valeur maximale globale est utilisé pour filtrer si une valeur maximale globale est un point de détection de cible, le premier filtrage de valeur de seuil détermine si un résultat de filtrage de candidat actuel est un point de détection de cible selon une relation entre un résultat de filtrage candidat et une valeur d'estimation de plancher de bruit, et le second filtrage de valeur de seuil détermine si le résultat de filtrage de candidat actuel est un point de détection de cible selon une relation entre le résultat de filtrage candidat et la valeur maximale globale.

6. Procédé de détection de cible selon la revendication 5, dans lequel,
le filtrage de valeur maximale globale comprend : le fait de déterminer si une différence entre une valeur d'amplitude spectrale de formation de faisceau numérique correspondant à une valeur maximale globale actuelle et la valeur d'estimation de plancher de bruit se situe au sein d'une première plage prédéfinie, et si la différence se situe au sein de la première plage prédéfinie, le résultat de filtrage candidat correspondant à la valeur maximale globale est un point de détection de cible ;
le premier filtrage de valeur de seuil comprend : le fait de déterminer si une différence entre le résultat de filtrage de candidat actuel et la valeur d'estimation de plancher de bruit se situe au sein d'une deuxième plage prédéfinie, et si la différence se situe au sein de la deuxième plage prédéfinie, le résultat de filtrage candidat actuel est un point de détection de cible ; et
le second filtrage de valeur de seuil comprend : le fait de déterminer si une différence entre une valeur de puissance du résultat de filtrage candidat actuel et une puissance de la valeur maximale globale se situe au sein d'une troisième plage prédéfinie, et si la différence se situe au sein de la troisième plage prédéfinie, le résultat de filtrage candidat actuel est un point de détection de cible.

7. Procédé de détection de cible selon la revendication 1, dans lequel l'exécution du traitement FFT inter-trame sur les données 1D-FFT multi-trame accumulées pour générer le spectre RD et la mise en œuvre de la détection de la cible dans la région d'intérêt sur la base du spectre RD comprend :
l'extraction de données de cible à partir des données 1D-FFT multi-trame accumulées ;
l'application d'un fenêtrage glissant aux données de cible ; et
l'exécution d'un traitement de signal numérique sur des données fenêtrées pour dériver des informations de cible ;
dans lequel la durée de la fenêtre coulissante est proportionnelle à l'ordre de grandeur du cycle de mouvement périodique d'une cible.

8. Procédé de détection de cible selon la revendication 7, dans lequel,
l'extraction de données de cible à partir des données 1D-FFT multi-trame accumulées comprend :
l'extraction d'au moins une partie de données ou de données traitées à partir de données de chaque trame obtenues après le traitement FFT de portée en tant que données de cible ; ou
la détermination de paramètres correspondant à chaque cellule de portée dans chaque donnée de trame sur la base des résultats de traitement FFT de portée pour générer les données de cible ;
et l'exécution d'un traitement de signal numérique sur les données fenêtrées pour dériver les informations de cible comprend :
la réalisation d'une estimation de plancher de bruit au sein d'une plage prédéfinie, où une limite supérieure de la plage prédéfinie est déterminée sur la base d'un résultat d'estimation de plancher de bruit d'une cellule de portée avec une distance la plus éloignée ; et
la réalisation d'une détection de taux constant de fausses alertes, CFAR, sur la base d'un plancher de bruit estimé pour obtenir les informations de cible ;
ou
l'extraction des données de cible à partir des données de trame obtenues après le traitement FFT de portée comprend :
l'extraction de données au sein d'une plage de distance prédéfinie à partir des données de trame obtenues après le traitement FFT de portée en tant que données cibles ; et/ou,
l'exécution du traitement de signal numérique sur les données fenêtrées, comprend :
la réalisation du traitement de signal numérique sur des données dans une portée Doppler prédéfinie à partir des données fenêtrées ;
ou
dans lequel les données sont mises en caches à l'aide d'un anneau FIFO, le procédé comprenant en outre : l'extraction de données à partir de l'anneau FIFO et le réordonnancement des données extraites ;
ou
dans lequel l'application d'un fenêtrage glissant aux données de cible et l'exécution du traitement de signal numérique sur les données fenêtrées pour dériver les informations de cible comprend :
l'application d'un fenêtrage glissant aux données de cible conformément à des périodes de divers paramètres physiologiques d'un sujet vivant, et le traitement des données fenêtrées associées à chaque longueur de fenêtre glissante distincte par l'intermédiaire d'un traitement de signal numérique ;
dans lequel les périodes des paramètres physiologiques associés à chaque fenêtre coulissante varient, et la durée de chaque fenêtre coulissante est proportionnelle à la période de son paramètre physiologique correspondant en termes d'ordre de grandeur.

9. Procédé de détection de cible selon la revendication 1 ou 7, dans lequel la détection de la cible dans la région d'intérêt sur la base du spectre RD comprend :
l'application d'un traitement de taux constant de fausses alertes, CFAR, individuel à au moins deux canaux d'émetteur-récepteur sur la base du spectre RD, pour acquérir des données de cible candidate pour chacun desdits au moins deux canaux d'émetteur-récepteur ; et
le traitement des données de cible candidate acquises à partir d'au moins deux canaux d'émetteur-récepteur pour produire des données de cible finale.

10. Procédé de détection de cible selon la revendication 9, dans lequel l'application d'un traitement de taux de fausses alertes constant, CFAR, individuel à au moins deux canaux d'émetteur-récepteur sur la base du spectre RD comprend :
l'application séparée d'un traitement CFAR à au moins deux canaux d'émetteur-récepteur sur la base du spectre RD ; ou
l'exécution d'une estimation de plancher de bruit pour chaque canal d'émetteur-récepteur subissant un traitement CFAR conformément au spectre RD, pour obtenir un résultat d'estimation de plancher de bruit pour chaque canal d'émetteur-récepteur individuel ; et l'utilisation du résultat d'estimation de plancher de bruit de chaque canal d'émetteur-récepteur individuel pour réaliser individuellement une détection CFAR sur le canal d'émetteur-récepteur correspondant ;
ou
dans lequel le traitement des données de cible candidate acquises à partir d'au moins deux canaux d'émetteur-récepteur comprend :
la réalisation d'une détection CFAR sur des données de cible candidate actuellement acquises par rapport à un seuil prédéfini pour obtenir l'ensemble de cible finale.

11. Procédé de détection de cible selon l'une quelconque des revendications 7 à 10, le procédé comprenant en outre :
la validation de cibles au sein de chaque zone prédéfinie en fonction de cibles situées dans celle-ci, les zones prédéfinies étant obtenues en partitionnant l'aire de surveillance ; et
la dissémination des conclusions de détection pour chaque zone prédéfinie sur la base de cibles validées.

12. Procédé de détection de cible selon la revendication 1, dans lequel pour un scénario d'application multicanal, la mise en œuvre de la détection de la cible dans la région d'intérêt sur la base du spectre RD comprend :
après la réalisation d'une intégration non cohérente sur le spectre RD de plusieurs canaux, la poursuite d'un traitement CFAR et d'une estimation de la direction d'arrivée, DoA, sur la base d'une estimation de bruit pour parvenir à une détection de cible ; ou
après la réalisation d'un traitement CFAR respectivement sur le spectre RD de plusieurs canaux, la poursuite d'un traitement d'intégration binaire dans le domaine des canaux et d'une estimation de DoA, puis la réalisation de l'une quelconque des opérations suivantes sur la cible : détermination, positionnement et classification ;
ou
dans lequel la détection de la cible dans la région d'intérêt sur la base du spectre RD comprend :
sur la base de l'extraction d'au moins une partie des données intégrées à partir de données de transformation de Fourier inter-trames, FFT, de multiples trames, de données intégrées non cohérentes et/ou de données de détection CFAR, la réalisation d'une identification /reconnaissance de cible.

13. Circuit intégré comprenant :
un module d'émission de signaux configuré pour émettre des ondes électromagnétiques pour la détection de cibles ;
un module de réception de signal configuré pour recevoir des échos formés par réflexion et/ou diffusion des ondes électromagnétiques ; et
un module de traitement conçu pour traiter les échos selon l'un quelconque des procédés de détection de cible selon l'une quelconque des revendications 1 à 12 pour détecter des cibles.

14. Capteur d'ondes électromagnétiques, comprenant :
une porteuse ;
un circuit intégré selon la revendication 13 monté sur la porteuse ;
une antenne, soit montée sur la porteuse soit intégrée au circuit intégré en tant que dispositif unifié sur la porteuse ;
dans lequel le circuit intégré est relié à l'antenne et configuré pour transmettre un signal d'onde électromagnétique et/ou recevoir un signal d'écho.

15. Dispositif terminal comprenant :
un corps de dispositif ; et
le capteur d'ondes électromagnétiques selon la revendication 14 monté sur le corps de dispositif ;
dans lequel le capteur d'ondes électromagnétiques est utilisé pour une détection de cible et/ou une communication pour fournir des informations de référence pour le fonctionnement du corps de dispositif.
